# EUROPÄISCHE PATENTSCHRIFT

(11) **EP 1 529 042 B1**
(45) Veröffentlichungstag und Bekanntmachung des Hinweises auf die Patenterteilung: **20.02.2013**
(21) Anmeldenummer: 03790823.3
(22) Anmeldetag: 28.07.2003
(51) Int. Cl.: C07D 401/12, C07D 207/40, C07D 403/12, C07D 417/12, C07D 413/12, C07D 413/14, A61K 31/40, A61P 31/04

(54) **ARYLAMIDE**
ARYLAMIDES
ARYLAMIDES

(30) Priorität: 08.08.2002 DE 10236340
(43) Veröffentlichungstag der Anmeldung: 11.05.2005
(73) Patentinhaber: InterMed Discovery GmbH, 44227 Dortmund (DE)
(72) Erfinder: BRUNNER, Nina, 4128 Essen (DE); FREIBERG, Christoph, 42115 Wuppertal (DE); LAMPE, Thomas, 40223 Düsseldorf (DE); NELL, Peter, 42115 Wuppertal (DE); NEWTON, Ben, HP6 6BX Amersham Bucks (GB); OTTENEDER, Michael, CH-4144 Arlesheim (CH); PERNERSTORFER, Josef, 40721 Hilden (DE); POHLMANN, Jens, CH-4058 Basel (DE); SCHIFFER, Guido, 42111 Wuppertal (DE); SHIMADA, Mitsuyuki 905 Laurel Court Nara Minami, Nara 630-8323 (JP); SVENSTRUP, Niels, 42553 Velbert (DE); ENDERMANN, Rainer, 42113 Wuppertal (DE)
(74) Vertreter: Bülle, Jan
(86) Internationale Anmeldenummer: PCT/EP2003/008300
(87) Internationale Veröffentlichungsnummer: WO 2004/020432

(56) Entgegenhaltungen:
- EP-A- 0 250 115
- NEEDHAM ET AL: "Andrimid and moiramides A-C, metabolites produced in culture by a marine isolate of the bacterium Pseudomonas fluorescens: structure elucidation and biosynthesis" JOURNAL OF ORGANIC CHEMISTRY, AMERICAN CHEMICAL SOCIETY. EASTON, US, Bd. 59, Nr. 8, 1994, Seiten 2058-2063, XP002234916 ISSN: 0022-3263 in der Anmeldung erwähnt

## Beschreibung

Die vorliegende Erfindung betrifft Verbindungen, Verfahren zur ihrer Herstellung, sie umfassende pharmazeutische Zusammensetzungen sowie ihre Verwendung bei der Behandlung und/oder Prophylaxe von Erkrankungen bei Menschen oder Tieren, insbesondere bakteriellen Infektionskrankheiten.

Die Naturstoffe Moiramid B (R^{a} = Wasserstoff, R^{b} = Methyl) und Andrimid (R^{a} = Wasserstoff, R^{b} = Propenyl) sind als antibakteriell wirksam beschrieben, während Moiramid C (R^{a} = Hydroxy, R^{b} = Propenyl) unwirksam ist. (A. Fredenhagen, S. Y. Tamura, P. T. M. Kenny, H. Komura, Y. Naya, K. Nakanishi, J. Am. Chem. Soc., 1987, 109, 4409-4411; J. Needham, M. T. Kelly, M. Ishige, R. J. Andersen, J. Org. Chem., 1994, 59, 2058-2063; M. P. Singh, M. J. Mroczenski-Wildey, D. A. Steinberg, R. J. Andersen, W. M. Maiese, M. Greenstein, J. Antibiot., 1997, 50(3), 270-273). Die Isolierung und antibakterielle Wirksamkeit von Andrimid ist auch in EP-A-250 115 beschrieben. JP 01301657 beschreibt die Verwendung von Andrimid und einiger amidischer Derivate als agrochemische Antibiotika.

Die Synthese von Andrimid wird in A. V. Rama Rao, A. K. Singh, Ch. V. N. S. Varaprasad, Tetrahedron Letters, 1991, 32, 4393-4396 beschrieben, diejenige von Moiramid B und Andrimid in S. G. Davies, D. J. Dixon, J. Chem. Soc. Perkin Trans. 1, 1998, 2635-2643.

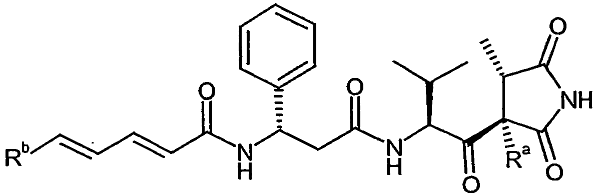

Auf dem Markt sind zwar strukturell andersartige antibakteriell wirkende Mittel vorhanden, es kann aber regelmäßig zu einer Resistenzentwicklung kommen. Neue Mittel für eine bessere und wirksame Therapie sind daher wünschenswert.

Eine Aufgabe der vorliegenden Erfindung ist es daher, neue und alternative Verbindungen mit gleicher oder verbesserter antibakterieller Wirkung zur Behandlung von bakteriellen Erkrankungen bei Menschen und Tieren zur Verfügung zu stellen.

Überraschenderweise wurde gefunden, dass Derivate dieser Verbindungsklasse, worin die beta-Phenylalanin-Amidgruppe durch ein heteroaromatisches oder substituiertes aromatisches Amid ersetzt wird, antibakteriell wirksam sind.

Gegenstand der Erfindung sind Verbindungen der Formel 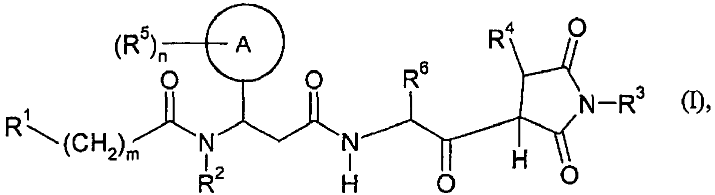 worin
- R¹: 5- bis 10-gliedriges Heteroaryl mit bis zu 5 Heteroatomen aus der Reihe S, O und N bedeutet, wobei Heteroaryl substituiert sein kann mit 0, 1, 2 oder 3 Substituenten R¹⁻¹, wobei die Substituenten R¹⁻¹ unabhängig voneinander ausgewählt werden aus der Gruppe bestehend aus Halogen, C₁-C₈-Alkyl, Nitro, Amino, C₁-C₆-Alkylamino, Cyano, Trifluormethyl, 3- bis 8-gliedriges Cycloalkyl, 4- bis 10-gliedriges Heterocyclyl mit bis zu 3 Heteroatomen und/oder Heterogruppen aus der Reihe N, O, S, SO, SO₂, C₆-C₁₄-Aryl, 5- bis 10-gliedriges Heteroaryl mit bis zu 5 Heteroatomen aus der Reihe S, O und N, Hydroxy, C₁-C₈-Alkoxy, C₆-C₁₄-Aryloxy, Benzyloxy, Carboxyl, C₁-C₈-Alkoxycarbonyl, Aminocarbonyl, C₁-C₈-Alkylcarbonylamino, C₁-C₆-Alkylaminocarbonyl und Aminosulfonyl,
oder
- R¹: C₆-C₁₄-Aryl bedeutet,
wobei Aryl substituiert ist mit 1, 2 oder 3 Substituenten R¹⁻², wobei die Substituenten R¹⁻² unabhängig voneinander ausgewählt werden aus der Gruppe bestehend aus Halogen, C₁-C₈-Alkyl, Nitro, Amino, C₁-C₆-Alkylamino, Cyano, Trifluormethyl, 3- bis 8-gliedriges Cycloalkyl, 4- bis 10-gliedriges Heterocyclyl mit bis zu 3 Heteroatomen und/oder Heterogruppen aus der Reihe N, O, S, SO, SO₂, C₆-C₁₄-Aryl, 5- bis 10-gliedriges Heteroaryl mit bis zu 5 Heteroatomen aus der Reihe S, O und N, Hydroxy, C₁-C₈-Alkoxy, C₆-C₁₄-Aryloxy, Benzyloxy, Carboxyl, C₁-C₈-Alkoxycarbonyl, Aminocarbonyl, C₁-C₈-Alkylcarbonylamino, C₆-C₁₄-Arylcarbonylamino, C₁-C₆-Alkylaminocarbonyl und Aminosulfonyl,
oder
zwei Substituenten R¹⁻² zusammen mit den Kohlenstoffatomen, an die sie gebunden sind, ein 3- bis 8-gliedriges Cycloalkyl oder 4- bis 10-gliedriges Heterocyclyl mit bis zu 3 Heteroatomen und/oder Heterogruppen aus der Reihe N, O, S, SO, SO₂ bilden, wobei dieses Cycloalkyl oder Heterocyclyl substituiert sein kann mit 0, 1 oder 2 Substituenten R¹⁻²⁻¹, wobei die Substituenten R¹⁻²⁻¹ unabhängig voneinander ausgewählt werden aus der Gruppe bestehend aus Halogen, Nitro, Amino, Trifluormethyl, Hydroxy, C₁-C₈-Alkyl und C₁-C₈-Alkoxy,
- R²: Wasserstoff oder Methyl bedeutet,
- R³: Wasserstoff, Hydroxy, Amino, C₁-C₃-Alkyl, Benzyl, C₁-C₃-Alkoxy, Benzyloxy, C₁-C₃-Alkylamino, C₁-C₃-Alkylcalbonylamino, Phenylcarbonyl-amino oder Benzylcarbonylamino bedeutet,
- R⁴: Wasserstoff oder C₁-C₃-Alkyl bedeutet,
- R⁵: Halogen, Trifluormethyl, Trifluormethoxy, Nitro, Amino, C₁-C₆-Alkylamino, Hydroxy, C₁-C₈-Alkyl, C₁-C₈-Alkoxy, Carbonyl, C₁-C₈-Alkoxycarbonyl, Aminocarbonyl, C₁-C₆-Alkylaminocarbonyl, C₆-C₁₄-Aryl oder 5- bis 10-gliedriges Heteroaryl mit bis zu 5 Heteroatomen aus der Reihe S, O und N bedeutet,
oder
zwei Substituenten R⁵ zusammen mit den Kohlenstoffatomen, an die sie gebunden sind, ein 3- bis 8-gliedriges Cycloalkyl oder 4- bis 10-gliedriges Heterocyclyl mit bis zu 3 Heteroatomen und/oder Heterogruppen aus der Reihe N, O, S, SO, SO₂ bilden, wobei dieses Cycloalkyl oder Heterocyclyl substituiert sein kann mit 0, 1 oder 2 Substituenten R⁵⁻¹, wobei die Substituenten R⁵⁻¹ unabhängig voneinander ausgewählt weiden aus der Gruppe bestehend aus Halogen, Nitro, Amino, Trifluormethyl, Hydroxy, C₁-C₈-Alkyl und C₁-C₈-Alkoxy,
- R⁶: C₁-C₈-Alkyl, 3- bis 8-gliedriges Cycloalkyl, 3- bis 8-gliedriges Cycloalkenyl oder 4- bis 10-gliedriges Heterocyclyl mit bis zu 3 Heteroatomen und/oder Heterogruppen aus der Reihe N, O, S, SO, SO₂ bedeutet,
wobei R⁶ substituiert sein kann mit 0, 1 oder 2 Substituenten R⁶⁻¹, wobei die Substituenten R⁶⁻¹ unabhängig voneinander ausgewählt weiden aus der Gruppe bestehend aus Halogen, Nitro, Amino, Trifluormethyl, Hydroxy, C₁-C₈-Alkyl und C₁-C₈-Alkoxy,
- n: eine Zahl 0, 1, 2 oder 3 bedeutet,
wobei bei n gleich 2 oder 3 die Reste R⁵ gleich oder verschieden sein können,
- m: eine Zahl 0, 1, 2, 3 oder 4 bedeutet,
- A: C₆-C₁₄-Aryl oder 5- bis 10-gliedriges Heteroaryl mit bis zu 5 Heteroatomen aus der Reihe S, O und N bedeutet,
und ihre Salze, ihre Solvate und die Solvate ihrer Salze.

Erfindungsgemäße Verbindungen sind die Verbindungen der Formel (I) und deren Salze, Solvate und Solvate der Salze, die von Formel (I) umfassten Verbindungen der nachfolgend genannten Formel (Ia) und deren Salze, Solvate und Solvate der Salze sowie die von Formel (I) und/oder (Ia) umfassten, nachfolgend als Ausführungsbeispiel(e) genannten Verbindungen und deren Salze, Solvate und Solvate der Salze, soweit es sich bei den von Formel (I) und/oder (Ia) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung betrifft daher die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Die Erfindung betrifft in Abhängigkeit von der Struktur der Verbindungen auch Tautomere der Verbindungen.

Als Salze sind im Rahmen der Erfindung physiologisch unbedenkliche Salze der erfindungsgemäßen Verbindungen bevorzugt.

Physiologisch unbedenkliche Salze der Verbindungen (I) umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Äpfelsäure, Zitronensäure, Fumarsäure, Maleinsäure und Benzoesäure.

Physiologisch unbedenkliche Salze der Verbindungen (I) umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclo-hexylamin, Dimethylaminoethanol, Prokain, Dibenzylamin, N-Methylmorpholin, Dihydroabietylamin, Arginin, Lysin, Ethylendiamin und Methylpiperidin.

Als Solvate werden im Rahmen der Erfindung solche Formen der Verbindungen bezeichnet, welche in festem oder flüssigem Zustand durch Koordination mit Lösungsmittehnolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:

Alkyl per se und "Alk" und "Alkyl" in Alkoxy, Alkylamino, Alkylaminocarbonyl, Alkylcarbonylamino und Alkoxycarbonyl stehen für einen linearen oder verzweigten Alkylrest mit in der Regel 1 bis 8, bevorzugt 1 bis 6, besonders bevorzugt 1 bis 4, ganz besonders bevorzugt 1 bis 3 Kohlenstoffatomen, beispielhaft und vorzugsweise für Methyl, Ethyl, n-Propyl, Isopropyl, tert-Butyl, n-Pentyl und n-Hexyl.

Alkoxy steht beispielhaft und vorzugsweise für Methoxy, Ethoxy, n-Propoxy, Isopropoxy, tert-Butoxy, n-Pentoxy und n-Hexoxy.

Alkylamino steht für einen Alkylaminorest mit einem oder zwei (unabhängig voneinander gewählten) Alkylsubstituenten, wobei die Alkylsubstituenten unabhängig voneinander in der Regel 1 bis 6, bevorzugt 1 bis 4, besonders bevorzugt 1 bis 3 Kohlenstoffatome aufweisen, beispielhaft und vorzugsweise für Methylamino, Ethylamino, n-Propylamino, Isopropylamino, tert-Butylamino, n-Pentylamino, n-Hexylamino, *N,N-*Dimethylamino, *N,N-*Diethylamino, *N*-Ethyl-*N*-methylamino, N-Methyl-*N*-n-propylamino, *N*-Isopropyl-*N*-n-propylamino, *N*-t-Butyl-*N*-methylamino, *N*-Ethyl-*N*-n-pentylamino und *N-*n-Hexyl-*N-*methylamino. C₁-C₃-Alkylamino steht beispielsweise für einen Monoalkylaminorest mit 1 bis 3 Kohlenstoffatomen oder für einen Dialkylaminorest mit jeweils 1 bis 3 Kohlenstoffatomen pro Alkylsubstituent.

Alkylaminocarbonyl steht für einen Alkylaminocarbonylrest mit einem oder zwei (unabhängig voneinander gewählten) Alkylsubstituenten, wobei die Alkylsubstituenten unabhängig voneinander in der Regel 1 bis 6, bevorzugt 1 bis 4, besonders bevorzugt 1 bis 3 Kohlenstoffatome aufweisen, beispielhaft und vorzugsweise für Methylaminocarbonyl, Ethylaminocarbonyl, n-Propylaminocarbonyl, Isopropylaminocarbonyl, tert-Butylaminocarbonyl, n-Pentylaminocarbonyl, n-Hexylaminocarbonyl, *N,N-*Dimethylaminocarbonyl, *N,N-*Diethylaminocarbonyl, *N*-Ethyl-*N*-methylaminocarbonyl, *N-*Methyl-*N-*n-propylaminocarbonyl, *N-*Isopropyl-*N-*n-propylaminocarbonyl, N-t-Butyl-*N*-methylaminocarbonyl, *N*-Ethyl-*N*-n-pentylamino-carbonyl und *N*-n-Hexyl-*N-*methylaminocarbonyl. C₁-C₃-Alkylaminocarbonyl steht beispielsweise für einen Monoalkylaminocarbonylrest mit 1 bis 3 Kohlenstoffatomen oder für einen Dialkylaminocarbonylrest mit jeweils 1 bis 3 Kohlenstoffatomen pro Alkylsubstituent.

Alkylcarbonylamino steht beispielhaft und vorzugsweise für Methylcarbonylamino, Ethylcarbonylamino, n-Propylcarbonylamino, Isopropylcarbonylamino, tert.-Butyl-carbonylamino, n-Pentylcarbonylamino und n-Hexylcarbonylamino.

Alkoxycarbonyl steht beispielhaft und vorzugsweise für Methoxycarbonyl, Ethoxycarbonyl, n-Propoxycarbonyl, Isopropoxycarbonyl, tert-Butoxycarbonyl, n-Pentoxycarbonyl und n-Hexoxycarbonyl.

Cycloalkyl steht für eine Cycloalkylgruppe mit in der Regel 3 bis 8, bevorzugt 5 bis 7 Kohlenstoffatomen, beispielhaft und vorzugsweise für Cycloalkyl sind genannt Cyclopropyl, Cyclobutyl, Cyclopentyl, Cyclohexyl und Cycloheptyl. Im Falle eines von zwei Aryl-Substituenten zusammen mit den Aryl-Kohlenstoffatomen, an die sie gebunden sind, gebildeten Cycloalkyl sind zwei Kohlenstoffatome der Cycloalkylgruppe sp²-hybridisiert.

Cycloalkenyl steht für eine Cycloalkenylgruppe mit in der Regel 3 bis 8, bevorzugt 5 bis 7 Kohlenstoffatomen, beispielhaft und vorzugsweise für Cycloalkenyl sind genannt Cyclopropenyl, Cyclobutenyl, Cyclopentenyl, Cyclohexenyl und Cycloheptenyl.

Aryl steht für einen mono- bis tricyclischen aromatischen Rest mit in der Regel 6 bis 14 Kohlenstoffatomen; beispielhaft und vorzugsweise für Aryl sind genannt Phenyl, Naphthyl und Phenanthrenyl.

Aryloxy steht für einen über ein Sauerstoffatom gebundenen mono- bis tricyclischen aromatischen Rest mit in der Regel 6 bis 14 Kohlenstoffatomen, beispielhaft und vorzugsweise für Phenoxy, Naphthyloxy und Phenanthrenyloxy.

Arylcarbonylamino steht beispielhaft und vorzugsweise für Phenylcarbonylamino, Naphthylcarbonylamino und Phenanthrenylcarbonylamino.

Heteroaryl steht für einen aromatischen, mono- oder bicyclischen Rest mit in der Regel 5 bis 10, vorzugsweise 5 bis 6 Ringatomen und bis zu 5, vorzugsweise bis zu 4 Heteroatomen aus der Reihe S, O und N, beispielhaft und vorzugsweise für Thienyl, Furyl, Pyrrolyl, Thiazolyl, Oxazolyl, Oxadiazolyl, Pyrazolyl, Imidazolyl, Pyridyl, Pyrimidyl, Pyridazinyl, Pyrazinyl, Indolyl, Indazolyl, Benzofuranyl, Benzothiophenyl, Chinolinyl, Isochinolinyl.

Heterocyclyl steht für einen mono- oder polycyclischen, vorzugsweise mono- oder bicyclischen, heterocyclischen Rest mit in der Regel 4 bis 10, vorzugsweise 5 bis 8 Ringatomen und bis zu 3, vorzugsweise bis zu 2 Heteroatomen und/oder Heterogruppen aus der Reihe N, O, S, SO, SO₂. Die Heterocyclyl-Reste können gesättigt oder teilweise ungesättigt sein. Bevorzugt sind 5- bis 8-gliedrige, monocyclische gesättigte Heterocyclylreste mit bis zu zwei Heteroatomen aus der Reihe O, N und S, wie beispielhaft und vorzugsweise Tetrahydrofuran-2-yl, Tetrahydrothienyl, Pyrrolidin-2-yl, Pyrrolidin-3-yl, Pyrrolinyl, Pyranyl, Piperidin-1-yl, Piperidin-2-yl, Piperidin-3-yl, Piperidin-4-yl, Thiopyranyl, Morpholin-1-yl, Morpholin-2-yl, Morpholin-3-yl, Perhydroazepinyl, Piperazin-1-yl, Piperazin-2-yl.

Halogen steht für Fluor, Chlor, Brom und Jod, vorzugsweise für Fluor und Chlor.

Wenn Reste in den erfindungsgemäßen Verbindungen substituiert sind, können die Reste, soweit nicht anders spezifiziert, ein- oder mehrfach gleich oder verschieden substituiert sein. Eine Substitution mit bis zu drei gleichen oder verschiedenen Substituenten ist bevorzugt. Ganz besonders bevorzugt ist die Substitution mit einem Substituenten.

Bevorzugt im Rahmen der vorliegenden Erfindung sind Verbindungen, welche der Formel 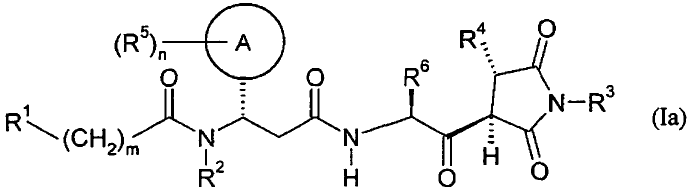 entsprechen, worin R¹ bis R⁶, A, m und n die gleiche Bedeutung wie in Formel (I) haben, und ihre Salze, ihre Solvate und die Solvate ihrer Salze.

Bevorzugt im Rahmen der vorliegenden Erfindung sind erfindungsgemäße Verbindungen, bei denen
- R¹: 5-, 6-, 9- oder 10-gliedriges Heteroaryl mit bis zu 5 Heteroatomen aus der Reihe S, O und N bedeutet,
wobei R¹ substituiert sein kann mit 0, 1 oder 2 Substituenten R¹⁻¹, wobei die Substituenten R¹⁻¹ unabhängig voneinander ausgewählt werden aus der Gruppe bestehend aus Halogen, C₁-C₈-Alkyl, Amino, C₁-C₆-Alkylamino, Cyano, Trifluormethyl, C₆-C₁₄-Aryl, 5- bis 10-gliedriges Heteroaryl mit bis zu 5 Heteroatomen aus der Reihe S, O und N, C₁-C₈-Alkoxy, C₁-C₈-Alkoxycarbonyl und Aminocarbonyl,
oder
- R¹: Phenyl oder Naphthyl bedeutet,
wobei Phenyl oder Naphthyl substituiert sind mit 1, 2 oder 3 Substituenten R¹⁻², wobei die Substituenten R¹⁻² unabhängig voneinander ausgewählt werden aus der Gruppe bestehend aus Halogen, C₁-C₈-Alkyl, Nitro, Amino, C₁-C₆-Alkylamino, Cyano, Trifluormethyl, 3- bis 8-gliedriges Cycloalkyl, 4-bis 10-gliedriges Heterocyclyl mit bis zu 3 Heteroatomen und/oder Heterogruppen aus der Reihe N, O, S, SO, SO₂, C₆-C₁₄-Aryl, 5- bis 10-gliedriges Heteroaryl mit bis zu 5 Heteroatomen aus der Reihe S, O und N, C₁-C₈-Alkoxy, C₆-C₁₄-Aryloxy, Benzyloxy, C₁-C₈-Alkoxycarbonyl, Aminocarbonyl, C₁-C₈-Alkylcarbonylamino, C₆-C₁₄-Arylcarbonylamino, C₁-C₆-Alkylaminocarbonyl und Aminosufonyl,
oder
zwei Substituenten R¹⁻² zusammen mit den Kohlenstoffatomen, an die sie gebunden sind, ein 5- oder 6-gliedriges Cycloalkyl oder ein 5- oder 6-gliedriges Heterocyclyl mit bis zu 2 Heteroatomen und/oder Heterogruppen aus der Reihe N, O, S, SO, SO₂ bilden,
- R²: Wasserstoff bedeutet,
- R³: Wasserstoff; Hydroxy, Amino, Methyl, Benzyl, C₁-C₃-Alkoxy, Benzyloxy oder C₁-C₃-Alkylamino bedeutet,
- R⁴: Methyl bedeutet,
- R⁵: Fluor, Chlor, Trifluormethyl, Trifluormethoxy, Nitro, Amino, C₁-C₄-Alkylamino, Hydroxy, C₁-C₄-Alkoxy, Aminocanbonyl, C₁-C₄-Alkoxycarbonyl, C₁-C₄-Alkyl, Phenyl oder 5- oder 6- gliedriges Heteroaryl mit bis zu 4 Heteroatomen aus der Reihe S, O und N bedeutet,
oder
zwei Substituenten R⁵ zusammen mit den Kohlenstoffatomen, an die sie gebunden sind, ein 5- oder 6-gliedriges Cycloalkyl oder 5- oder 6-gliedriges Heterocyclyl mit bis zu 2 Heteroatomen und/oder Heterogruppen aus der Reihe N, O, S, SO, SO₂ bilden,
- R⁶: C₂-C₇-Alkyl oder 3- bis 7-gliedriges Cycloalkyl bedeutet,
wobei R⁶ substituiert sein kann mit 0, 1 oder 2 Substituenten R⁶⁻¹, wobei die Substituenten R⁶⁻¹ unabhängig voneinander ausgewählt werden aus der Gruppe bestehend aus Halogen, C₁-C₄-Alkoxy, C₁-C₄-Alkyl und Trifluormethyl,
- n: eine Zahl 0, 1 oder 2 bedeutet,
wobei bei n gleich 2 die Reste R⁵ gleich oder verschieden sein können,
- m: eine Zahl 0, 1, 2 oder 3 bedeutet,
und
- A: Phenyl, Naphthyl oder 5-, 6-, 9- oder 10-gliedriges Heteroaryl mit bis zu 5 Heteroatomen aus der Reihe S, O und N bedeutet.

Bevorzugt im Rahmen der vorliegenden Erfindung sind auch erfindungsgemäße Verbindungen, bei denen
- R¹: Pyridyl, Imidazolyl, Thienyl, Furyl, Oxadiazolyl, Pyrazolyl, Pyrazinyl, Pyridazinyl, Pyrimidinyl, Chinolinyl oder Isochinolinyl bedeutet,
wobei R¹ substituiert sein kann mit 0, 1 oder 2 Substituenten R¹⁻¹, wobei die Substituenten R¹⁻¹ unabhängig voneinander ausgewählt werden aus der Gruppe bestehend aus Halogen, C₁-C₈-Alkyl, Amino, Trifluormethyl, Phenyl und C₁-C₈-Alkoxy,
oder
- R¹: Phenyl oder Naphthyl bedeutet,
wobei Phenyl oder Naphthyl substituiert sind mit 1, 2 oder 3 Substituenten R¹⁻², wobei die Substituenten R¹⁻² unabhängig voneinander ausgewählt werden aus der Gruppe bestehend aus Halogen, C₁-C₄-Alkyl, Dimethylamino, Cyano, Trifluormethyl, 3- bis 7-gliedriges Cycloalkyl, 5- oder 6-gliedriges Heterocyclyl mit bis zu 2 Heteroatomen und/oder Heterogruppen aus der Reihe N, O, S, SO, SO₂, Phenyl, 5- oder 6-gliedriges Heteroaryl mit bis zu 4 Heteroatomen aus der Reihe S, O und N, C₁-C₃-Alkoxy, Phenyloxy, Benzyloxy, Phenylcarbonylamino und Aminosulfonyl,
oder
zwei Substituenten R¹⁻² zusammen mit den Kohlenstoffatomen, an die sie gebunden sind, ein 1,3-Benzodioxol oder ein 1,4-Benzodioxan bilden,
- R²: Wasserstoff bedeutet,
- R³: Wasserstoff, Amino, Methyl, Methoxy, Ethoxy, Methylamino oder Dimethylamino bedeutet,
- R⁴: Methyl bedeutet,
- R⁵: Fluor, Chlor, Trifluormethyl, C₁-C₄-Alkoxy, Methoxycarbonyl, C₁-C₄-Alkyl, Phenyl oder Pyridyl bedeutet,
oder
zwei Substituenten R⁵ zusammen mit dem Phenylring, an den sie gebunden sind, ein 1,3-Benzodioxol oder ein 1,4-Benzodioxan bilden,
- R⁶: C₃-C₆-Alkyl oder 3- bis 6-gliedriges Cycloalkyl bedeutet,
- n: eine Zahl 0, 1 oder 2 bedeutet,
wobei bei n gleich 2 die Reste R⁵ gleich oder verschieden sein können,
- m: eine Zahl 0, 1, 2 oder 3 bedeutet,
und
- A: Phenyl, Naphthyl, Pyridyl, Thienyl, Furanyl, Chinolinyl oder Isochinolinyl bedeutet.

Bevorzugt im Rahmen der vorliegenden Erfindung sind auch erfindungsgemäße Verbindungen, bei denen
- R¹: Pyridyl, Thienyl, Furyl, Chinolinyl oder Isochinolinyl bedeutet,
wobei R¹ substituiert sein kann mit 0, 1 oder 2 Substituenten R¹⁻¹, wobei die Substituenten R¹⁻¹ unabhängig voneinander ausgewählt werden aus der Gruppe bestehend aus Halogen, C₁-C₄-Alkyl, Trifluormethyl, Phenyl und C₁-C₃-Alkoxy,
oder
- R¹: Phenyl oder Naphthyl bedeutet,
wobei Phenyl oder Naphthyl substituiert sind mit 1, 2 oder 3 Substituenten R¹⁻², wobei die Substituenten R¹⁻² unabhängig voneinander ausgewählt werden aus der Gruppe bestehend aus Halogen, C₁-C₄-Alkyl, Dimethylamino, Cyano, Trifluormethyl, Tetrahydrofuran-2-yl, Tetrahydrothienyl, Pyrrolidin-2-yl, Pyrrolidin-3-yl, Pyrrolinyl, Pyranyl, Piperidin-1-yl, Piperidin-2-yl, Piperidin-3-yl, Piperidin-4-yl, Thiopyranyl, Morpholin-1-yl, Morpholin-2-yl, Morpholin-3-yl, Piperazin-1-yl, Piperazin-2-yl, Thienyl, Furyl, Pyrrolyl, Thiazolyl, Oxazolyl, Oxadiazolyl, Pyrazolyl, Imidazolyl, Pyridyl, Pyrimidyl, Pyridazinyl, Pyrazinyl, C₁-C₃-Alkoxy, Phenyloxy oder Benzyloxy,
oder
zwei Substituenten R¹⁻² zusammen mit den Kohlenstoffatomen, an die sie gebunden sind, ein 1,3-Benzodioxol oder ein 1,4-Benzodioxan bilden,
- R²: Wasserstoff bedeutet,
- R³: Wasserstoff; Amino, Methylamino oder Dimethylamino bedeutet,
- R⁴: Methyl bedeutet,
- R⁵: Fluor, Chlor, Trifluormethyl, C₁-C₃-Alkoxy, C₁-C₄-Alkyl, Phenyl oder Pyridyl bedeutet,
- R⁶: Isopropyl, tert-Butyl, Isopentyl, Cyclopentyl oder Cyclohexyl bedeutet,
- n: eine Zahl 0, 1 oder 2 bedeutet,
wobei bei n gleich 2 die Reste R⁵ gleich oder vei schieden sein können,
- m: eine Zahl 0, 1 oder 2 bedeutet,
und
- A: Phenyl, Naphthyl, Pyridyl, Thienyl, Chinolinyl oder Isochinolinyl bedeutet.

Bevorzugt im Rahmen der vorliegenden Erfindung sind auch erfindungsgemäße Verbindungen, bei denen
- R¹: Pyridyl, Thienyl, Furyl, Chinolinyl oder Isochinolinyl bedeutet,
wobei R¹ substituiert sein kann mit 0, 1 oder 2 Substituenten R¹⁻¹, wobei die Substituenten R¹⁻¹ unabhängig voneinander ausgewählt werden aus der Gruppe bestehend aus Fluor, Chlor, Trifluormethyl, C₁-C₄-Alkyl, Phenyl und Methoxy

Bevorzugt im Rahmen der vorliegenden Erfindung sind auch erfindungsgemäße Verbindungen, bei denen
- R¹: Phenyl oder Naphthyl bedeutet,
wobei Phenyl oder Naphthyl substituiert sind mit 1, 2 oder 3 Substituenten R¹⁻², wobei die Substituenten R¹⁻² unabhängig voneinander ausgewählt werden aus der Gruppe bestehend aus Halogen, C₁-C₄-Alkyl, Dimethylamino, Cyano, Trifluormethyl, Tetrahydrofuzan-2-yl, Tetrahydrothienyl, Pyrolidin-2-yl, Pyrrolidin-3-yl, Pyrrolinyl, Pyranyl, Piperidin-1-yl, Piperidin-2-yl, Piperidin-3-yl, Piperidin-4-yl, Thiopyranyl, Morpholin-1-yl, Morpholin-2-yl, Morpholin-3-yl, Piperazin-1-yl, Piperazin-2-yl, Thienyl, Furyl, Pyrrolyl, Thiazolyl, Oxazolyl, Oxadiazolyl, Pyrazolyl, Imidazolyl, Pyridyl, Pyrimidyl, Pyridazinyl, Pyrazinyl, C₁-C₃-Alkoxy, Phenyloxy oder Benzyloxy,
oder
zwei Substituenten R¹⁻² zusammen mit den Kohlenstoffatomen, an die sie gebunden sind, ein 1,3-Benzodioxol oder ein 1,4-Benzodioxan bilden.

Bevorzugt im Rahmen der vorliegenden Erfindung sind auch erfindungsgemäße Verbindungen, bei denen R² Wasserstoff bedeutet

Bevorzugt im Rahmen der vorliegenden Erfindung sind auch erfindungsgemäße Verbindungen, bei denen R³ Wasserstoff oder Amino bedeutet.

Bevorzugt im Rahmen der vorliegenden Erfindung sind auch erfindungsgemäße Verbindungen, bei denen R⁴ Methyl bedeutet.

Bevorzugt im Rahmen der vorliegenden Erfindung sind auch erfindungsgemäße Verbindungen, bei denen n die Zahl Null bedeutet.

Bevorzugt im Rahmen der vorliegenden Erfindung sind auch erfindungsgemäße Verbindungen, bei denen n die Zahl 1 bedeutet, A Phenyl bedeutet und R⁵ Fluor, Chlor, Trifluormethyl, C₁-C₈-Alkoxy, C₁-C₄-Alkyl, Phenyl oder Pyridyl bedeutet, wobei R⁵ in der meta- oder para-Position zur Verknüpfüngsstelle des Phenyl-Ringes vorliegt. Unter der Verknüpfungsstelle des Phenyl-Ringes wird dabei das Kohlenstoffatom des R⁵ tragenden Phenyl-Ringes verstanden, mit dem der R⁵ tragende Phenyl-Ring gemäß Formel (I) oder (Ia) als A an den Rest der Verbindung gebunden ist.

Bevorzugt im Rahmen der vorliegenden Erfindung sind auch erfindungsgemäße Verbindungen, bei denen R⁶ C₃-C₆-Alkyl oder 3- bis 6-gliedriges Cycloalkyl bedeutet, insbesondere Isopropyl, Isobutyl, 1-Methylpropyl oder Cyclopentyl, ganz besonders Isopropyl oder Cyclopentyl.

Bevorzugt im Rahmen der vorliegenden Erfindung sind auch erfindungsgemäße Verbindungen, bei denen m die Zahl Null bedeutet.

Bevorzugt im Rahmen der vorliegenden Erfindung sind auch erfindungsgemäße Verbindungen, bei denen A Phenyl, Naphthyl, Pyridyl, Thienyl, Chinolinyl oder Isochinolinyl bedeutet.

Bevorzugt im Rahmen der vorliegenden Erfindung sind auch erfindungsgemäße Verbindungen, bei denen A Phenyl bedeutet.

Bevorzugt im Rahmen der vorliegenden Erfindung sind auch folgende Verbindungen:
6-Fluor-N-{(1*S*)-3-[((1*S*)-2-methyl-1-{[(3*R*,4*S*)-4-methyl-2,5-dioxo-3-pyrrolidinyl]-carbonyl}propyl)amino]-3-oxo-1-phenylpropyl}-2-pyridincarboxamid
N-{(1*S*)-3-[((1*S*)-2-Methyl-1-{[(3*R*,4*S*)-4-methyl-2,5-dioxo-3-pyrrolidinyl]-carbonyl}propyl)amino]-3-oxo-1-phenylpropyl}-3-chinolincarboxamid
N-{(1*S*)-3-[((1*S*)-2-Methyl-1-{[(3*R*,4*S*)-4-methyl-2,5-dioxo-3-pyrrolidinyl]-carbonyl}propyl)amino]-3-oxo-1-phenylpropyl}-4-phenyl-2-pyridincarboxamid
N-[(1*S*)-3-({(1*S*)-1-Cyclohexyl-2-[(3*R*,4*S*)-4-methyl-2,5-dioxo-3-pyrrolidinyl]-2-oxoethyl}amino)-3-oxo-1-phenylpropyl]-6-fluor-2-pyridincarboxamid
N-[(1*S*)-3-({1-Cyclopentyl-2-[(3*R*,4*S*)-4-methyl-2,5-dioxo-3-pyrrolidinyl]-2-oxoethyl}amino)-3-oxo-1-phenylpropyl]-2-pyridincarboxamid
(3*S*)-N-((1*S*)-2-Methyl-1-{[(3*R*,4*S*)-4-methyl-2,5-dioxo-3-pyrrolidinyl]-carbonyl}propyl)-3-phenyl-3-[(2-thienylacetyl)amino]propanamid
N-[(1*S*)-3-({(1*S*)-1-Cyclopentyl-2-[(3*R*,4*S*)-4-methyl-2,5-dioxo-3-pyrrolidinyl]-2-oxoethyl}amino)-3-oxo-1-phenylpropyl]-4-phenyl-2-pyridincarboxamid
N-[(1*S*)-3-({(1*S*)-1-Cyclopentyl-2-[(3*R*,4*S*)-4-methyl-2,5-dioxo-3-pyrrolidinyl]-2-oxoethyl}amino)-3-oxo-1-phenylpropyl]-3-chinolincarboxamid
N-[(1*S*)-3-({(1S*)*-1-Cyclopentyl-2-[(3*R*,4*S*)-4-methyl-2,5-dioxo-3-pyrrolidinyl]-2-oxoethyl}amino)-3-oxo-1-phenylpropyl]-5-fluor-1H-indol-2-carboxamid

Gegenstand der Erfindung ist weiterhin ein Verfahren zur Herstellung der Verbindungen der Formel (I), wobei
[A] Verbindungen der Formel 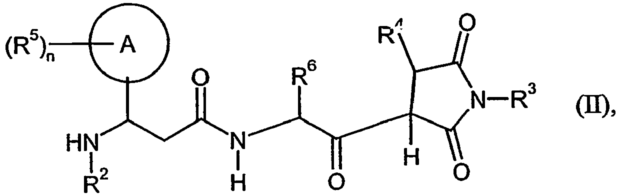 worin R² bis R⁶, A und n die oben angegebene Bedeutung aufweisen, mit Verbindungen der Formel 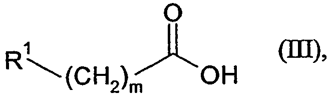 worin R¹ und m die oben angegebene Bedeutung aufweisen,
   wobei diese gegebenenfalls in aktivierter Form vorliegen können, umgesetzt werden,
   oder
[B] Verbindungen der Formel 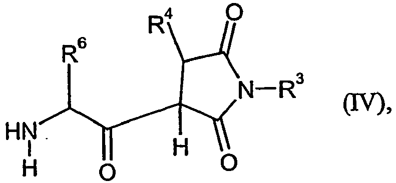 worin R³, R⁴ und R⁶ die oben angegebene Bedeutung aufweisen,
   mit Verbindungen der Formel 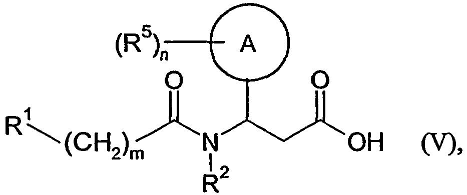 worin R¹, R², R⁵, A, m und n die oben angegebene Bedeutung aufweisen,
   wobei diese gegebenenfalls in aktivierter Form vorliegen können, umgesetzt werden.

Zur Überführung der Verbindungen in die aktivierte Form in den obengenannten Verfahren sind beispielsweise Carbodiimide wie z.B. N,N'-Diethyl-, N,N,'-Dipropyl-, N.N'-Diisopropyl-, N,N'-Dicyclohexylcarbodiimid, N-(3-Dimethylaminoisopropyl)-N'-ethylcarbodiimid-Hydrochlorid (EDC) (gegebenenfalls in Gegenwart von Pentafluorphenol (PFP)), N-Cyclohexylcarbodiimid-N'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder O-(Benzotriazol-1-yl)-N,N,N',N'-tetra-methyluronium-hexafluorophosphat (HBTU), 2-(2-Oxo-1-(2H)-pyridyl)-1,1,3,3-tetramethyluroniumtetrafluoro-borat (TPTU) oder O-(7-Azabenzotriazol-1-yl)-N,N,N',N'-tetramethyl-uroniumhexafluorophosphat (HATU), oder 1-Hydroxybenztriazol (HOBt), oder Benzotriazol-1-yloxytris-(dimethylamino)-phosphoniumhexafluoro-phosphat (BOP), oder Mischungen aus diesen mit Basen geeignet.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, N-Methylpiperidin, 4- Dimethylaminopyridin oder Diisopropylethylamin.

Bevorzugt ist die Verwendung von HATU mit Diisopropylethylamin und von EDC mit HOBt und Triethylamin.

Als Lösemittel eignen sich hierbei inerte organische Lösemittel, die sich unter den Reaktionsbedingungen nicht verändern. Hierzu gehören Halogenkohlenwasserstoffe wie Dichlormethan oder Trichlormethan, Kohlenwasserstoff wie Benzol, Xylol, Toluol, Hexan, Cyclohexan, oder Erdölfraktionen, Nitromethan, Dimethylformamid oder Acetonitril oder Ether wie Diethylether, Tetrahydrofuran oder Dioxan. Ebenso ist es möglich, Gemische der Lösemittel einzusetzen. Besonders bevorzugt sind Dichlormethan oder eine Mischung von Dichlormethan und Dimethylformamid.

### Verfahren [A]

Die Verbindungen der Formel (II) sind bekannt oder können hergestellt werden, indem Verbindungen der Formel 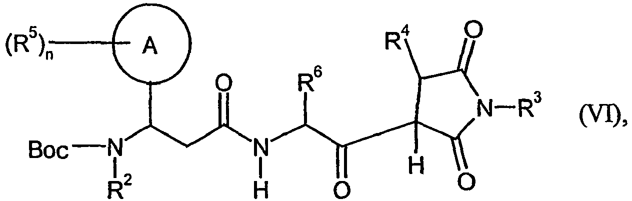 worin R² bis R⁶, A und n die oben angegebene Bedeutung aufweisen,
mit Säure, insbesondere mit Salzsäure oder Trifluoressigsäure versetzt werden. Die Verbindungen der Formel (II) fallen dabei in Form der entsprechenden Salze an, z.B. in Form ihrer Hydrochloride und können in dieser Form weiter eingesetzt werden oder durch chromatographische Reinigung in ihre salzfreie Form überführt werden.

Als Lösemittel eignen sich hierbei inerte organische Lösemittel, die sich unter den Reaktionsbedingungen nicht verändern. Hierzu gehören Halogenkohlenwasserstoffe wie Dichlormethan oder Trichlormethan, Kohlenwasserstoffe wie Benzol, Xylol, Toluol, Hexan, Cyclohexan, oder Erdölfraktionen, Nitromethan, Dimethylformamid oder Acetonitril oder Ether wie Diethylether, Tetrahydrofuran oder Dioxan. Ebenso ist es möglich, Gemische der Lösemittel einzusetzen. Besonders bevorzugt ist die Verwendung von Salzsäure in Dioxan oder Trifluoressigsäure in Dichlormethan.

Die Verbindungen der Formel (VI) sind bekannt oder können hergestellt werden, indem Verbindungen der Formel (IV) mit Verbindungen der Formel 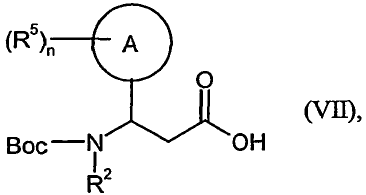 worin R², R⁵, A und n die oben angegebene Bedeutung aufweisen,
wobei diese gegebenenfalls in aktivierter Form vorliegen können, umgesetzt werden.

Zur Überführung der Verbindungen in die aktivierte Form sind beispielsweise Carbodiimide wie z.B. N,N'-Diethyl-, N,N,'-Dipropyl-, N,N-Diisopropyl-, N,N'-Dicyclohexylcarbodiimid, N-(3-Dimethylaminoisopropyl)-N'-ethylcarbodiimid-Hydrochlorid (EDC) (gegebenenfalls in Gegenwart von Pentafluorphenol (PFP)), N-Cyclohexylcarbodiimid-N'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder O-(Benzotriazol-1-yl)-N,N,N',N'-tetramethyluronium-hexafluorophosphat (HBTU), 2-(2-Oxo-1-(2H)-pyridyl)-1,1,3,3-tetramethyluroniumtetrafluoro-borat (TPTU) oder O-(7-Azabenzotriazol-1-yl)-N,N,N',N'-tetramethyl-uroniumhexafluorophosphat (HATU), oder 1-Hydroxybenztriazol (HOBt), oder Benzotriazol-1-yloxytris(dimethylamino)-phosphonium-hexafluoro-phosphat (BOP), oder Mischungen aus diesen mit Basen geeignet.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, N-Methylpiperidin, 4- Dimethylaminopyridin oder Diisopropylethylamin.

Bevorzugt ist die Verwendung von HATU mit Diisopropylethylamin und von EDC mit HOBt und Triethylamin.

Als Lösemittel eignen sich hierbei inerte organische Lösemittel, die sich unter den Reaktionsbedingungen nicht verändern. Hierzu gehören Halogenkohlenwasserstoffe wie Dichlormethan oder Trichlormethan, Kohlenwasserstoff wie Benzol, Xylol, Toluol, Hexan, Cyclohexan, oder Erdölfraktionen, Nitromethan, Dimethylformamid oder Acetonitril oder Ether wie Diethylether, Tetrahydrofuran oder Dioxan. Ebenso ist es möglich, Gemische der Lösemittel einzusetzen. Besonders bevorzugt sind Dichlormethan oder eine Mischung von Dichlormethan und Dimethylformamid.

Die Verbindungen der Formel (IV) sind literaturbekannt oder neu und können hergestellt werden, indem Verbindungen der Formel 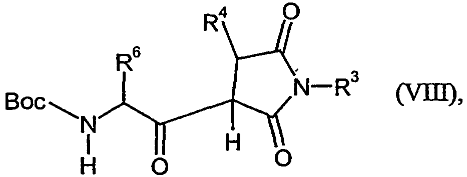 worin R³, R⁴ und R⁶ die oben angegebene Bedeutung aufweisen,
mit Säure, insbesondere mit Salzsäure oder Trifluoressigsäure versetzt werden.

Als Lösemittel eignen sich hierbei inerte organische Lösemittel, die sich unter den Reaktionsbedingungen nicht verändern. Hierzu gehören Halogenkohlenwasserstoffe wie Dichlormethan oder Trichlormethan, Kohlenwasserstoffe wie Benzol, Xylol, Toluol, Hexan, Cyclohexan, oder Erdölfraktionen, Nitromethan, Dimethylformamid oder Acetonitril oder Ether wie Diethylether, Tetrahydrofuran oder Dioxan. Ebenso ist es möglich, Gemische der Lösemittel einzusetzen. Besonders bevorzugt ist die Verwendung von Salzsäure in Dioxan oder Trifluoressigsäure in Dichlormethan.

Die Verbindungen der Formel (VII) sind bekannt oder können nach literaturbekannten Vorschriften hergestellt werden. (Bzgl. der Darstellung von aromatischen beta-Aminosäuren s. z. B. S. Rault, P. Dallemagne, M. Robba, Bull. Soc. Chim. Fr., 1987, 1079-1083; S. G. Davies et al., J. Chem. Soc., Chem. Commun., 1993, 1153-1155; V. A. Soloshonok et al., Tetrahedron Asymmetry, 1995, 1601-1610; bzgl. der Umsetzung zu den tert-Butoxycarbonyl-geschützten Verbindungen s. T. W. Greene, P. G. M. Wuts, Protective Groups in Organic Synthesis, 3rd Edt. 1999, J. Wiley & Sons, Inc.).

Die Verbindungen der Formel (VIII) sind bekannt oder können nach literaturbekannten Verfahren hergestellt werden. (Vgl. z.B. S. G. Davies, D. J. Dixon, J. Chem. Soc., Perkin Trans. 1, 1998, 17, 2635-2643; A. V. Rama Rao, A. K. Singh, Ch. V. N. S. Varaprasad, Tetrahedron Letters, 1991, 32, 4393-4396).

Die Verbindungen der Formel (III) sind bekannt oder können nach literaturbekannten Verfahren hergestellt werden (Houben-Weyl, Methoden der organischen Chemie, Bd. E5, Carbonsäuren und Carbonsäure-Derivate, Thieme Verlag, Stuttgart, 1985).

### Verfahren [B]

Die Verbindungen der Formel (V) sind literaturbekannt oder neu und können hergestellt werden, indem Verbindungen der Formel 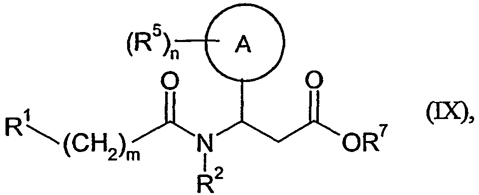 worin R¹, R², R⁵, A, m und n die oben angegebene Bedeutung aufweisen, und
R⁷ für einen Alkylrest steht, verseift werden.

Die Verseifung kann nach Standardverfahren durchgeführt werden, z.B. bei Raumtemperatur in einem Gemisch aus Ethanol und Wasser mit 40 %iger Natronlauge oder mit 10 %iger methanolischer Kaliumhydroxidlösung in einem Gemisch aus Dioxan und Wasser.

Die Verbindungen der Formel (IX) sind literaturbekannt oder neu und können hergestellt werden, indem Verbindungen der Formel 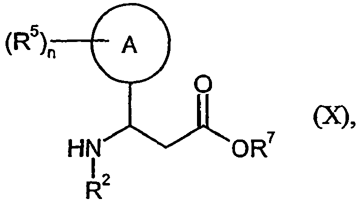 worin R², R⁵, R⁷, A und n die oben angegebene Bedeutung aufweisen,
mit Verbindungen der Formel (III), wobei diese gegebenenfalls in aktivierter Form vorliegen können, umgesetzt werden.

Zur Überführung der Verbindungen in die aktivierte Form in den obengenannten Verfahren sind beispielsweise Carbodiimide wie z.B. N,N'-Diethyl-, N,N,'-Dipropyl-, N,N'-Diisopropyl-, N,N'-Dicyclohexylcarbodiimid, N-(3-Dimethylaminoisopropyl)-N'-ethylcarbodiimid-Hydrochlorid (EDC) (gegebenenfalls in Gegenwart von Pentafluorphenol (PFP)), N-Cyclohexylcarbodümid-N'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder O-(Benzotriazol-1-yl)-N,N,N',N'-tetra-methyluronium-hexafluorophosphat (HBTU), 2-(2-Oxo-1-(2H)-pyridyl)-1,1,3,3-tetramethyluroniumtetrafluoro-borat (TPTU) oder O-(7-Azabenzotriazol-1-yl)-N,N,N',N'-tetramethyl-uroniumhexafluorophosphat (HATU), oder 1-Hydroxybenztriazol (HOBt), oder Benzotriazol-1-yloxytris-(dimethylamino)-phosphoniumhexafluoro-phosphat (BOP), oder Mischungen aus diesen mit Basen geeignet.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, N-Methylpiperidin, 4- Dimethylaminopyridin oder Diisopropylethylamin.

Bevorzugt ist die Verwendung von HATU mit Diisopropylethylamin und von EDC mit HOBt und Triethylamin.

Als Lösemittel eignen sich hierbei inerte organische Lösemittel, die sich unter den Reaktionsbedingungen nicht verändern. Hierzu gehören Halogenkohlenwasserstoffe wie Dichlormethan oder Trichlormethan, Kohlenwasserstoff wie Benzol, Xylol, Toluol, Hexan, Cyclohexan, oder Erdölfraktionen, Nitromethan, Dimethylformamid oder Acetonitril oder Ether wie Diethylether, Tetrahydrofuran oder Dioxan. Ebenso ist es möglich, Gemische der Lösemittel einzusetzen. Besonders bevorzugt sind Dichlormethan oder eine Mischung von Dichlormethan und Dimethylformamid.

Die Verbindungen der Formel (X) sind literaturbekannt oder neu und können analog literaturbekannten Verfahren hergestellt werden (Bzgl. der Darstellung von aromatischen beta-Aminosäuren und ihrer Umwandlung in die entsprechenden Alkylester s. z.B. S. Rault, P. Dallemagne, M. Robba, Bull. Soc. Chim. Fr., 1987, 1079-1083; S. G. Davies et al., J. Chem. Soc., Chem. Commun., 1993, 1153-1155; V. A. Soloshonok et al., Tetrahedron Asymmetry, 1995, 1601-1610; S. J. Faulconbridge et al., Tetrahedron Letters, 2000, 41, 2679-2682).

Die Synthese kann auch an einem polymeren Träger erfolgen. In diesem Fall bedeutet R² in der Synthesesequenz ein Polymer (Resin), bevorzugt ist die Verwendung von 4-(4-Formyl-3-methoxyphenoxy)butyryl-Aminomethyl-Polystyrol oder eines anderen Harzes, bei dem an ein polymeres Rückgrat wie z.B. Polystyrol oder BlockCopolymere von Polystyrol mit Ethylenglycol über eine Linkergruppe wie z.B. 3-Methoxyphenoxyethyl, 3,5-Dimethoxyphenoxyethoxymethyl oder 3-Methoxyphenoxybutyrylaminomethyl ein Formylrest oder ein anderer Rest gebunden ist, der die Anbindung von Aminen an den polymeren Träger ermöglicht.

Die Herstellung der erfindungsgemäßen Verbindungen kann durch folgende Syntheseschemata verdeutlicht werden.

### Ausgangsverbindungen:

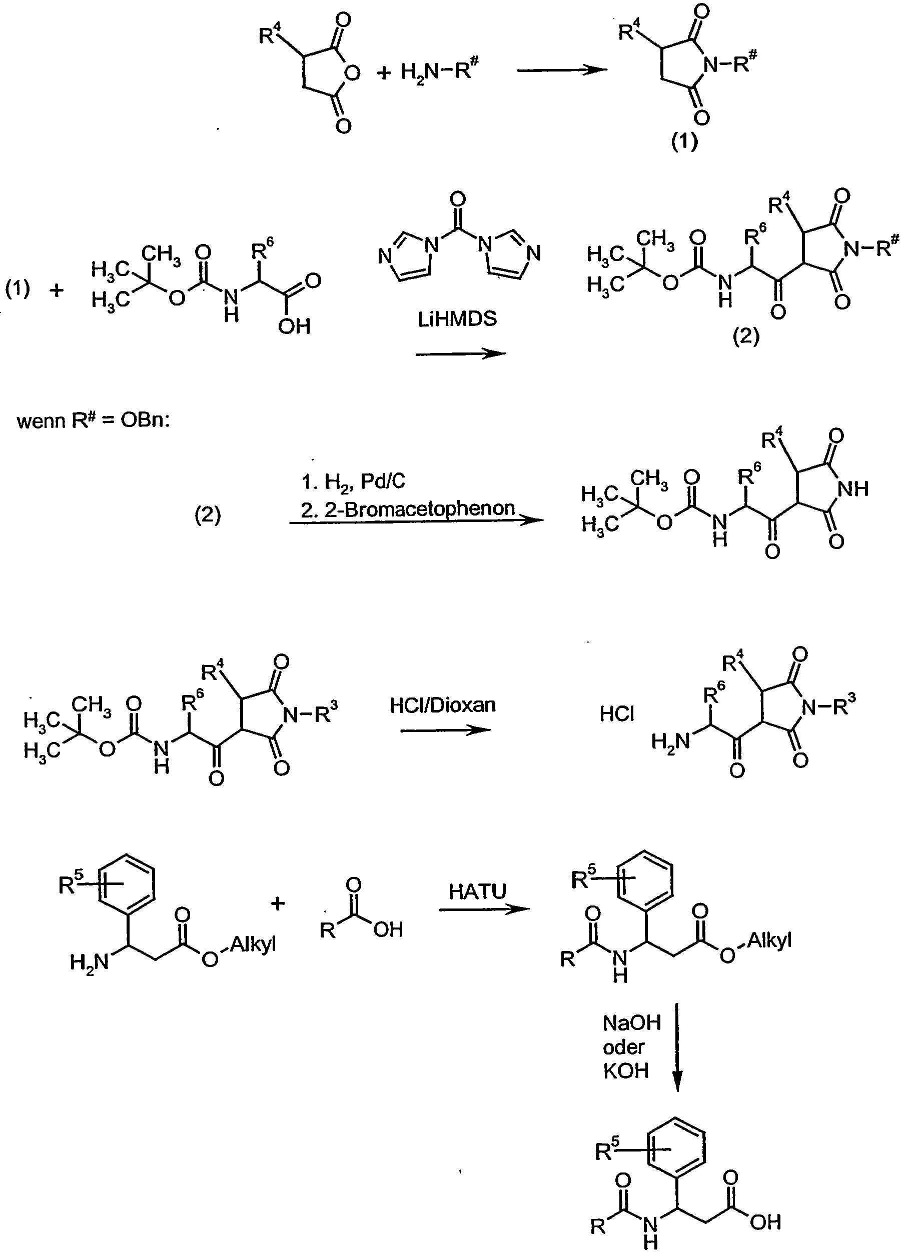

### Herstellungsbeispiele:

### Methode A

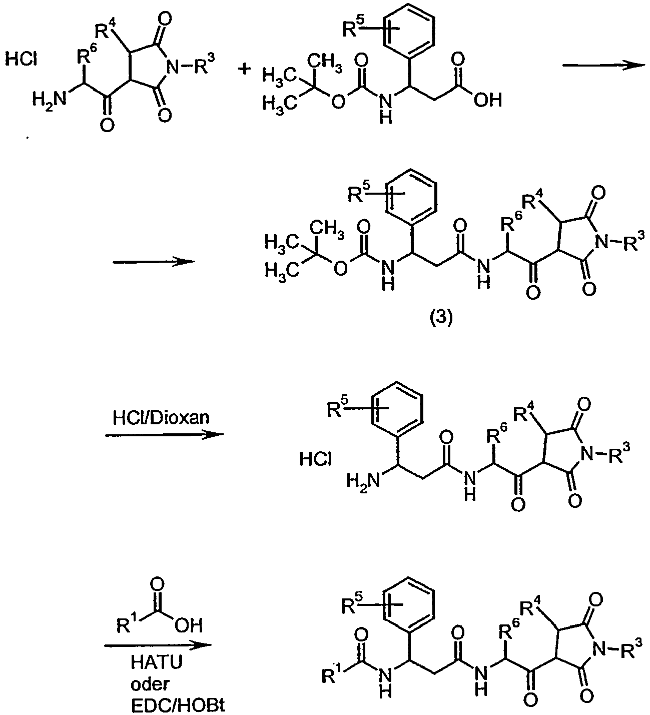

### Methode B

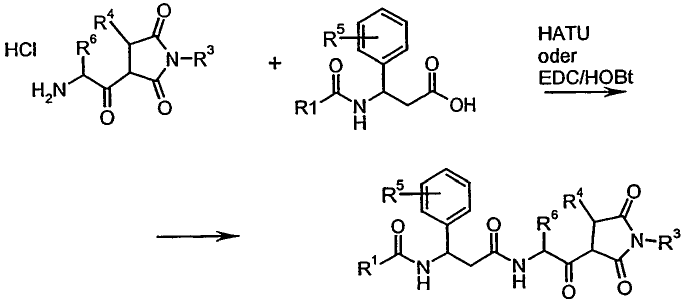

### Festphasensynthese:

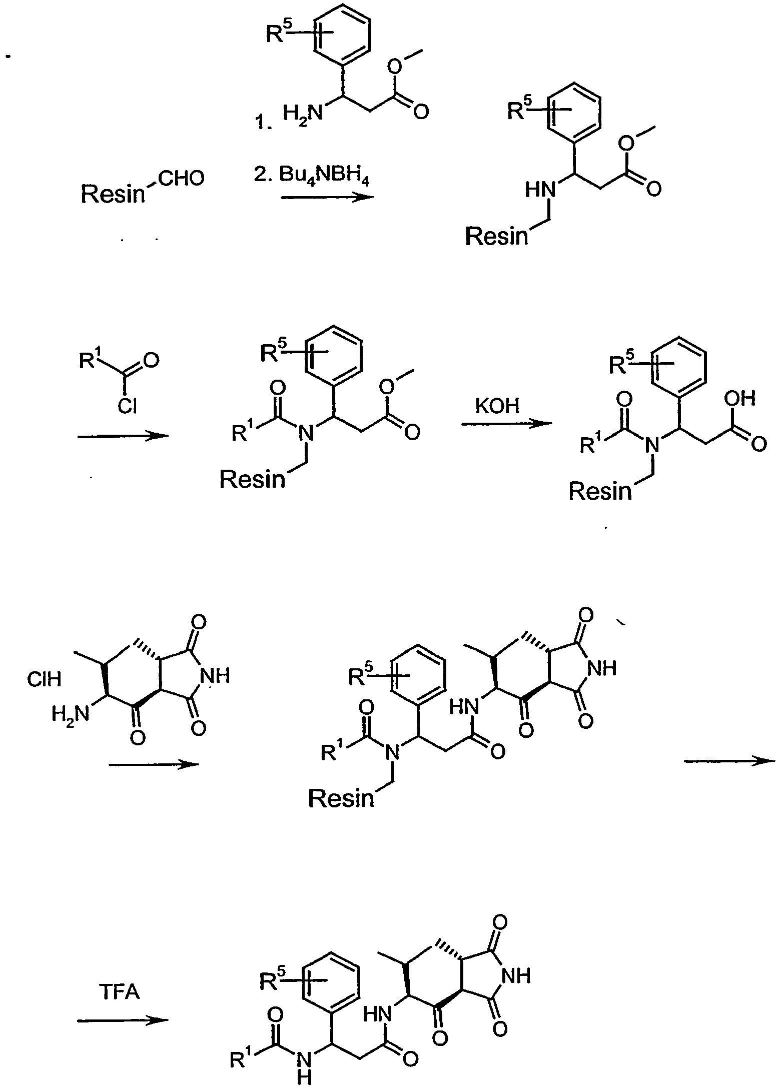

Die vorliegende Erfindung betrifft weiterhin Verbindungen der Formel (I) zur Bekämpfung von Erkrankungen, insbesondere bakterieller Erkrankungen, sowie Arzneimittel, enthaltend Verbindungen der Formel (I) und Hilfsstoffe und auch die Verwendung von Verbindungen der Formel (I) zur Herstellung eines Arzneimittels zur Behandlung von bakteriellen Erkrankungen.

Besonders wirksam sind die erfindungsgemäßen Zubereitungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Gram-positive Kokken, z.B. Staphylokokken (Staph. aureus, Staph. epidermidis), Enterokokken (E. faecalis, E. faecius) und Streptokokken (Strept. agalactiae, Strept. pneumoniae); gram-negative Kokken (Neisseria gonorrhoeae) sowie gram-negative Stäbchen wie Enterobakteriaceen, z.B. Escherichia coli, Hämophilus influenzae, Citrobacter (Citrob. freundii, Citrob. divernis), Salmonella und Shigella; ferner Klebsiellen (Klebs. pneumoniae, Klebs. oxytocy), Enterobacter (Ent. aerogenes, Ent. agglomerans), Hafinia, Serratia (Serr. marcescens), Providencia, Yersinia, sowie die Gattung Acinetobacter. Darüber hinaus umfaßt das antibakterielle Spektrum strikt anaerobe Bakterien wie z.B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, z.B. Mycobacterium tuberculosis.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Zubereitungen verhindert, gebessert oder geheilt werden können, seien beispielsweise genannt:

Infektionskrankheiten beim Menschen wie z. B. septische Infektionen, Knochen- und Gelenkinfektionen, Hautinfektionen, postoperative Wundinfektionen, Abszesse, Phlegmone, Wundinfektionen, infizierte Verbrennungen, Brandwunden, Infektionen im Mundbereich, Infektionen nach Zahnoperationen, septische Arthritis, Mastitis, Tonsillitis, Genital-Infektionen und Augeninfektionen.

Außer beim Menschen können bakterielle Infektionen auch bei anderen Spezies behandelt werden. Beispielhaft seien genannt:
Schwein: Coli-diarrhoe, Enterotoxamie, Sepsis, Dysenterie, Salmonellose, Metritis-Mastitis-Agalaktiae-Syndrom, Mastitis;
Wiederkäuer (Rind, Schaf, Ziege): Diarrhoe, Sepsis, Bronchopneumonie, Salmonellose, Pasteurellose, Mykoplasmose, Genitalinfektionen;
Pferd: Bronchopneumonien, Fohlenlähme, puerperale und postpuerperale Infektionen, Salmonellose;
Hund und Katze: Bronchopneumonie, Diarrhoe, Dermatitis, Otitis, Harnwegsinfekte, Prostatitis;
Geflügel (Huhn, Pute, Wachtel, Taube, Ziervögel und andere): Mycoplasmose, E. coli-Infektionen, chronische Luftwegserkrankungen, Salmonellose, Pasteurellose, Psittakose.

Ebenso können bakterielle Erkrankungen bei der Aufzucht und Haltung von Nutz - und Zierfischen behandelt werden, wobei sich das antibakterielle Spektrum über die vorher genannten Erreger hinaus auf weitere Erreger wie z.B. Pasteurella, Brucella, Campylobacter, Listeria, Erysipelothris, Corynebakterien, Borellia, Treponema, Nocardia, Rikettsie, Yersinia, erweitert.

Der Wirkstoff kann systemisch und/oder lokal wirken. Zu diesem Zweck kann er auf geeignete Weise appliziert werden, wie z.B. oral, parenteral, pulmonal, nasal, sublingual, lingual, buccal, rectal, transdermal, conjunctival, otisch oder als Implantat.

Für diese Applikationswege kann der Wirkstoff in geeigneten Applikationsformen verabreicht werden.

Für die orale Applikation eignen sich bekannte, den Wirkstoff schnell und/oder modifiziert abgebende Apphkationsformen, wie z.B. Tabletten (nichtüberzogene sowie überzogene Tabletten, z.B. mit magensaflresistenten Überzüge versehene Tabletten oder Filmtabletten), Kapseln, Dragees, Granulate, Pellets, Pulver, Emulsionen, Suspensionen und Lösungen.

Die parenterale Applikation kann unter Umgehung eines Resorptionsschrittes geschehen (intravenös, intraarteriell, intrakardial, intraspinal oder intralumbal) oder unter Einschaltung einer Resorption (intramuskulär, subcutan, intracutan, percutan, oder intraperitoneal). Für die parenterale Applikation eignen sich als Applikationsformen u.a. Injektions- und Infusionszubereitungen in Form von Lösungen, Suspensionen, Emulsionen, Lyophilisaten und sterilen Pulvern.

Bevorzugt ist die parenterale, insbesondere die intravenöse Applikation.

Für die sonstigen Applikationswege eignen sich z.B. Inhalationsarzneiformen (u.a. Pulverinhalatoren, Nebulizer), Nasentropfen/-lösungen, Sprays; lingual, sublingual oder buccal zu applizierende Tabletten oder Kapseln, Suppositorien, Ohren- und Augenpräparationen, Vaginalkapseln, wässrige Suspensionen (Lotionen, Schüttelmixturen), lipophile Suspensionen, Salben, Cremes, Milch, Pasten, Streupuder oder Implantate.

Die Wirkstoffe können in an sich bekannter Weise in die angeführten Applikationsformen überführt werden. Dies geschieht unter Verwendung inerter nichttoxischer, pharmazeutisch geeigneter Hilfsstoffe. Hierzu zählen u.a. Trägerstoffe (z.B. mikrokristalline Cellulose), Lösungsmittel (z.B. flüssige Polyethylenglycole), Emulgatoren (z.B. Natriumdodecylsulfat), Dispergiermittel (z.B. Polyvinylpyrrolidon), synthetische und natürliche Biopolymere (z.B. Albumin), Stabilisatoren (z.B. Antioxidantien wie Ascorbinsäure), Farbstoffe (z.B. anorganische Pigmente wie Eisenoxide) oder Geschmacks- und/oder Geruchskorrigentien.

Im Allgemeinen hat es sich als vorteilhaft erwiesen, bei parenteraler Applikation Mengen von etwa 5 bis 250 mg/kg Körpergewicht je 24 Stunden zur Erzielung wirksamer Ergebnisse zu verabreichen. Bei oraler Applikation beträgt die Menge etwa 5 bis 100 mg/kg Körpergewicht je 24 Stunden.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

Reaktionsschemata, die bei Allgemeinen Vorschriften gezeigt werden, zeigen eine Auswahl an Beispielen, sind aber jeweils für alle Beispiele anwendbar die darauf Bezug nehmen.

### Abkürzungen:

| | |
|---|---|
| Boc | tert.-Butoxycarbonyl |
| CDCl₃ | Deuterochloroform |
| DCI | Direkte Chemische Ionisation |
| DIEA | *N,N* Düsopropylethylamin |
| DMSO | Dimethylsulfoxid |
| d. Th. | der Theorie |
| EDC | N-(3-Dimethylaminoisopropyl)-N'-ethylcarbodiimid-Hydrochlorid |
| eq. | Äquivalent |
| ES | Elektrospray-Ionisation (bei MS) |
| Fmoc | Fluorenylmethoxycarbonyl |
| ges. | gesättigt |
| HATU | *O*-(7-Azabenzotriazol-l-yl)-*N,N,N,N'*-tetramethyluronium-Hexafluorphosphat |
| h | Stunde |
| HOBt | 1-Hydroxybenzotriazol |
| HPLC | Hochdruck-, Hochleistungsflüssigchromatographie |
| LC-MS | Flüssigchromatographie-gekoppelte Massenspektroskopie |
| MS | Massenspektroskopie |
| MW | Molekulargewicht [g/mol] |
| NMR | Kernresonanzspektroskopie |
| PS-DIEA | *N,N* Diisopropylethylamin-Polystyrol (-Harz) |
| R_{f} | Retentionsindex (bei DC) |
| RP-HPLC | Reverse Phase HPLC |
| RT | Raumtemperatur |
| Rₜ | Retentionszeit (bei HPLC) |
| THF | Tetrahydrofuran |

### HPLC und LC-MS Methoden:

Methode 1: Säule: Kromasil C18, L-R Temperatur: 30°C, Fluss = 0.75 mlmin⁻¹, Eluent: A = 0.01 M HClO₄, B = Acetonitril, Gradient: → 0.5 min 98 %A → 4.5 min 10 %A→ 6.5 min 10 %A.
Methode 2: Säule: Kromasil C18 60*2 mm, L-R Temperatur: 30°C, Fluss = 0.75 mlmin⁻¹, Eluent: A = 0.01 M H₃PO₄, B = Acetonitril, Gradient: → 0.5 min 90 %A → 4.5 min 10 %A → 6.5 min 10 %A.
Methode 3: Säule: Kromasil C18 60*2 mm, L-R Temperatur: 30°C, Fluss = 0.75 mlmin⁻¹, Eluent: A = 0.005 M HClO₄, B = Acetonitril, Gradient: → 0.5 min 98 %A → 4.5 min 10 %A → 6.5 min 10 %A.
**Methode 4**: Säule: Symmetry C18 2.1x150 mm, Säulenofen: 50°C, Fluss = 0.6 mlmin⁻¹, Eluent: A = 0.6 g 30 %ige Salzsäure/ 1 Wasser, B = Acetonitril, Gradient: 0.0 min 90 %A → 4.0 min 10 %A → 9 min 10 %A.
**Methode 5**: Instrument Micromass Quattro LCZ
   Säule Symmetry C18, 50 mm x 2.1 mm, 3.5 µm, Temperatur: 40°C, Fluss = 0.5 mlmin⁻¹, Eluent A = Acetonitril + 0.1% Ameisensäure, Eluent B = Wasser + 0.1 % Ameisensäure, Gradient: 0.0 min 10 % A → 4 min 90 % A → 6 min 90 % A.
Methode 6: Instrument Micromass Platform LCZ
   Säule Symmetry C18, 50 mm x 2.1 mm, 3.5 µm, Temperatur: 40°C, Fluss = 0.5 mlmin⁻¹, Eluent A = Acetonitril + 0.1 % Ameisensäure, Eluent B = Wasser + 0.1 % Ameisensäure, Gradient: 0.0 min 10 % A → 4 min 90 % A→ 6 min 90 % A.
Methode 7: Instrument Micromass Quattro LCZ
   Säule Symmetry C18, 50 mm x 2.1 mm, 3.5 µm, Temperatur: 40°C, Fluss = 0.5 mlmin⁻¹, Eluent A = Acetonitril + 0.1 % Ameisensäure, Eluent B = Wasser + 0.1 % Ameisensäure, Gradient: 0.0 min 5 % A → 1 min 5 % A → 5 min 90 % A → 6 min 90 % A.
Methode 8: Säule: Symmetry C18 2.1 mm x 150 mm, 5 µm, Säulenofen: 70°C, Fluss = 0.9 mlmin⁻¹, Eluent: A = Acetonitril, B = 0.3 g 30 %ige Salzsäure/ 1 Wasser, Gradient: 0.0 min 2 % A → 2.5 min 95 % A → 5 min 95 % A.
**Methode 9:** Säule: Symmetry C18 3.9 mm x 150 mm, Säulenofen: 40°C, Fluss = 1.5 mlmin⁻¹, Eluent: A = Wasser + 0.05% H₃PO₄, B = Acetonitril, Gradient: 0.0 min 10 % B → 0.6 min 10 % B → 3.8 min 100% B → 5.0 min 100 % B.
**Methode 10:** Instrument: Waters Alliance 2790 LC; Säule: Symmetry C18, 50 mm x 2.1 mm, 3.5 µm; Eluent A: Wasser + 0.1 % Ameisensäure, Eluent B: Acetonitril + 0.1 % Ameisensäure; Gradient: 0.0 min 5 % B → 5.0 min 10% B →6.0 min 10 % B; Temperatur: 50°C, Fluss: 1.0 ml/min, UV-Detektion: 210 nm.
**Methode 11:** Gerätetyp MS: Micromass ZQ; Gerätetyp HPLC: Waters Alliance 2790; Säule: Symmetry C 18, 50 mm x 2.1 mm, 3.5 µm; Eluent B: Acetonitril + 0.05 % Ameisensäure, Eluent A: Wasser + 0.05 % Ameisensäure; Gradient: 0.0 min 10 %B → 3.5 min 90 %B → 5.5 min 90 % B; Ofen: 50°C, Fluss: 0.8 ml/min, UV-Detektion: 210 nm.
**Methode 12:** Instrument: Waters Alliance 2790 LC; Säule: Symmetry C18, 50 mm x 2.1 mm, 3.5 µm; Eluent A: Wasser + 0.05 % Ameisensäure, Eluent B: Acetonitril + 0.05 % Ameisensäure; Gradient: 0.0 min 5 % B → 4.5 min 10 % B → 5.5 min 10 % B; Temperatur: 50°C, Fluss: 1.0 ml/min, UV-Detektion: 210 nm.
**Methode 13:** Instrument: Micromass Quattro LCZ, HP1100; Säule: Symmetry C18, 50 mm x 2.1 mm, 3.5 µm; Eluent A: Wasser + 0.05 % Ameisensäure, Eluent B: Acetonitril + 0.05 % Ameisensäure; Gradient: 0.0 min 90 % A → 4.0 min 10 % A → 6.0min 10 % A; Ofen: 40°C, Fluss: 0.5 ml/min, UV-Detektion: 208-400 nm.
**Methode 14:** Instrument: Micromass Platform LCZ, HP1100; Säule: Symmetry C18, 50 mm x 2.1 mm, 3.5 µm; Eluent A: Wasser + 0.05 % Ameisensäure, Eluent B: Acetonitril + 0.05 % Ameisensäure; Gradient: 0.0 min 90 %A 4.0 min 10 % A→ 6.0 min 10 % A; Ofen: 40°C, Fluss: 0.5 ml/min, UV-Detektion: 208-400 nm.
**Methode 15:** Instrument: Waters Alliance 2790 LC; Säule: Symmetry C18, 50 mm x 2.1 mm, 3.5 µm; Eluent A: Wasser + 0.05 % Ameisensäure, Eluent B: Acetonitril + 0.05 % Ameisensäure; Gradient: 0.0 min 10 % B → 4.0 min 90 % B → 6.0 min 90 % B; Temperatur: 50°C, Fluss: 0.0 min 0.5 ml/min → 4.0 min 0.8 ml/min, UV-Detektion: 210 nm.
**Methode 16:** Gerätetyp MS: Micromass ZQ; Gerätetyp HPLC: Waters Alliance 2790; Säule: Symmetry C 18, 50 mm x 2.1 mm, 3.5 µm; Eluent B: Acetonitril + 0.05 % Ameisensäure, Eluent A: Wasser + 0.05 % Ameisensäure; Gradient: 0.0 min 5 %B 4.5 min 90 %B 5.5 min 90 %B; Ofen: 50°C, Fluss: 1.0ml/, UV-Detektion: 210 nm.
**Methode 17:** Gerätetyp MS: Micromass ZQ; Gerätetyp HPLC: Waters Alliance 2790; Säule: Uptisphere C 18, 50 mm x 2.0 mm, 3.0 µm; Eluent B: Acetonitril + 0.05 % Ameisensäure, Eluent A: Wasser + 0.05 % Ameisensäure; Gradient: 0.0 min 5 %B → 2.0min 40 %B → 4.5min 90 %B → 5.5min 90 %B; Ofen: 45°C, Fluss: 0.0 min 0.75 ml/min → 4.5 min 0.75 ml/min→ 5.5 min 1.25 ml/min, UV-Detektion: 210 nm.
Methode 18: Instrument: Micromass Platform LCZ mit HPLC Agilent Serie 1100; Säule: Grom-SIL120 ODS-4 HE, 50 mm x 2.0 mm, 3 µm; Eluent A: 11 Wasser + 1 ml 50 %ige Ameisensäure, Eluent B: 11 Acetonitril + 1 ml 50 %ige Ameisensäure ; Gradient: 0.0 min 100 % A → 0.2 min 100 % A → 2.9 min 30 % A → 3.1 min 10 % A → 4.5 min 10 % A; Ofen: 55°C, Fluss: 0.8 ml/min, UV-Detektion: 208-400 nm.
Methode 19: Instrument: Micromass Quattro LCZ, mit HPLC Agilent Serie 1100; Säule: Grom-SIL120 ODS-4 HE, 50 mm x 2.0 mm, 3 →m; Eluent A: 11 Wasser + 1 ml 50 %ige Ameisensäure, Eluent B: 11 Acetonitril + 1 ml 50 %ige Ameisensäure; Gradient: 0.0 min 100 % A → 0.2 min 100 % A → 2.9 min 30 % A → 3.1 min 10 % A → 4.5 min 10 % A; Ofen: 55°C, Fluss: 0.8 ml/min, UV-Detektion: 208-400 nm.
**Methode 20:** Gerätetyp MS: Micromass ZQ; Gerätetyp HPLC: Waters Alliance 2790; Säule: Grom-Sil 120 ODS-4 HE 50 x 2 mm, 3.0 µm; Eluent B: Acetonitril + 0.05 % Ameisensäure, Eluent A: Wasser + 0.05 % Ameisensäure; Gradient: 0.0 min 5 % B → 2.0 min 40 % B → 4.5 min 90 % B→ 5.5 min 90 % B; Ofen: 45°C; Fluss: 0.0 min 0.75 ml/min → 4.5 min 0.75 ml/min→ 5.5 min 1.25 ml/min; UV-Detektion: 210 nm.
**Methode 21:** Gerätetyp MS: Micromass ZQ; Gerätetyp HPLC: Waters Alliance 2790; Säule: Grom-Sil 120 ODS-4 HE 50x2 mm, 3.0 µm; Eluent B: Acetonitril + 500 ul 50 %ige Ameisensäure / 1; Eluent A: Wasser + 500µl 50%ige Ameisensäure /1; Gradient: 0.0 min 0 % B → 0.2 min 0 % B → 2.9 min 70 % B→ 3.1 min 90 % B → 4.5 min 90 % B, Ofen: 50°C, Fluss:0.8 ml/min; UV-Detektion: 210 nm.
**Methode 22:** Instrument: Micromass Quattro LCZ mit HPLC Agilent Serie 1100; Säule: UPTISPHERE HDO, 50 mm x 2.0 mm, 3 µm; Eluent A: 11 Wasser + 1 ml 50 %ige Ameisensäure, Eluent B: 11 Acetonitril + 1 ml 50 %ige Ameisensäure; Gradient: 0.0 min 100 % A → 0.2 min 100 % A → 2.9 min 30 % A → 3.1 min 10 % A → 4.5 min 10 % A; Ofen: 55°C, Fluss: 0.8 ml/min, UV-Detektion: 208-400 nm.

### Ausgangsverbindungen:

### Beispiel 1A

### (3S)-1,3-Dimethyl-2,5-pyrrolidindion

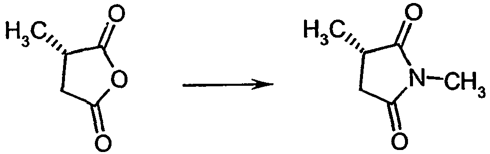

600 mg (5.26 mmol) (3S)-3-Methyldihydro-2,5-furandion (Darstellung: S. G. Davies, D. J. Dixon, J. Chem. Soc., Perkin Trans. 1, 1998, 17, 2635 - 2643) werden zusammen mit 559 mg (0.77 ml, 5.52 mmol) Triethylamin in 5 ml Dichlormethan bei 0°C vorgelegt und mit 373 mg (5.52 mmol) Methylamin Hydrochlorid versetzt. Das Reaktionsgemisch wird über Nacht bei Raumtemperatur gerührt und dann portionsweise mit 938 mg (5.78 mmol) N,N-Carbonyldiimidazol versetzt. Es wird 1.5 h bei Raumtemperatur und 30 min bei Rückflusstemperatur gerührt. Nach Abkühlen auf Raumtemperatur wird das Reaktionsgemisch mit 5 %iger Salzsäure und Wasser gewaschen, die organische Phase wird über Magnesiumsulfat getrocknet, filtriert und eingeengt und das Produkt wird am Hochvakuum getrocknet Es werden 605 mg des Produktes erhalten (88 % der Theorie).
MS (ES+): m/z (%) = 128 (M+H)⁺ (100).
HPLC (Methode 6): Rₜ = 0.81 min.
¹H-NMR (300 MHz, CDCl₃): δ = 3.10 (dd, 1 H), 2.99 (s, 3 H), 2.90-2.82 (m, 1 H), 2.32 (dd, 1 H), 1.35 (d, 3 H).

### Beispiel 2A

### (3R,4S)-3-[(2S)-2-(tert-Butoxycarbonyl)amino- 3-methylbutanoyl]-1,4-dimethyl- 2,5-pyrrolidindion

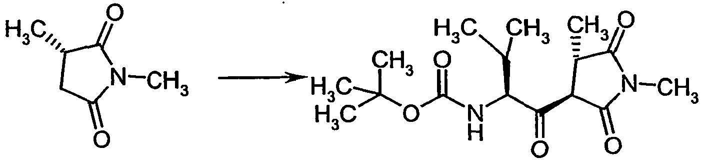

684 mg (3.15 mmol) N-(*tert*.-Butoxycarbonyl)-L-valin und 561 mg (3.46 mmol) N,N-Carbonyldiimidazol werden in 4 ml Tetrahydrofuran 2 h lang bei Raumtemperatur gerührt. Zu diesem Gemisch werden dann 400 mg (3.15 mmol) (3*S*)-1,3-Dimethyl-2,5-pyrrolidindion gegeben und die gesamte Mischung wird innerhalb von 30 min zu 6.3 ml einer auf -65°C gekühlten 1 molaren Lösung von Lithium-hexamethyldisilazid in THF getropft. Nach beendeter Zugabe wird weitere 15 min bei -65°C gerührt, bevor 6 ml gesättigter wässriger Ammoniumchlorid-Lösung zugegeben werden. Nach Erwärmung auf Raumtemperatur wird das Reaktionsgemisch mit Diethylether verdünnt und die organische Phase mit gesättigter wässriger Natriumchlorid-Lösung gewaschen und anschließend eingeengt. Das Rohprodukt wird durch RP-HPLC (Eluent: Wasser-Acetonitril, Gradient) gereinigt. Es werden 223 mg (22 % der Theorie) des gewünschten Produkts erhalten.
MS (ES-): m/z (%) = 325 (M-H)⁻ (35).
HPLC (Methode 5): Rₜ = 3.99 min.
¹H-NMR (200 MHz, CDCl₃): δ = 5.70 (br. d, 1 H), 4.57 (dd, 1 H), 3.78 (d, 1 H), 3.47-3.30 (m, 1 H), 2.98 (s, 3 H), 2.50- 2.32 (m, 1 H), 1.46 (s, 9 H), 1.32 (d, 3 H), 1.02 (d, 3 H), 0.80 (d, 3 H).

Analog lassen sich durch Umsetzung der entsprechenden N-tert.-Butoxycarbonyl-geschützten Aminosäuren mit (3*S*)-1-(Benzyloxy)-3-methyl-2,5-pyrrolidindion (Darstellung: S. G. Davies, D. J. Dixon, J. Chem. Soc., Perkin Trans. 1, 1998, 17, 2635-2643) folgende Derivate darstellen:

| **Beispiel** | **Struktur** | **MW** | **MS** | **HPLC** |
|---|---|---|---|---|
| 3A | 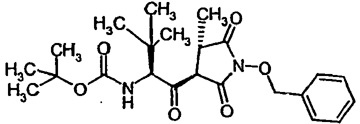 | 432.52 | MS (ES-), m/z(%):431 (M-H)⁻ (100) | HPLC (Methode 6): Rₜ = 4.87 min |
| 4A | 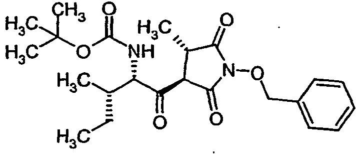 | 432.51 | MS (ES-), m/z (%): 431 (M-H)⁻ (100) | HPLC (Methode 10): Rₜ = 4.10 min |
| 5A | 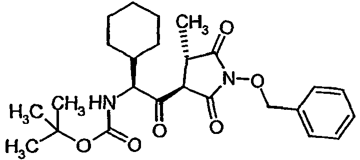 | 458.55 | MS (ES-), m/z (%): 457 (M-11)⁻ (100) | HPLC (Methode 12): Rₜ = 4.12 min |
| 6A | 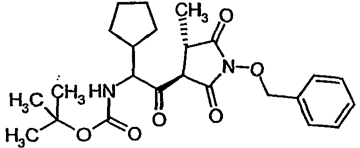 | 444.53 | MS (ES-), m/z (%): 443 (M-H)⁻ (100) | HPLC (Methode 12): Rₜ = 3.97 min |

### Allgemeine Vorschrift A: Reduktive Entschützung von 1-Benzyloxy-2,5-pyrrolidindionen

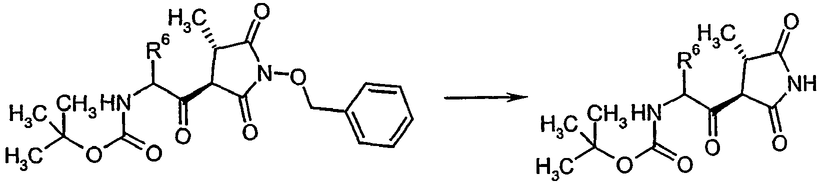

Die Entschützung erfolgt analog zu S. G. Davies, D. J. Dixon, J. Chem. Soc., Perkin Trans. 1, 1998, 17, 2635 - 2643.

Das 1-Benzyloxy-2,5-pyrrolidindion (1 eq) wird in Methanol gelöst (ca. 0.02 mol/l), mit einer katalytischen Menge Palladium-Kohle (10 %) versetzt und 1 h unter Wasserstoffatmosphäre (Normaldruck) gerührt. Dann wird das Reaktionsgemisch filtriert und eingeengt. Der Rückstand wird in Acetonitril gelöst (ca. 0.05 mol/l) und zu einer Lösung von 2-Bromacetophenon (1 eq) in Acetonitril (ca. 0.03 mol/l) bei Raumtemperatur getropft. Danach werden über einen Zeitraum von 2 h 1.5 eq Triethylamin in Acetonitril (ca. 0.35 mol/l) zu der Reaktionsmischung getropft. Das Reaktionsgemisch wird über Nacht bei Raumtemperatur gerührt, eingeengt und das Rohprodukt wird mittels RP-HPLC (Eluent: Acetonitril/Wasser + 0.3 ml 37 %ige Salzsäure/l, Gradient) gereinigt.

Nach der Allgemeinen Vorschrift A können folgende Verbindungen erhalten werden:

| **Beispiel** | **Struktur** | **MW** | **MS** | **HPLC** |
|---|---|---|---|---|
| 7A | 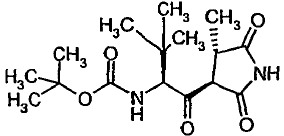 | 326.40 | MS (ES+), m/z (%): 327 (M+H)⁺ (100) | HPLC (Methode 5): Rₜ = 3.87 min |
| 8A | 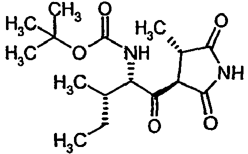 | 326.40 | MS(ES⁺), m/z (%): 349 (M+Na)⁺ (100) | HPLC (Methode 12): Rₜ = 2.97 min |
| 9A | 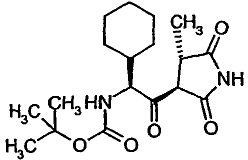 | 352.43 | MS (ES-), m/z (%): 351 (M-H)⁻ (100) | HPLC (Methode 12): Rₜ = 3.23 min |
| 10A | 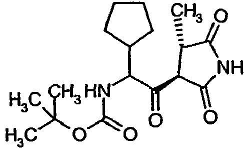 | 338.40 | MS (ES-), m/z (%): 337 (M-H)⁻(100) | HPLC (Methode 13): Rₜ = 4.16 min |

### Beispiel 11A

### (3R,4S)-3-[(2S)-2-Amino-3-methylbutanoyl]-4-methyl-2,5-pyrrolidindion Hydrochlorid

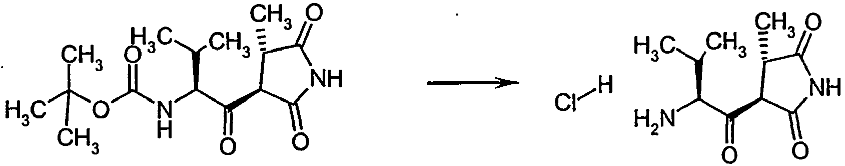

Zu einer auf 0°C abgekühlten Lösung von 4.40 g (14.09 mmol) (3*R*,4*S*)-3-[(2*S*)-2-(*tert*-Butoxycarbonyl)amino-3-methylbutanoyl]-4-methyl-2,5-pyrrolidindion (Darstellung: S. G. Davies, D. J. Dixon, J. Chem. Soc., Perkin Trans. 1, 1998, 17, 2635 - 2643) in Dioxan werden 35 ml 4N Salzsäure-Lösung in 1,4-Dioxan getropft. Nach Ende der Zugabe wird die Mischung auf Raumtemperatur erwärmt und 2 h gerührt, bevor die Mischung im Vakuum eingeengt wird. Das Rohprodukt kann direkt in der nächsten Stufe eingesetzt werden. Gegebenenfalls wird der Rückstand mit Diethylether behandelt und die ausgefallenen Kristalle werden abfiltriert und am Hochvakuum getrocknet.
Ausbeute: 2.99 g (86 % der Theorie).
MS (ES+): m/z (%) = 213 (M+H)⁺ (100).
HPLC (Methode 4): Rₜ = 0.41 min.

Analog können aus den entsprechenden *tert*.-Butoxycarbonylamino-Derivaten durch Behandlung mit Salzsäure in Dioxan folgende Amine in Form ihrer Hydrochloride dargestellt und direkt weiter umgesetzt werden:

| **Beispiel** | **Struktur** | **MW** | **MS** |
|---|---|---|---|
| 12A | 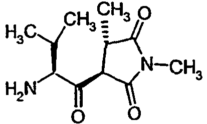 | 226.28 | MS(ES+), m/z (%): 227 (M+H)⁺(80) |
| 13A | 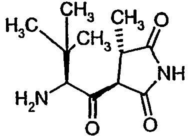 | 226.28 | MS (ES+), m/z (%): 227 (M+H)⁺ (100) |
| 14A | 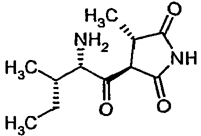 | 226.28 | |
| 15A | 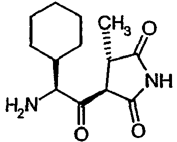 | 252.32 | |
| 16A | 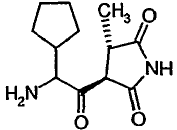 | 238.29 | |

### Allgemeine Vorschrift B: Darstellung der beta-Aminosäuremethylester

Die beta-Aminosäure [Synthese nach literaturbekannten Vorschriften (z. B. S. Rault, P. Dallemagne, M. Robba, Bull. Soc. Chim. Fr., 1987, 1079-1083; L. Lázár, T. Martinek, G. Bernáth, F. Fülöp, Synth. Comm., 1998, 28, 219-224)] wird in Methanol vorgelegt (ca. 0.5 bis 1.0 mol/l) und bei 0°C tropfenweise mit 1.2 eq Thionylchlorid versetzt. Nach beendeter Zugabe wird das Reaktionsgemisch über Nacht bei Raumtemperatur gerührt und anschließend eingeengt. Der Rückstand wird in wenig Methanol gelöst und mit Diethylether wird das Produkt ausgefällt. Der Feststoff wird abgesaugt, mehrfach mit Diethylether gewaschen und im Vakuum getrocknet.

Alternativ kann die Aufarbeitung auch folgendermaßen erfolgen: Nach Eindampfen zur Trockene wird der Rückstand in Wasser aufgenommen und zweimal mit Essigsäureethylester gewaschen. Die organische Phase wird verworfen, die wässrige Phase wird mit gesättigter Natriumhydrogencarbonat-Lösung neutralisiert und erneut dreimal mit Essigsäureethylester extrahiert. Die organischen Phasen der letzten Extraktion werden über Natriumsulfat oder Magnesiumsulfat getrocknet, dekantiert und zur Trockne eingedampft.

Nach der Allgemeinen Vorschrift B können folgende Verbindungen erhalten werden:

| **Beispiel** | **Struktur** | **MW** | **MS** |
|---|---|---|---|
| 17A | 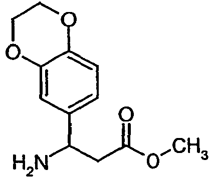 | 237.25 | MS (ES+), m/z (%): 238 (M+H)⁺ |
| 18A | 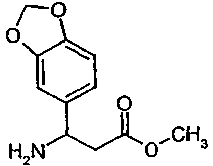 | 223.23 | MS (ES+), m/z (%): 224 (M+H)⁺ |

### Beispiel 19A

### 3-Amino-3-(3-chlorphenyl)propionsäuremethylester

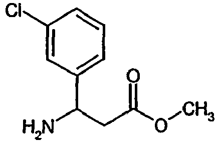

Methanol (110 ml) wird auf-10 °C gekühlt und langsam mit Thionylchlorid (12.0 g, 101.2 mmol) versetzt. 3-Amino-3-(3-chlorphenyl)propionsäure (10.1 g, 50.6 mmol) wird zugegeben und über Nacht bei Raumtemperatur gerührt. Die Lösung wird im Vakuum stark eingeengt und zwischen Ethylacetat (100 ml) und ges. Natriumhydrogencarbonat-Lösung (200 ml) verteilt. Dabei liegt der pH-Wert der wässrigen Phase oberhalb von pH 7. Die wässrige Phase wird erneut zweimal mit Ethylacetat (100 ml) extrahiert. Die vereinigten Ethylacetatphasen werden über Natriumsulfat getrocknet, filtriert und eingedampft.
Ausbeute: 9.7 g (87 %).
¹H-NMR (300 MHz, CDCl₃): δ = 7.37 (s, 1 H), 7.30-7.20 (m, 3 H), 4.40 (t, 1 H), 3.68 (s, 3 H), 2.67-2.60 (m, 2 H).

### Beispiel 20A

### (S)-3-Amino-3-phenylpropionsäuremethylester

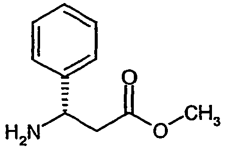

2.3 g (11.65 mmol) (*S*)-3-Amino-3-phenylpropionsäure werden in 100 ml Methanol vorgelegt und mit einer katalytischen Menge konzentrierter Schwefelsäure (0.02 eq.) versetzt. Das Reaktionsgemisch wird 24 h lang zum Rückfluss erhitzt und anschließend eingeengt. Das Rohprodukt kann ohne weitere Reinigung in der nächsten Stufe eingesetzt werden.
Ausbeute: 2.7 g (65 %).
¹H-NMR (300 MHz, d₆-DMSO): δ = 8.50 (s, 2 H), 7.52-7.37 (m, 5 H), 4.61 (t, 1 H), 3.58 (s, 3 H), 3.13 (dd, 1 H), 2.98 (dd, 1 H).
MS (ES+): m/z (%) =180 (M+H)⁺ (100).

### Allgemeine Vorschrift C: Umsetzung von 3-Amino-3-phenyl-propionsäure-alkylestern mit Carbonsäuren

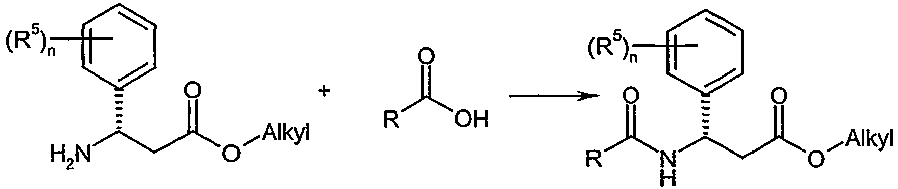

Die Carbonsäure (1.3 -1.5 eq) wird in Dichlormethan (ca. 0.1 mol/l) bei 0°C vorgelegt und mit 1.3 - 1.5 eq HATU versetzt. Zu dieser Mischung werden zunächst eine Lösung des 3-Amino-3-phenyl-propionsäure-alkylesters (1 eq.) in einem 1:1 Gemisch aus Dichlormethan und N,N-Dimethylformamid (ca. 0.1 mol/l) und anschließend über einen Zeitraum von 1h eine Lösung von Diisopropylethylamin (3.5 eq) in einem 1:1 Gemisch aus Dichlormethan und N,N-Dimethylformamid (ca. 1 mol/l) zugetropft. Es wird 30 min bei 0°C und anschließend über Nacht bei Raumtemperatur gerührt. Dann wird das Reaktionsgemisch eingeengt und mittels RP-HPLC (Eluent: Wasser-Acetonitril, Gradient) gereinigt.

Alternativ kann die Umsetzung auch nach folgendem Verfahren erfolgen:
Zu einer Lösung des 3-Aminopropionsäurealkylesters (1 eq.) in absolutem Dichlormethan oder einer Mischung (5:1 bis 1:1) von absolutem Dichlormethan und N,N-Dimethylformamid (ca. 0.1 bis 0.3 mol/l) werden das Carbonsäurederivat (1.1 - 1.5 eq.), Triethylamin (3 eq.), HOBt (3 eq.) und abschliessend 1.2 eq. EDC gegeben. Die Reaktionsmischung wird bei Raumtemperatur gerührt (2 h bis über Nacht), bevor im Vakuum eingeengt wird. Der Rückstand wird in Essigsäureethylester oder Dichlormethan aufgenommen und die organische Phase wird mit Wasser, gesättigter Natriumhydrogencarbonatlösung und gesättigter Kochsalzlösung gewaschen, über Natriumsulfat getrocknet, filtriert und eingeengt. Das Produkt kann durch Chromatographie an Silicagel (Eluenten: Gemische aus Cyclohexan/ Essigsäureethylester oder Gemische aus Dichlormethan und Ethanol) oder durch RP-HPLC (Eluenten: variable Gradienten aus Wasser und Acetonitril), alternativ durch eine Kombination beider Verfahren, gereinigt werden.

### Beispiel 21A

### (3S)-3-Phenyl-3-[(2-pyridinylcarbonyl)amino)propionsäuremethylester

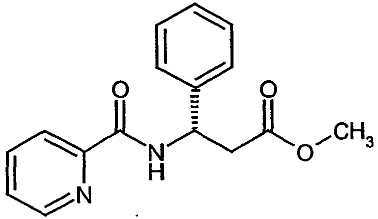

Synthese nach Allgemeiner Vorschrift C.
¹H-NMR (300 MHz, d₆-DMSO): δ= 9.37 (d, 1 H), 8.67 (d, 1 H), 8.05-7.95 (m, 2 H), 7.66-7.57 (m, 1 H), 7.48-7.19 (m, 5 H), 5.49 (br. q, 1 H), 3.55 (s, 3 H), 3.19 (dd, 1 H), 2.96 (dd, 1 H).
MS (ES+): m/z (%) = 285 (M+H)⁺ (35).
HPLC (Methode 5): Rₜ = 3.63 min.

### Beispiel 22A

### (3S)-3-{[(6-Fluor-2-pyridinyl)carbonyl]aminol}-3-phenylpropionsäuremethylester

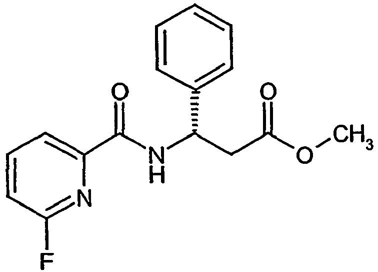

Synthese nach Allgemeiner Vorschrift C.
¹H-NMR (300 MHz, d₆-DMSO): δ = 9.23 (d, 1 H), 8.21-8.15 (m, 1 H), 7.96-7.92 (m, 1 H), 7.45-7.20 (m, 6 H), 5.46 (q, 1 H), 3.55 (s, 3 H), 3.18 (dd, 1 H), 2.96 (dd, 1 H). MS (ES+): m/z (%) = 325 (M+Na)⁺ (60).
HPLC (Methode 14): Rₜ = 4.35 min.

Nach der Allgemeinen Vorschrift C können folgende Verbindungen erhalten werden:

| **Beispiel** | **Struktur** | **MW** | **MS** |
|---|---|---|---|
| 23A | 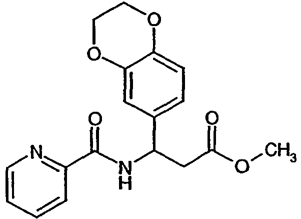 | 342.35 | MS (ES+), m/z (%): 343 (M+H)⁺ |
| 24A | 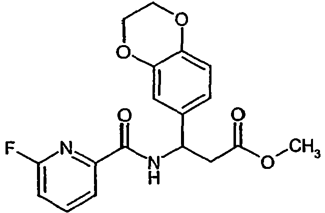 | 360.34 | MS (ES+), m/z (%): 361 (M+H)⁺ |
| 25A | 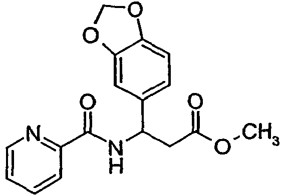 | 328.32 | MS (ES+), m/z (%): 329 (M+H)⁺ |
| 26A | 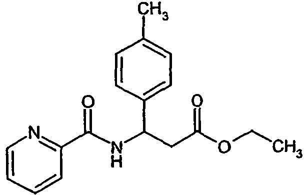 | 312.37 | MS(ES+), m/z (%): 313 (M+H)⁺ |

### Allgemeine Vorschrift D: Verseifung der Propionsäurealkylester

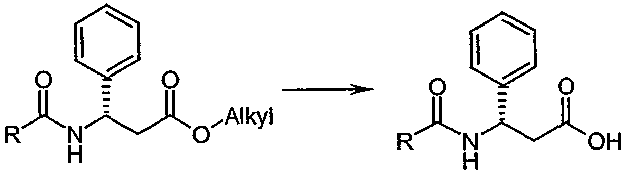

Der Propionsäurealkylester wird in einem 3:1 Gemisch aus Ethanol und Wasser vorgelegt (ca. 0.1 - 0.15 mol/l) und mit 5 eq 40 %iger Natronlauge versetzt. Das Reaktionsgemisch wird 24 h bei Raumtemperatur gerührt, mit verdünnter Salzsäure angesäuert (ca. pH 3) und eingeengt. Der Rückstand wird in Essigsäureethylester aufgenommen und mit gesättigter wässriger Natriumchloridlösung gewaschen. Die organische Phase wird über Magnesiumsulfat getrocknet, filtriert und eingeengt. Das erhaltene Produkt kann ohne weitere Reinigung in der nächsten Stufe eingesetzt werden.

### Alternativ kann auch folgende Methode Verwendung finden:

Der Propionsäurealkylester wird in einem 1:1 Gemisch aus Dioxan und Wasser vorgelegt (ca. 0.1 - 0.15 mol/l) und mit 3 eq einer Lösung von Kaliuhydroxid in Methanol (100 mg/ml) versetzt. Das Reaktionsgemisch wird 2 h bei Raumtemperatur gerührt und anschließend eingeengt. Der Rückstand wird in Wasser aufgenommen und mit verdünnter Salzsäure angesäuert. Die wässrige Phase wird dreimal mit einem 1:1 1 Gemisch aus Dichlormethan und Essigsäureethylester extrahiert. Die vereinigten organischen Phasen werden über Natriumsulfat getrocknet, filtriert und eingeengt. Das erhaltene Produkt kann ohne weitere Reinigung in der nächsten Stufe eingesetzt werden.

### Beispiel 27A

### (3S)-3-Phenyl-3-[(2-pyridnylcarbonyl)amino]propionsäure

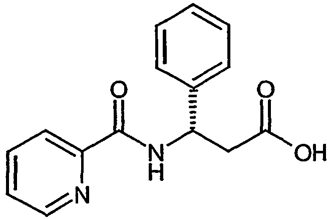

Synthese nach Allgemeiner Vorschrift D.
¹H-NMR (300 MHz, d₆-DMSO): δ = 12.22 (s, 1 H), 9.30 (d, 1 H), 8.68 (d, 1 H), 8.05-7.95 (m, 2 H), -7.66-7.58 (m, 1 H), 7.46-7.19 (m, 5 H), 5.45 (q, 1 H), 3.04 (dd, 1 H), 2.87 (dd, 1 H).
MS (ES-): m/z (%) = 269 (M-H)- (100).
HPLC (Methode 5): Rₜ = 3.12 min.

### Beispiel 28A

### (3S)-3-{[(6-Fluor-2-pyridinyl)carbonyl]amino}-3-phenylpropionsäure

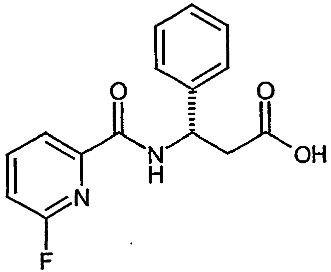

Synthese nach Allgemeiner Vorschrift D.
MS (ES-): m/z (%) = 287 (M-H)⁻ (100).
HPLC (Methode 13): Rₜ = 3.61 min.

Nach der Allgemeinen Vorschrift D können folgende Verbindungen erhalten werden:

| **Beispiel** | **Struktur** | **MW** | **MS** | **HPLC** |
|---|---|---|---|---|
| 29A | 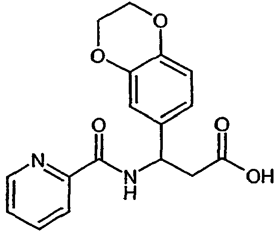 | 328.32 | MS (ES+), m/z (%): 329 (M+H)⁺ | |
| 30A | 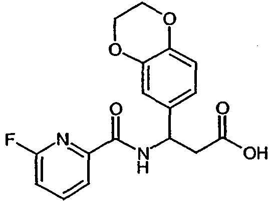 | 346.31 | MS (ES-), m/z(%): 345 (M-H)⁻ | HPLC (Methode 9): Rₜ = 3.69 min |
| 31A | 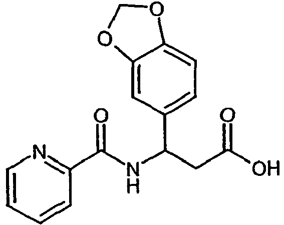 | 314.3 | MS (ES+), m/z (%): 315 (M+H) ⁺ | |
| 32A | 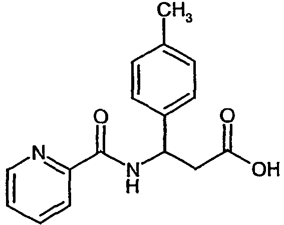 | 284.31 | MS (ES+), m/z (%): 285 (M+H)⁺ | HPLC (Methode 9): Rₜ = 3.82 min |

Die so erhaltenen Propionsäurederivate können nach der unten beschriebenen Vorschrift (Umsetzung von 3-[2-Amino-alkanoyl]-2,5-pyrrolidindion Hydrochlorid Derivaten mit Carbonsäurederivaten) umgesetzt werden.

### Allegemeine Vorschrift E: Darstellung von N-tert-butoxycarbonyl-geschützten beta-Aminosäuren

Die beta-Aminosäure (1 eq.) [Synthese nach literaturbekannten Vorschriften (z. B. S. Rault, P. Dallemagne, M. Robba, Bull. Soc. Chim. Fr., 1987, 1079-1083; L. Lázár, T. Martinek, G. Bernáth, F. Fülöp, Synth. Comm., 1998, 28, 219-224)] wird in Wasser vorgelegt (Konzentration ca. 0.3 - 1 mol/l) und mit Triethylamin (1.5 - 3 eq.) versetzt. Dann wird eine Lösung von 2-(*tert*-Butoxycarbonyloximino)-phenylacetonitril (1.1 eq.) in Dioxan (0.3 - 1 mol/l) zugegeben. Das Reaktionsgemisch wird 3 h bei Raumtemperatur gerührt, mit Wasser verdünnt und mit Diethylether gewaschen. Die wässrige Phase wird mit 5 %iger Zitronensäure angesäuert (ca. pH 2) und dreimal mit Essigsäureethylester extrahiert. Die vereinigten organischen Phasen werden mit gesättigter Kochsalzlösung gewaschen, über Natriumsulfat getrocknet, filtriert und eingeengt. Das Rohprodukt kann gegebenenfalls aus Essigsäureethylester/n-Hexan umkristallisiert werden.

Nach der Allgemeinen Vorschrift E können folgende Verbindungen erhalten werden:

| **Beispiel** | **Struktur** | **MW** | **MS** | **HPLC** |
|---|---|---|---|---|
| 33A | 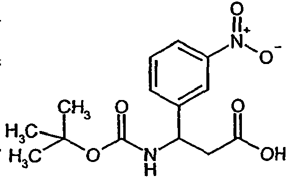 | 310.3 | MS (ES+), m/z (%): 311 (M+H)⁺ | HPLC (Methode 8): Rₜ = 3.87 min |
| 34A | 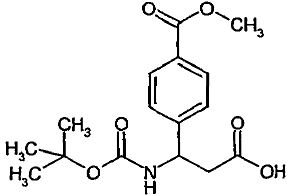 | 323.34 | MS (ES+), m/z (%): 324 (M+H)⁺ | HPLC (Methode 8): Rt = 2.39 min |
| 35A | 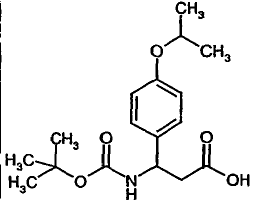 | 323.39 | MS (ES-), m/z (%): 322 (M-H)⁻ | HPLC (Methode 14): Rₜ = 4.35 min |

### Beispiel 36A

### (3S)-3-[(tert-Butoxycarbonyl)amino]-3-phenylpropionsäure

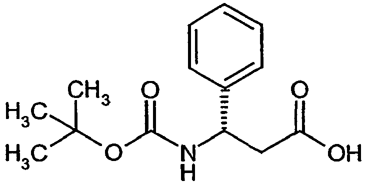

2.82 g (17 mmol) (*S*)-3-Amino-3-phenylpropionsäure werden in 60 ml Dioxan aufgeschlemmt und bei 0°C mit 4.1 g (18.8 mmol) Di-tert-butyl-dicarbonat (Boc-Anhydrid) und 43 ml einer 1N Natriumhydroxidlösung in Wasser versetzt. Das Reaktionsgemisch wird noch 30 min bei 0°C und dann 3 h bei Raumtemperatur gerührt. Anschließend wird das Reaktionsgemisch eingeengt und der Rückstand wird in Methylenchlorid aufgenommen. Die organische Phase wird mit 1N Salzsäure und gesättigter Kochsalzlösung gewaschen, über Magnesiumsulfat getrocknet und eingeengt. Das Rohprodukt (3.12 g) kann ohne weitere Reinigung weiter umgesetzt werden.
MS (ES-): m/z (%) = 264 (M-H)⁻ (100).
HPLC (Methode 14): Rₜ = 3.89 min.

### Allgemeine Vorschrift F: Acylierung von 3-[2-Amino-alkanoyl]-2,5-pyrrolidindion Hydrochlorid Derivaten mit Carbonsäurederivaten

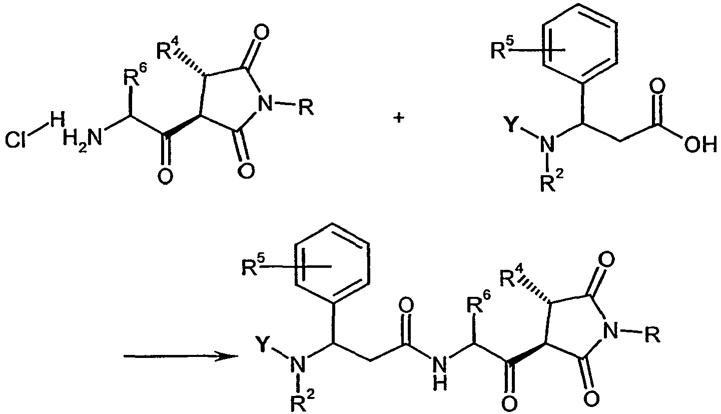

Zu einer Lösung des Carbonsäurederivates (1.2 - 1.5 eq.) in absolutem Dichlormethan oder einer Mischung (5:1 bis 1:1) von absolutem Dichlormethan und N,N-Dimethylformamid (ca. 0.1 bis 0.3 mol/l) werden bei 0°C zunächst eine äquimolare Menge HATU und dann das 3-[2-Amino-alkanoyl]-2,5-pyrrolidindion Hydrochlorid Derivat (1 eq., gegebenenfalls als Lösung in N,N-Dimethylformamid oder Dichlormethan/N,N-Dimethylformamid Gemischen) zugegeben. Anschließend wird bei 0°C eine Lösung von 2.5 - 3.5 eq. Diisopropylethylamin in einem 1:1 Gemisch von absolutem Dichlormethan und N,N-Dimethylformamid (0.2 - 1 mol/l) über einen Zeitraum von 1 h zugetropft. Nach Ende der Zugabe wird die Reaktionsmischung weitere 30 min bei 0°C und anschliessend über Nacht bei Raumtemperatur gerührt, bevor im Vakuum eingeengt wird. Das Produkt kann durch Chromatographie an Silicagel (Eluenten: Gemische aus Cyclohexan/Ethylacetat oder Gemische aus Dichlormethan und Ethanol) oder durch RP-HPLC (Eluenten: variable Gradienten aus Wasser und Acetonitril), alternativ durch eine Kombination beider Verfahren, erhalten werden.

### Alternativ kann die Umsetzung auch nach folgendem Verfahren erfolgen:

Zu einer Lösung des 3-[2-Amino-alkanoyl]-2,5₋pyrrolidindion Hydrochlorid Derivates (1 eq.) in absolutem Dichlormethan oder einer Mischung (5:1 bis 1:1) von absolutem Dichlormethan und N,N-Dimethylformamid (ca. 0.1 bis 0.3 mol/l) werden das Carbonsäurederivat (1.1 - 1.5 eq.), Triethylamin (3 eq.), HOBt (3 eq.) und abschließend 1.2 eq. EDC gegeben. Die Reaktionsmischung wird bei Raumtemperatur gerührt (2 h bis über Nacht), bevor im Vakuum eingeengt wird. Der Rückstand wird in Essigsäureethylester oder Dichlormethan aufgenommen und die organische Phase wird mit Wasser, gesättigter Natriumhydrogencarbonatlösung und gesättigter Kochsalzlösung gewaschen, über Natriumsulfat getrocknet, filtriert und eingeengt. Das Produkt kann durch Chromatographie an Silicagel (Eluenten: Gemische aus Cyclohexan/Ethylacetat oder Gemische aus Dichlormethan und Ethanol) oder durch RP-HPLC (Eluenten: variable Gradienten aus Wasser und Acetonitril), alternativ durch eine Kombination beider Verfahren, gereinigt werden.

### Beispiel 37A

### ((S)-2- {(S)-2-Methyl-1-[1-((3R,4S)-4-methyl-2,5-dioxo-pyrrolidin-3-yl)-methanoyl]-propylcarbamoyl}-1-phenyl-ethyl)-carbarmidsäure-tert-butylester

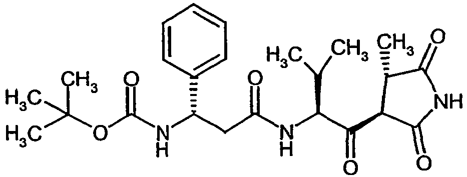

Synthese nach Allgemeiner Vorschrift F.
¹H-NMR (400 MHz, d₆-DMSO): δ = 11.45 (s, 1 H), 7.98 (d, 1 H), 7.31-7.24 (m, 5 H), 7.20 (br. s, 1 H), 4.88-4.82 (br. s, 1 H), 4.69 (br. s, 1 H), 3.98 (d, 1 H), 2.95-2.89 (m, 1 H), 2.77-2.69 (m, 1 H), 2.51-2.44 (m, 1 H), 2.35-2.29 (m, 1 H), 1.10 (d, 3 H), 0.85 (d, 3 H), 0.78 (d, 3 H).
MS (ES+): m/z (%) = 460 (M+H)⁺ (100).
HPLC (Methode 6): Rₜ = 3.90 min.

Nach der Allgemeinen Vorschrift F können folgende Verbindungen erhalten werden:

| **Beispiel** | **Struktur** | **MW** | **MS** | **HPLC** |
|---|---|---|---|---|
| 38A | 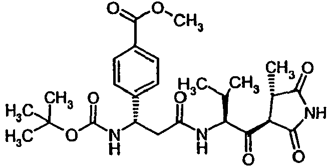 | 517.58 | MS (ES+), m/z (%): 518 (M+H)⁺ | HPLC (Methode 8): Rₜ = 2.60 min |
| 39A | 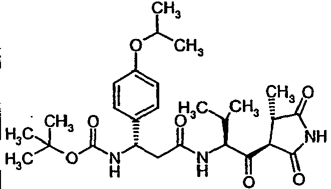 | 517.62 | MS (ES+), m/z (%): 518 (M+H)⁺ | HPLC (Methode 14): Rₜ = 4.42 min |
| 40A | 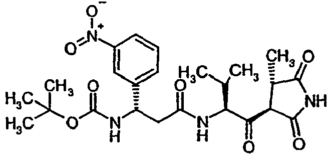 | 504.54 | MS (ES-), m/z (%): 503 (M-H)⁻ | HPLC (Methode 6): Rₜ = 3.99 min |

### Allgemeine Vorschrift G: Deblockierung von Boc-geschützten Derivaten

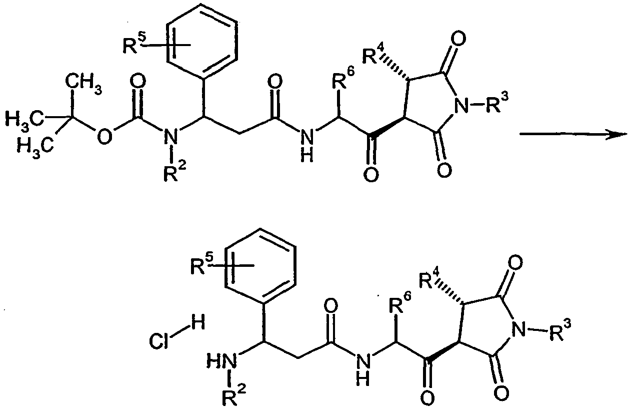

Das *tert*.-Butyloxycarbonyl (BOC) geschützte Aminderivat (gegebenenfalls als Lösung in Dioxan) wird bei 0°C oder Raumtemperatur mit 4N Salzsäure-Lösung in 1,4-Dioxan versetzt (ca. 0.1 mol/l) und 2 - 24 h bei Raumtemperatur gerührt, bevor im Vakuum eingeengt wird. Der Rückstand kann ohne weitere Reinigung weiter umgesetzt werden oder wird gegebenenfalls mit Dichlormethan und Diethylether behandelt. Die ausgefallenen Kristalle werden abgesaugt und am Hochvakuum getrocknet. Man erhält das Produkt als Hydrochlorid.

### Beispiel 41A

### (S)-3-Amino- {(S)-2-methyl-1-[1-((3R,4S)-4-methyl-2,5-dioxo-pyrrolidin-3-yl)-methanoyl]-propyl} - -3-phenylpropionamid Hydrochlorid

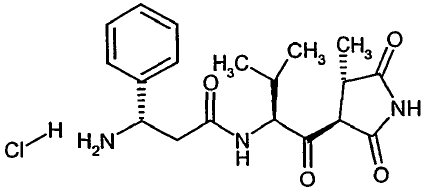

Synthese nach Allgemeiner Vorschrift G.
¹H-NMR (200 MHz, d₆-DMSO): δ = 11.49 (br. s, 1 H), 8.5 (br. s, ca. 3 H), 7.54-7.32 (m, 5 H), 4.69-4.55 (m, 2 H), 3.89 (d, 1 H), 3.06-2.80 (m, 3 H), 2.39-2.25 (m, 1 H), 1.01 (d, 3 H), 0.81 (d, 3 H), 0.75 (d, 3 H).
MS (ES+): m/z (%) = 360 (M+H)⁺ (100).
HPLC (Methode 4): Rₜ = 1.44 min.

Nach der Allgemeinen Vorschrift G können folgende Verbindungen erhalten werden:

| **Beispiel** | **Struktur** | **MW** | **MS** | **HPLC** |
|---|---|---|---|---|
| 42A | 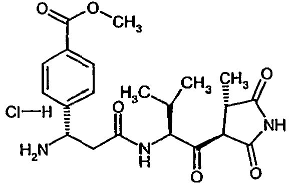 | 417.46 | MS (ES-), m/z (%): 416 (M-H)⁻ | HPLC (Methode 5): Rₜ = 2.23 min |
| 43A | 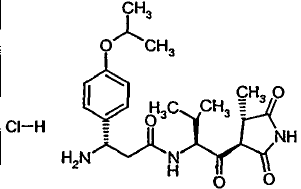 | 417.51 | MS (ES-), m/z (%): 416 (M-H)⁻ | HPLC (Methode 9): Rₜ = 2.97 min |
| 44A | 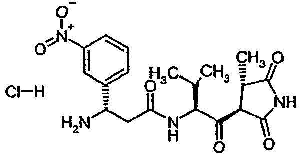 | 404.42 | | |

Analog zu Beispiel 1A werden folgende Verbindungen durch Umsetzung von (3*S*)-3-Methyldihydro-2,5-furandion mit den entsprechenden primären Aminen, Hydroxylamin- oder Hydrazinderivaten erhalten. Die Rohprodukte können durch RP-HPLC (Eluent: Wasser-Acetonitril, Gradient) gereinigt werden.

| **Beispiel** | **Struktur** | **MW** | **MS** | **HPLC** |
|---|---|---|---|---|
| 45A | 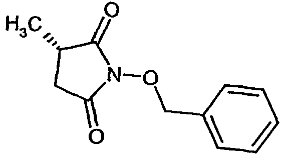 | 219.24 | | HPLC (Methode 6): Rₜ = 3.37 min |
| 46A | 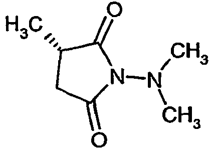 | 156.18 | MS (ES+), m/z: 157 (M+H)⁺ | HPLC (Methode 19): Rₜ = 2.62 min |
| 47A | 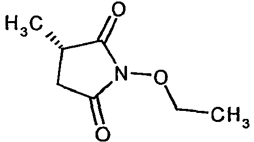 | 157.17 | MS(DCI), m/z: 175 (M+NH4) ⁺ | HPLC (Methode 20): Rₜ= 1.70 min |
| 48A | 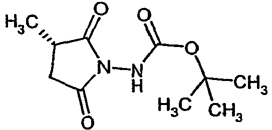 | 228.25 | MS (DCl), m/z: 246 (M+NH₄)⁺ | HPLC (Methode 20): Rₜ= 2.09 min |
| 49A | 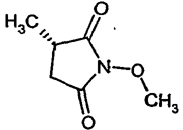 | 143.14 | MS (ES+), m/z: 144 (M+H)⁺ | |
| 50A | 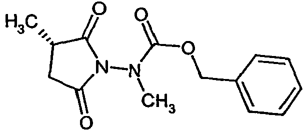 | 276.29 | MS (DCI), m/z: 294 (M+NH₄)⁺ | HPLC (Methode 21): Rₜ = 2.80 min |

### Allgemeine Vorschrift J: Umsetzung von N-tert.-Butoxycarbonyl-geschützten Aminosäuren mit 2,5-Pyrrolidindion Derivaten

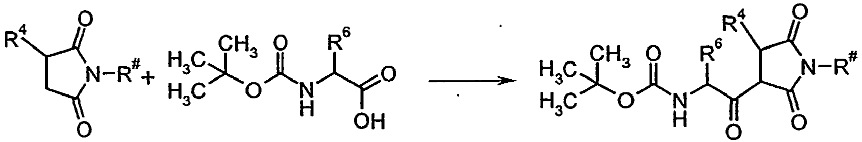

Die N-*tert*.-Butoxycarbonyl-geschützte Aminosäure (1 eq.) und N,N-Carbonyldiimidazol (1.1 eq.) werden in Tetrahydrofuran (ca. 0.1 - 1 mol/l) 2 h lang bei Raumtemperatur gerührt. Zu diesem Gemisch wird dann das 2,5-Pyrrolidindion (1 eq.) gegeben und die gesamte Mischung wird innerhalb von 30 min zu einer auf -65°C gekühlten 1 molaren Lösung von Lithium-hexamethyldisilazid (2 eq.) in THF getropft. Nach beendeter Zugabe wird weitere 15 min bei -65°C gerührt, bevor gesättigte wässrige Ammoniumchlorid-Lösung zugegeben wird. Nach Erwärmung auf Raumtemperatur wird das Reaktionsgemisch mit Diethylether verdünnt und die organische Phase mit gesättigter wässriger Natriumchlorid-Lösung gewaschen, über Magnesiumsulfat getrocknet, filtriert und anschließend eingeengt. Das Rohprodukt wird durch RP-HPLC (Eluent: Wasser-Acetonitril, Gradient) gereinigt.

Nach der Allgemeinen Vorschrift J können durch Umsetzung der entsprechenden N-*tert*.-Butoxycarbonyl-geschützten Aminosäuren (bzgl. der Darstellung von unnatürlichen alpha-Aminosäuren siehe z. B.: A. A. Cordi et al., J. Med. Chem. 2001, 44, 787-805; K. Mai, G. Patil, Tetrahedron Lett. 1984, 25, 4583-4586; N. A. Hassan, E. Bayer, J. C. Jochims, J. Chem. Soc., Perkin Trans. 1 1998, 3747-3757; bzgl. der *tert*.-Butoxycarbonyl-Schützung siehe z. B.: T. W. Greene, P. G. M. Wuts, Protective Groups in Organic Synthesis, 3rd Edt. 1999, J. Wiley & Sons, Inc.) mit 2,5-Pyrrolidindionen folgende Derivate erhalten werden:

| **Beispiel** | **Struktur** | **MW** | **MS** | **HPLC** |
|---|---|---|---|---|
| 51A | 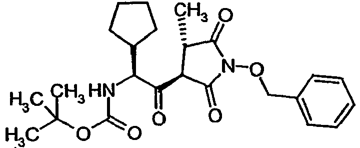 | 444.53 | MS (ES-), m/z: 443 (M-H)⁻ | HPLC (Methode 18): Rₜ = 4.11 min |
| 52A | 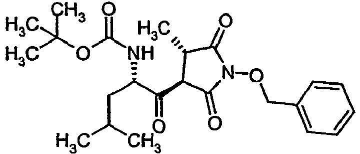 | 432.51 | MS (ES-), m/z: 431 (M-H)⁻ | HPLC (Methode 6): Rₜ = 4.88 min |
| 53A | 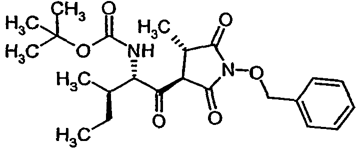 | 432.51 | MS (ES-), m/z: 431 (M-H)⁻ | HPLC (Methode 13): Rₜ = 5.08 min |
| 54A | 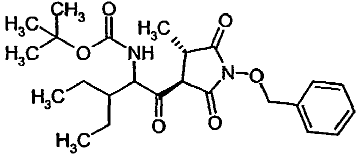 | 446.54 | MS (ES-), m/z: 445 (M-H)⁻ | HPLC (Methode 17): Rₜ = 4.42 min |
| 55A | 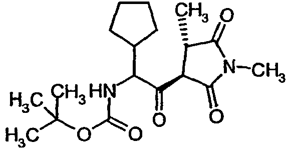 | 352.43 | MS (ES-), m/z: 351 (M-H)⁻ | HPLC (Methode 14): Rₜ = 4.61 min |
| 56A | 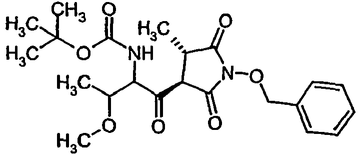 | 434.49 | MS (ES-), m/z: 433 (M-H)⁻ | HPLC (Methode 17): Rₜ = 3.98 min |
| 57A | 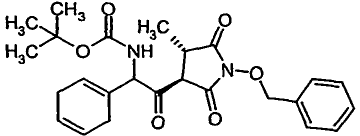 | 454.52 | MS (ES-), m/z: 453 (M-H)⁻ | HPLC (Methode 22): Rₜ = 4.41 min |
| 58A | 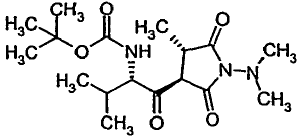 | 355.43 | MS (ES+), m/z: 378 (M+Na)⁺ | HPLC (Methode 19): Rₜ = 4.12 min |
| 59A | 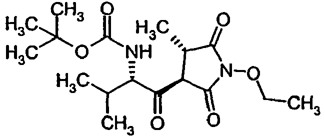 | 356.42 | MS (ES-), m/z: 355 (M-H)⁻ | HPLC (Methode 20): Rₜ = 3.64 min |
| 60A | 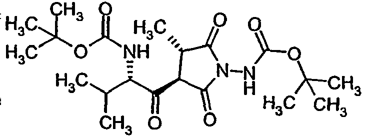 | 427.50 | MS (ES-), m/z: 426 (M-H)⁻ | HPLC (Methode 20): Rₜ = 3.73 min |
| 61A | 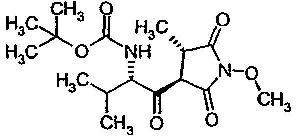 | 342.39 | MS (ES-), m/z: 341 (M-H)⁻ | HPLC (Methode 19): Rₜ = 4.18 min |
| 62A | 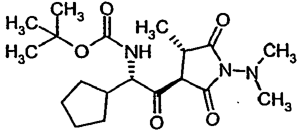 | 381.47 | MS (ES-), m/z: 380 (M-H)⁻ | HPLC (Methode 21): Rₜ = 3.43 min |
| 63A | 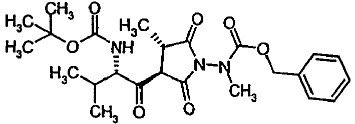 | 475.54 | MS (ES-), m/z: 474 (M-H)⁻ | HPLC (Methode 20): Rₜ = 3.91 min |
| 64A | 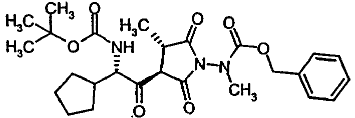 | 501.58 | MS (ES-), m/z: 500 (M-H)⁻ | HPLC (Methode 21): Rₜ = 3.93 min |
| 65A | 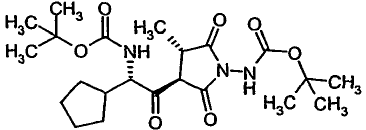 | 453.53 | MS (ES-), m/z: 452 (M-H)⁻ | HPLC (Methode 21): Rₜ = 3.61 min |
| 66A | 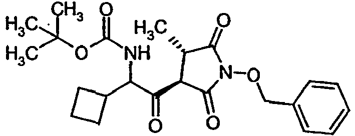 | 430.51 | MS (ES+), m/z: 453 (M+Na)⁺ | HPLC (Methode 20): Rₜ = 4.32 min |

Nach der Allgemeinen Vorschrift A können folgende Verbindungen erhalten werden:

| **Beispiel** | **Struktur** | **MW** | **MS** | **HPLC** |
|---|---|---|---|---|
| 67A | 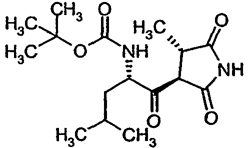 | 326.39 | MS (ES-), m/z: 325 (M-H)⁻ | HPLC (Methode 5): Rₜ = 3.91 min |
| 68A | 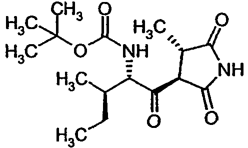 | 326.39 | MS (ES-), m/z: 325 (M-H)⁻ | HPLC (Methode 12): Rₜ = 2.88 min |
| 69A | 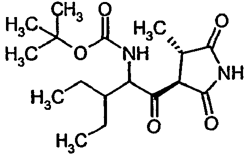 | 340.42 | MS (ES-), m/z: 339 (M-H)⁻ | HPLC (Methode 17): Rₜ = 3.59 min |
| 70A | 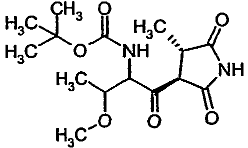 | 328.26 | MS (ES+), m/z: 351 (M+Na)⁺ | HPLC (Methode 20): Rₜ = 3.18 min |
| 71A | 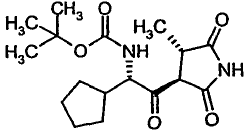 | 338.40 | MS (ES-), m/z: 337 (M-H)⁻ | HPLC (Methode 17): Rₜ = 3.57 min |
| 72A | 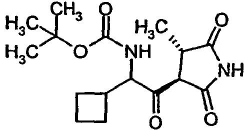 | 324.38 | MS (ES-), m/z: 323 (M-H)⁻ | HPLC (Methode 19): Rₜ = 4.02 min |
| 73A | 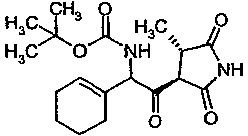 | 350.41 | MS (ES-), m/z: 349 (M-H)⁻ | HPLC (Methode 18): Rₜ = 3.66 min |

Die Verbindung 73A ist bei der Umsetzung von Verbindung 57A entstanden.

### Allgemeine Vorschrift K: Deblockierung von Benzyloxycarbonyl-geschützten Hydrazin-Derivaten

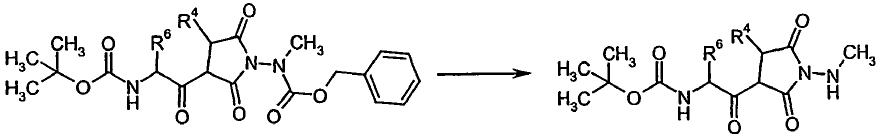

Das Benzyloxycarbonyl-geschützte Hydrazin-Derivat (1 eq.) wird in Methanol oder Ethanol gelöst (ca. 0.05 mol/l), mit einer katalytischen Menge Palladium-Kohle (10 %) versetzt und 3 - 4 h unter Wasserstoffatmosphäre (Normaldruck) gerührt. Dann wird das Reaktionsgemisch filtriert und eingeengt. Das Rohprodukt kann ohne weitere Reinigung umgesetzt werden.

Nach der Allgemeinen Vorschrift K können folgende Verbindungen erhalten werden:

| **Beispiel** | **Struktur** | **MW** | **MS** | **HPLC** |
|---|---|---|---|---|
| 74A | 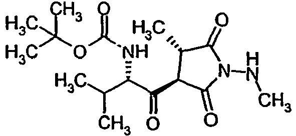 | 341.41 | MS (ES-), m/z: 340 (M-H)⁻ | HPLC (Methode 19): Rₜ = 4.00 min |
| 75A | 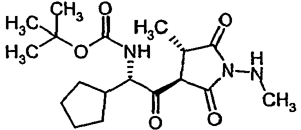 | 367.44 | MS (ES-), m/z: 366 (M-H)⁻ | HPLC (Methode 20): Rₜ = 3.47 min |

### Allgemeine Vorschrift L: Deblockierung von Boc-geschützten Derivaten

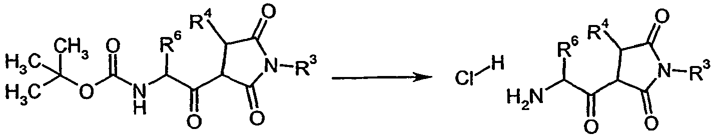

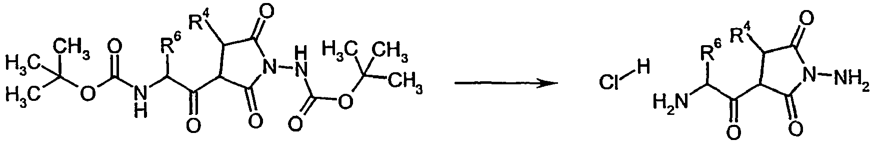

Das *tert*.-Butyloxycarbonyl (BOC) geschützte Aminderivat (gegebenenfalls als Lösung in Dioxan) wird mit 4N Salzsäure-Lösung in 1,4-Dioxan versetzt (ca. 0.1 mol/l) und 2 - 24 h bei Raumtemperatur gerührt, bevor im Vakuum eingeengt wird. Der Rückstand kann ohne weitere Reinigung weiter umgesetzt werden oder wird gegebenenfalls mit Dichlormethan und Diethylether behandelt. Die ausgefallenen Kristalle werden abgesaugt und am Hochvakuum getrocknet. Man erhält das Produkt als Hydrochlorid.

Nach der Allgemeinen Vorschrift L können folgende Verbindungen erhalten werden:

| **Beispiel** | **Struktur** | **MW** |
|---|---|---|
| 76A | 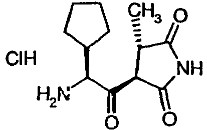 | 238.29 |
| 77A | 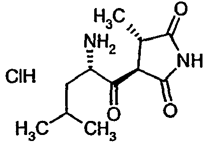 | 226.28 |
| 78A | 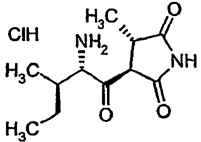 | 226.28 |
| 79A | 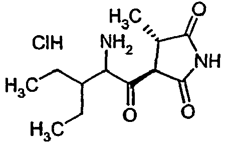 | 240.30 |
| 80A | 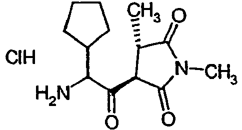 | 252.32 |
| 81A | 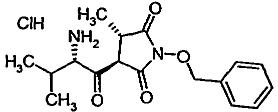 | 318.38 |
| 82A | 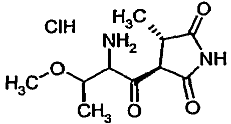 | 228.25 |
| 83A | 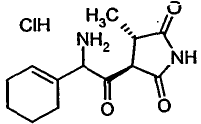 | 250.30 |
| 84A | 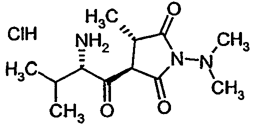 | 255.32 |
| 85A | 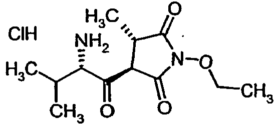 | 256.30 |
| 86A | 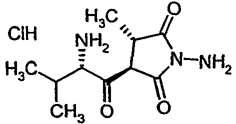 | 227.27 |
| 87A | 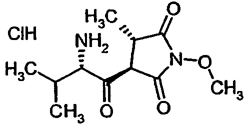 | 242.28 |
| 88A | 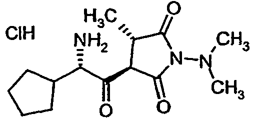 | 281.36 |
| 89A | 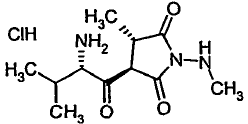 | 241.29 |
| 90A | 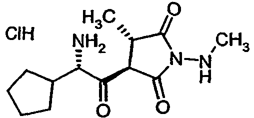 | 267.33 |
| 91A | 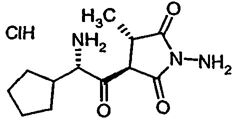 | 253.30 |
| 92A | 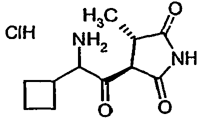 | 224.26 |

Gemäß der Allgemeinen Arbeitsvorschrift B können folgende Verbindungen hergestellt werden:

| **Beispiel** | **Struktur** | **MW** | **MS** | **HPLC** |
|---|---|---|---|---|
| 93A | 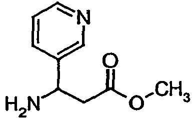 | 180.2 | MS (ES+), m/z: 180 (M+H)⁺ | |
| 94A | 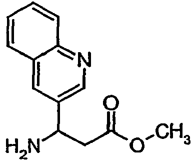 | 230.3 | MS (ES+), m/z: 231 (M+H)⁺ | HPLC (Methode 20): Rₜ = 1.70 min |
| 95A | 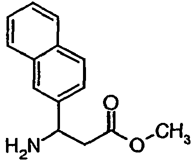 | 229.3 | MS (ES+), m/z: 230 (M+H)⁺ | HPLC (Methode 19): Rₜ = 1.75 min |
| 96A | 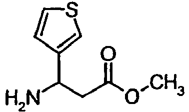 | 185.3 | MS (ES+), m/z: 186 (M+H)⁺ | |
| 97A | 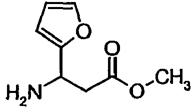 | 169.2 | MS (ES+), m/z: 170 (M+H)⁺ | |
| 98A | 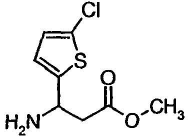 | 219.69 | MS (ES+), m/z: 220 (M+H)⁺ | |
| 99A | 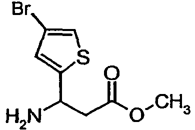 | 264.14 | MS (ES+), m/z: 264 (M+H)⁺ | |

Nach der Allgemeinen Vorschrift C können folgende Verbindungen erhalten werden:

| **Beispiel** | **Struktur** | **MW** | **MS** |
|---|---|---|---|
| 100A | 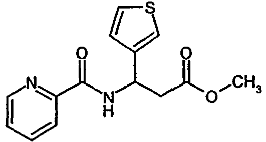 | 290.34 | MS(ES+), m/z (%):291 (M+H)⁺ |
| 101A | 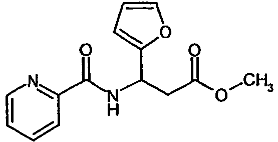 | 274.28 | MS (ES+), m/z (%): 275 (M+H)⁺ |
| 102A | 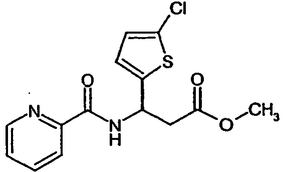 | 324.79 | MS (ES+), m/z (%): 325 (M+H)⁺ |
| 103A | 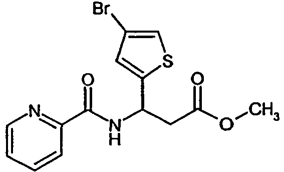 | 369.24 | MS (ES+), m/z (%): 369 (M+H)⁺ |

### Allgemeine Vorschrift M: Umsetzung von 3-Amino-propionsäure-alkylestern mit Carbonsäurechloriden

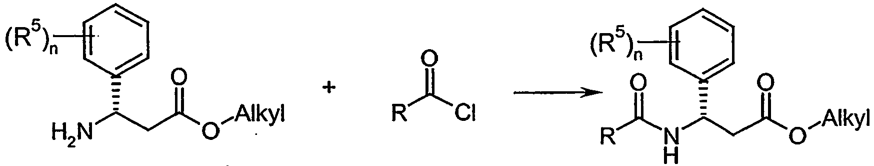

Der 3-Aminopropionsäurealkylester wird in Dichlormethan (ca. 0.1 - 0.4 mol/l) bei Raumtemperatur vorgelegt und mit 2 - 3 eq. Diisopropylethylamin und 1.2 eq. des Carbonsäurechlorids versetzt. Es wird 2 - 3 h bei Raumtemperatur gerührt. Dann wird Wasser zu dem Reaktionsgemisch gegeben, die organische Phase wird abgetrennt, über Natriumsulfat getrocknet, filtriert und eingeengt. Der Rückstand kann mit Dichlormethan und Diethylether umkristallisiert oder mittels Chromatographie an Kieselgel (Eluent: Gemische aus Dichlormethan und Essigsäureethylester) gereinigt werden.

Nach der Allgemeinen Vorschrift M können folgende Verbindungen erhalten werden:

| **Beispiel** | **Struktur** | **MW** | **MS** |
|---|---|---|---|
| 104A | 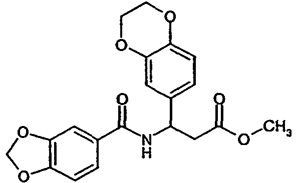 | 385.38 | MS (ES+), m/z (%): 386 (M+H)⁺ |
| 105A | 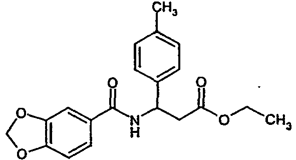 | 355.39 | Ms (ES+), m/z (%): 356 (M+H)⁺ |

Nach der Allgemeinen Vorschrift D können folgende Verbindungen erhalten werden:

| **Beispiel** | **Struktur** | **MW** | **MS** | **HPLC** |
|---|---|---|---|---|
| 106A | 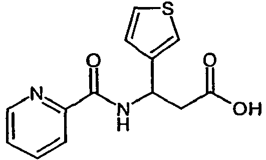 | 276.32 | MS (ES+), m/z (%): 277 (M+H)⁺ | HPLC (Methode 9): Rₜ = 3.55 min |
| 107A | 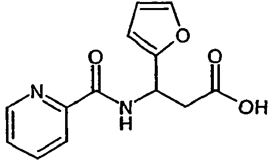 | 260.25 | MS (ES+), m/z (%): 261 (M+H)⁺ | HPLC (Methode 9): Rₜ = 3.43 min |
| 108A | 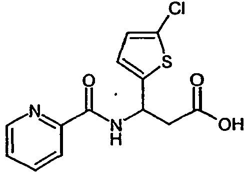 | 310.76 | MS (ES+), m/z (%): 311 (M+H)⁺ | HPLC (Methode 9): Rₜ = 3.92 min |
| 109A | 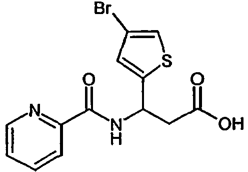 | 355.21 | MS (ES+), m/z (%): 355 (M+H)⁺ | HPLC (Methode 9): Rₜ = 3.89 min |
| 110A | 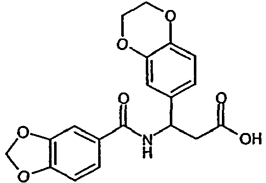 | 371.35 | MS (ES+), m/z (%): 372 (M+H)⁺ | HPLC (Methode 9): Rₜ = 3.64 min |
| 111A | 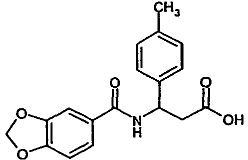 | 327.34 | MS (ES+), m/z (%): 328 (M+H)⁺ | |

Die so erhaltenen Propionsäurederivate können nach der unten beschriebenen Allgemeinen Vorschrift F (Acylierung von 3-[2-Amino-alkanoyl]-2,5-pyrrolidindion Hydrochlorid Derivaten mit Carbonsäurederivaten) umgesetzt werden.

Nach der Allgemeinen Vorschrift E können folgende Verbindungen erhalten werden:

| **Beispiel** | **Struktur** | **MW** | **MS** | **HPLC** |
|---|---|---|---|---|
| 112A | 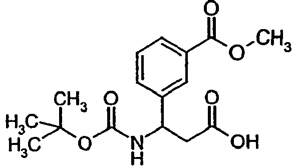 | 323.35 | | HPLC (Methode 9): Rₜ = 3.96 min |
| 113A | 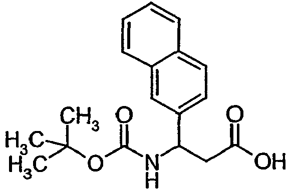 | 315.37 | MS (ESI-), m/z: 314 (M-H)⁻ | HPLC (Methode 21): Rₜ = 3.21 min |
| 114A | 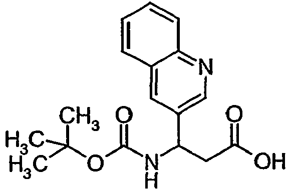 | 316.36 | MS (ESI+), m/z: 317 (M+H)⁺ | HPLC (Methode 19): Rₜ = 3.47 min |
| 115A | 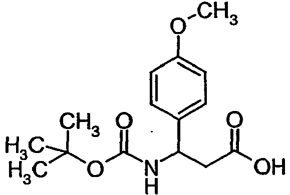 | 295.33 | MS (ESI+), m/z: 296 (M+H)⁺ | HPLC (Methode 20): Rₜ = 3.00 min |
| 116A | 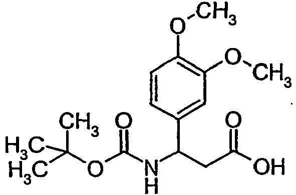 | 325.36 | | HPLC (Methode 9): Rₜ = 3.76 min |
| 117A | 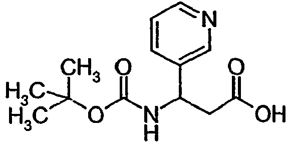 | 266.30 | MS (DCI), m/z: 167 (M-100+H)⁺ | HPLC (Methode 9): Rₜ = 1.92 min |
| 118A | 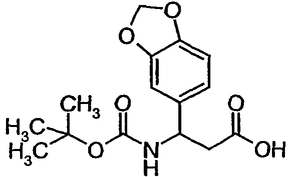 | 309.32 | MS (ESI-), m/z: 308 (M-H)⁻ | HPLC (Methode 13): Rₜ = 3.69 min |

Nach der Allgemeinen Vorschrift F können folgende Verbindungen erhalten werden:

| **Beispiel** | **Struktur** | **MW** | **MS** | **HPLC** |
|---|---|---|---|---|
| 119A | 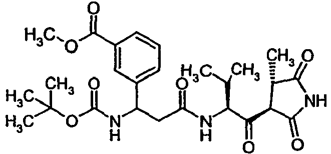 | 517.58 | MS (ESI+), m/z: 518 (M+H)⁺ | HPLC (Methode 16): Rₜ = 2.89 min |
| 120A | 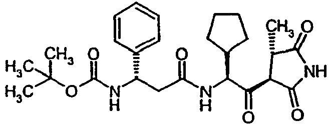 | 485.58 | MS (ESI-), m/z: 484 (M-H)⁻ | HPLC (Methode 20): ^{.} Rₜ = 3.72 min |
| 121A | 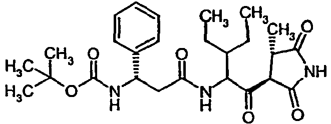 | 487.59 | MS (ESI+), m/z: 488 (M+H)⁺ | HPLC (Methode 17): Rₜ = 3.73 min |
| 122A | 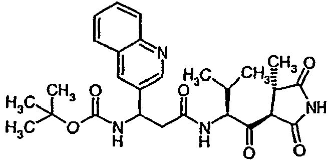 | 510.59 | MS (ESI-), m/z: 509 (M-H)⁻ | HPLC (Methode 19): Rₜ = 3.99 min |
| 123A | 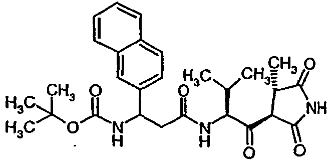 | 509.61 | MS (ESI-), m/z: 508 (M-H)⁻ | HPLC (Methode 20): Rₜ = 3.77 min |
| 124A | 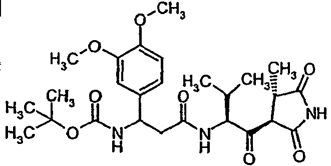 | 519.60 | MS (ESI-), m/z: 518 (M-H)⁻ | HPLC (Methode 18): Rₜ = 3.36 min |
| 125A | 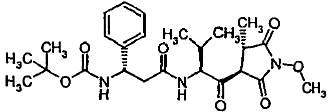 | 489.57 | MS (ESI-), m/z: 488 (M-H)⁻ | HPLC (Methode 19): Rₜ = 4.19 min |
| 126A | 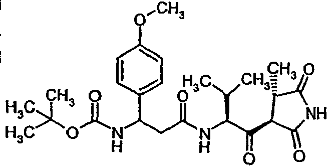 | 489.57 | MS (ESI-), m/z: 488 (M-H)⁻ | HPLC (Methode 20): Rₜ = 3.36 min |
| 127A | 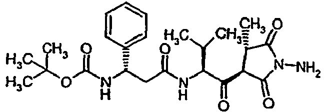 | 474.56 | MS (ESI-), m/z: 473 (M-H)⁻ | HPLC (Methode 19): Rₜ = 2.55 min |
| 128A | 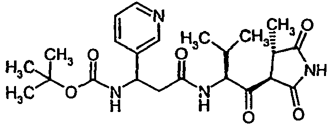 | 460.53 | MS (ESI+), m/z: 461 (M+H)⁺ | HPLC (Methode 5): Rₜ = 2.86 min |
| 129A | 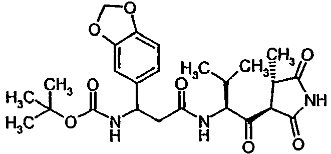 | 503.56 | MS (ESI+), m/z: 504 (M+H)⁺ | HPLC (Methode 14): Rₜ = 4.05 min |

Nach der Allgemeinen Vorschrift G können folgende Verbindungen erhalten werden:

| **Beispiel** | **Struktur** | **MW** | **MS** | **HPLC** |
|---|---|---|---|---|
| 130A | 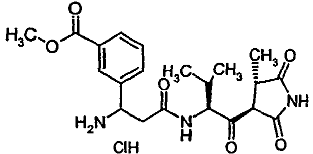 | 417.47 | | |
| 131A | 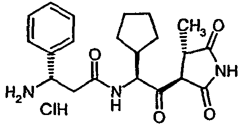 | 385.47 | MS (ESI-), m/z: 384 (M-H)⁻ | HPLC (Methode 20): Rₜ =1.90 min |
| 132A | 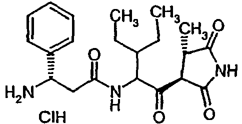 | 387.48 | | |
| 133A | 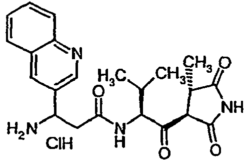 | 410.48 | | HPLC (Methode 9): Rₜ = 2.79 min |
| 134A | 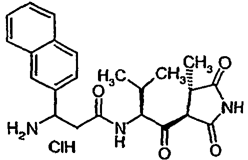 | 409.49 | MS (ESI-), m/z: 408 (M-H)⁻ | HPLC (Methode 20): Rₜ = 2.14 und 2.23 min |
| 135A | 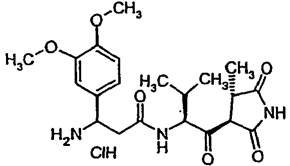 | 419.48 | | HPLC (Methode 9): Rₜ = 2.80 min |
| 136A | 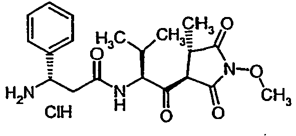 | 389.46 | MS (ESI-), m/z: 388 (M-H)⁻ | HPLC (Methode 19): Rₜ = 3.17 min |
| 137A | 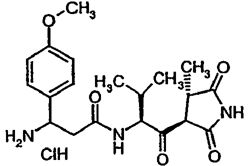 | 389.46 | | HPLC (Methode 9): Rₜ = 2.86 min |
| 138A | 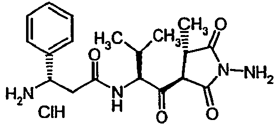 | 374.44 | | |
| 139A | 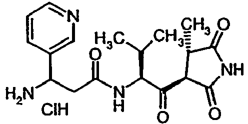 | 360.42 | MS (ESI+), m/z: 361 (M+H)⁺ | |
| 140A | 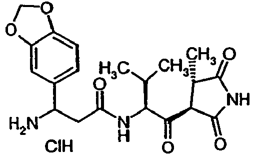 | 403.44 | MS (ESI-), m/z: 402 (M-H)⁻ | HPLC (Methode 14): Rₜ = 2.40 min |

### Herstellungsbeispiele:

### Allgemeine Vorschrift H: Acylierung von acylalkylamino substituierten 3-[2-Amino-alkanoyl]-2,5-pyrrolidindion Hydrochlorid Derivaten mit Carbonsäurederivaten

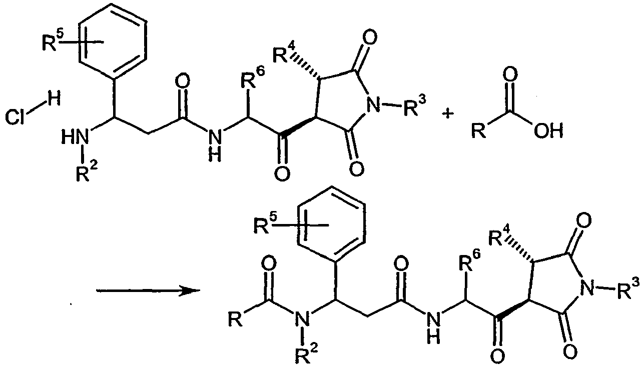

Zu einer Mischung aus Aminhydrochlorid (1.0 eq.), Carbonsäure (1.2 bis 1.3 eq.) und HATU (1.2 - 1.4 eq.) gelöst in absolutem N,N-Dimethylformamid oder in einer 1:1 Mischung aus N,N-Dimethylformamid und Dichlormethan (0.02 - 0.2 mol/l) wird bei 0°C eine 0.2 - 1.0 molare Lösung von Diisopropylethylamin (2.5 bis 3.5 eq.) in N,N-Dimethylformamid oder einer 1:1 Mischung aus N,N-Dimethylformamid und Dichlormethan über den Zeitraum von 1 h zugetropft. Nach Ende der Zugabe wird die Reaktionsmischung noch 30 min bei 0°C und über Nacht bei Raumtemperatur gerührt, bevor das Gemisch im Vakuum eingeengt wird. Das Produkt kann durch Chromatographie an Silicagel (Eluenten: Gemische aus Cyclohexan/Ethylacetat oder Gemische aus Dichlormethan und Ethanol) oder durch RP-HPLC (Eluenten: variable Gradienten aus Wasser und Acetonitril), alternativ durch eine Kombination beider Verfahren, erhalten werden.

### Alternativ kann die Umsetzung auch nach folgendem Verfahren erfolgen:

Zu einer Mischung aus Aminhydrochlorid (1.0 eq.), Carbonsäure (1.2 bis 1.3 eq.), Triethylamin (2.4 - 3 eq.) und HOBt (2.4 - 3 eq.) in absolutem Dichlormethan oder in einer Mischung aus N,N-Dimethylformamid und Dichlormethan (0.02 - 0.2 mol/l) werden abschliessend 1.2 eq. EDC gegeben. Die Reaktionsmischung wird bei Raumtemperatur gerührt (2 h - über Nacht), bevor im Vakuum eingeengt wird. Der Rückstand wird in Essigsäureethylester oder Dichlormethan aufgenommen und die organische Phase wird mit Wasser, gesättigter Natriumhydrogencarbonatlösung und gesättigter Kochsalzlösung gewaschen, über Natriumsulfat getrocknet, filtriert und eingeengt. Das Produkt kann durch Chromatographie an Silicagel (Eluenten: Gemische aus Cyclohexan/Ethylacetat oder Gemische aus Dichlormethan und Ethanol) oder durch RP-HPLC (Eluenten: variable Gradienten aus Wasser und Acetonitril), alternativ durch eine Kombination beider Verfahren, gereinigt werden.

Gemäß den oben beschriebenen Vorschriften zur Acylierung von 3-[2-Amino-alkanoyl]-2,5-pyrrolidindion Hydrochlorid Derivaten oder von acylalkylamino substituierten 3-[2-Amino-alkanoyl]-2,5-pyrrolidindion Hydrochlorid Derivaten mit Carbonsäurederivaten können folgende Verbindungen erhalten werden.

### Beispiel 1

### N-{(1S)-3-[((1S)-1-{[(3R,4S)-1,4-Dimethyl-2,5-dioxo-3-pyrrolidinyl]carbonyl}-2-methylpropyl)amino]-3-oxo-1-phenylpropyl}-2-pyridincarboxamid

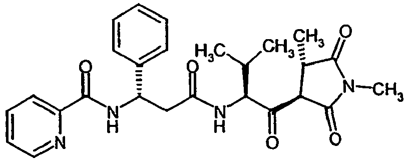

¹H-NMR (200 MHz, d₆-DMSO): δ = 9.59 (d, 1 H), 8.65 (d, 1 H), 8.21 (d, 1 H), 7.98 (d, 2 H), 7.61 (q, 1 H), 7.42-7.16 (m, 5 H), 5.48-5.32 (m, 1 H), 4.76 (dd, 1 H), 3.99 (d, 1 H), 3.20-2.92 (m, 2 H), 2.82 (s, 3 H), 2.61 (dd, 1 H), 2.32-2.13 (m, 1 H), 1.13 (d, 3 H), 0.65 (d, 3 H), 0.59 (d, 3 H).
MS (ES+): m/z (%) = 479 (M+H)⁺ (80).
HPLC (Methode 5): Rₜ = 3.79 min.

### Beispiel 2

### 6-Fluor-N-{(1S)-3-[((1S)-2-methyl-1-{[(3R,4S)-4-methyl-2,5-dioxo-3-pyrrolidinyl]-carbonyl}propyl)amino]-3-oxo-1-phenylpropyl}-2-pyridincarboxamid

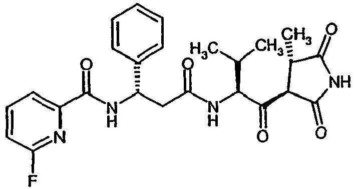

¹H-NMR (300 MHz, d₆-DMSO): δ = 11.31 (s, 1 H), 9.32 (d, 1 H), 8.22-8.10 (m, 2 H), 7.95-7.90 (m, 1 H), 7.45-7.18 (m, 6 H), 5.43-5.33 (m, 1 H), 4.70 (dd, 1 H), 4.02 (d, 1 H), 3.15 (dd, 1 H), 3.01-2.90 (m, 1 H), 2.62 (dd, 1 H), 2.30-2.18 (m, 1 H), 1.10 (d, 3 H), 0.62 (d, 3 H), 0.59 (d, 3 H).
MS (ES-): m/z (%) = 481 (M-H)⁻ (100).
HPLC (Methode 3): Rₜ = 4.19 min.

### Beispiel 3

### N-{(1S)-3-[((1S)-2-Methyl-1-{[(3R,4S)-4-methyl-2,5-dioxo-3-pyrrolidinyl]-carbonyl}propyl)amino]-3-oxo-1-phenylpropyl}-2-pyridincarboxamid

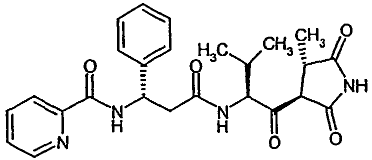

¹H-NMR (300 MHz, d₆-DMSO): δ = 11.32 (s, 1 H), 9.53 (d, 1 H), 8.69-8.65 (m, 1 H), 8.15 (d, 1 H), 8.02-7.98 (m, 2 H), 7.63-7.57 (m, 1 H), 7.40-7.18 (m, 5 H), 5.45-5.35 (m, 1 H), 4.67 (dd, 1 H), 4.00 (d, 1 H), 3.12 (dd, 1 H), 2.97 (dd, 1 H), 2.64 (dd, 1 H), 2.29-2.15 (m, 1 H), 1.10 (d, 3 H), 0.60 (d, 3 H), 0.56 (d, 3 H).
MS (ES+): m/z (%) = 465 (M+H)⁺ (100).
HPLC (Methode 6): Rₜ = 3.65 min.

### Beispiel 4

### N-{(1S)-3-[((1S)-2-Methyl-1-{[(3R,4S)-4-methyl-2,5-dioxo-3-pyrrolidinyl]-carbonyl}propyl)amino]-3-oxo-1-phenylpropyl}-3-chinolincarboxamid

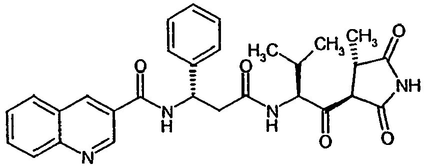

¹H-NMR (Hauptkonformer) (300 MHz, d₆-DMSO): δ = 11.31 (s, 1 H), 9.29-9.12 (m, 2 H), 8.82-8.78 (m, 1 H), 8.15-8.07 (m, 3 H), 7.90-7.82 (m, 1 H), 7.72-7.68 (m, 1 H), 7.50-7.20 (m, 5 H), 5.59-5.48 (m, 1 H), 4.71 (dd, 1 H), 3.98 (d, 1 H), 3.04-2.70 (m, 3 H), 2.31-2.22 (m, 1 H), 1.09 (d, 3 H), 0.79 (d, 3 H), 0.68 (d, 3 H).
MS (ES+): m/z (%) = 515 (M+H)⁺ (100).
HPLC (Methode 6): Rₜ = 3.52 min.

### Beispiel 5

### N-{(1S)-3-[((1S)-2-Methyl-1-{[(3R,4S)-4-methyl-2,5-dioxo-3-pyrrolidinyl]-carbonyl}propyl)amino]-3-oxo-1-phenylpropyl}-1-isochinolincarboxamid

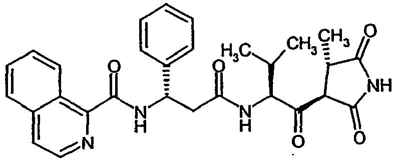

¹H-NMR (Hauptkonformer) (400 MHz, d₆-DMSO): δ = 11.34 (s, 1 H), 9.56 (d, 1 H), 9.00 (d, 1 H), 8.58 (d, 1 H), 8.13 (d, 1 H), 8.04 (d, 2 H), 7.80 (t, 1 H), 7.70 (t, 1 H), 7.45 (d, 2 H), 7.34 (t, 2 H), 7.23 (t, 1 H), 5.52-5.45 (m, 1 H), 4.69 (dd, 1 H), 4.00 (d, 1 H), 3.10 (dd, 1 H), 2.97-2.88 (m, 1 H), 2.70-2.62 (m, 1 H), 2.21-2.12 (m, 1 H), 1.10 (d, 3 H), 0.59 (d, 3 H), 0.50 (d, 3 H).
MS (ES+): m/z (%) = 515 (M+H)⁺ (100).
HPLC (Methode 6): Rₜ = 4.32 min.

### Beispiel 6

### N-{(1S)-3-[((1S)-2-Methyl-1-{[(3R,4S)-4-methyl-2,5-dioxo-3-pyrrolidinyl]-carbonyl}propyl)amino]-3-oxo-1-phenylpropyl}-4-phenyl-2-pyridincarboxamid

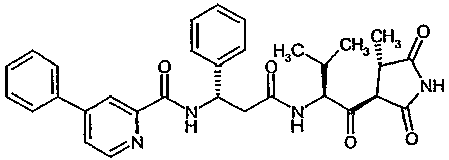

¹H-NMR (200 MHz, d₆-DMSO): δ = 11.36 (s, 1 H), 9.67 (d, 1 H), 8.72 (d, 1 H), 8.26-8.18 (m, 2 H), 7.98-7.92 (m, 1 H), 7.88-7.81 (m, 2 H), 7.60-7.50 (m, 3 H), 7.45-7.20 (m, 5 H), 5.50-5.38 (m, 1 H), 4.75-4.68 (m, 1 H), 4.02 (d, 1 H), 3.20-2.82 (m, 3 H), 2.25-2.15 (m, 1 H), 1.10 (d, 3 H), 0.61 (d, 3 H), 0.57 (d, 3 H).
MS (ES+): m/z (%) = 541 (M+H)⁺ (100).
HPLC (Methode 14): Rₜ = 4.88 min.

### Beispiel 7

### 6-Fluor-N-{(1S)-3-[((1S,2S)-2-methyl-1-{[(3R,4S)-4-methyl-2,5-dioxo-3-pyrrolidinyl]carbonyl}butyl)amino]-3-oxo-1-phenylpropyl}-2-pyridincarboxamid

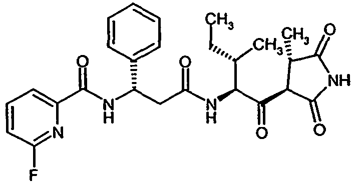

¹H-NMR (300 MHz, d₆-DMSO): δ = 11.33 (s, 1 H), 9.30 (d, 1 H), 8.22-8.12 (m, 2 H), 7.95-7.91 (m, 1 H), 7.45-7.20 (m, 6 H), 5.45-5.35 (m, 1 H), 4.67 (dd, 1 H), 4.03 (d, 1 H), 3.17-3.09 (m, 1 H), 2.97-2.88 (m, 1 H), 2.62-2.54 (m, 1 H), 2.02-1.90 (m, 1 H), 1.30-1.15 (m, 2 H), 1.12 (d, 3 H), 0.60 (d, 3 H), 0.56 (t, 3 H).
MS (ES+): m/z (%) = 497 (M+H)⁺ (100).
HPLC (Methode 12): Rₜ = 2.95 min.

### Beispiel 8

### N-[(1S)-3-({(1S)-1-Cyclohexyl-2-[(3R,4S)-4-methyl-2,5-dioxo-3-pyrrolidinyl]-2-oxoethyl}amino)-3-oxo-1-phenylpropyl]-6-fluor-2-pyridincarboxamid

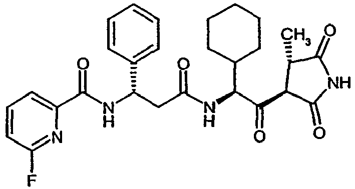

¹H-NMR (Hauptkonformer) (200 MHz, d₆-DMSO): δ = 11.35 (s, 1 H), 9.40 (d, 1 H), 8.27-8.10 (m, 2 H), 7.99-7.90 (m, 1 H), 7.50-7.18 (m, 6 H), 5.46-5.32 (m, 1 H), 4.73 (dd, 1 H), 4.08 (d, 1 H), 3.26-3.10 (m, 1 H), 3.00-2.80 (m, 2 H), 1.98-1.80 (m, 1 H), 1.60-1.25 (m, 5 H), 1.12 (d, 3 H), 1.10-0.65 (m, 5 H).
MS (ES+): m/z (%) = 523 (M+H)⁺ (100).
HPLC (Methode 12): Rₜ = 3.12 min.

### Beispiel 9

### N-[(1S)-3-({1-Cyclopentyl-2-[(3R,4S)-4-methyl-2,5-dioxo-3-pyrrolidinyl]-2-oxoethyl}amino)-3-oxo-1-phenylpropyl]-2-pyridincarboxamid

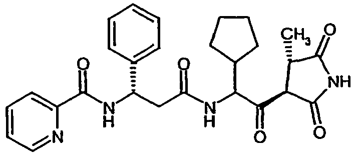

¹H-NMR (2 Diastereomere, ca. 2:1 Verhältnis; mehrere Konformere) (400 MHz, d₆-DMSO): δ = 11.35 + 11.31 (2x s, 1 H), 9.56 + 9.51-9.41 (d + m, 1 H), 8.71-8.63 (m, 1 H), 8.60-8.42 + 8.30 (m + d, 1 H), 8.02-7.92 (m, 2 H), 7.65-7.57 (m, 1 H), 7.42-7.18 (m, 5 H), 5.48-5.37 (m, 1 H), 4.65 + 4.45-4.28 (t + m, 1 H), 3.99 + 3.87 + 3.80 (d + t + d, 1 H), 3.13 + 3.07-2.98 (dd + m, 1 H), 2.95-2.80 (m, 1 H), 2.70-2.58 (m, 1 H), 2.36-2.18 (m, 1 H), 1.65-0.85 (m, 8 H), 1.12 + 1.01 (2x d, 3 H).
MS (ES+): m/z (%) = 491 (M+H)⁺ (100).
HPLC (Methode 12): Rₜ = 2.79 min.

### Beispiel 10

### (3S)-N-((1S)-2-Methyl-1-{[(3R,4S)-4-methyl-2,5-dioxo-3-pyrrolidinyl]carbonyl}-propyl)-3-phenyl-3-[(2-thienylacetyl)amino]propanamid

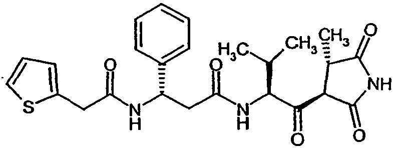

¹H-NMR (300 MHz, d₆-DMSO): δ = 11.31 (s, 1 H), 8.50 (d, 1 H), 8.08 (d, 1 H), 7.34-7.18 (m, 6 H), 6.95-6.90 (m, 1 H), 6.89-6.85 (m, 1 H), 5.25-5.16 (m, 1 H), 4.61 (dd, 1 H), 3.91 (d, 1 H), 3.66 (d, 2 H), 2.90 (dd, 1 H), 2.80-2.60 (m, 2 H), 2.32-2.23 (m, 1 H), 1.07 (d, 3 H), 0.81 (d, 3 H), 0.75 (d, 3 H).
MS (ES-): m/z (%) = 482 (M-H)⁻ (100).
HPLC (Methode 5): Rₜ = 3.72 min.

| **Beispiel** | **Struktur** | **MW** | **MS** | **HPLC** |
|---|---|---|---|---|
| 11 | 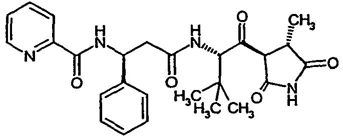 | 478.55 | MS (ES+), m/z (%): 479 (M+H)⁺ (100) | HPLC (Methode 5) Rₜ = 3.69 min |
| 12 | 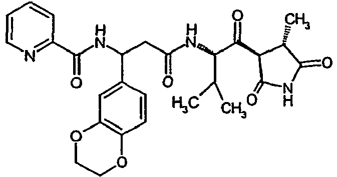 | 522.55 | MS (ES+), m/z (%): 523 (M+H)⁺ (75) | HPLC (Methode 9) Rₜ = 3.81 min |
| 13 | 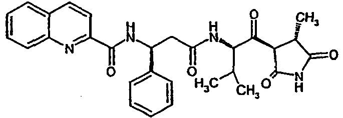 | 514.58 | MS (ES+), m/z (%): 515 (M+H)⁺ (100) | HPLC (Methode 5) Rₜ = 3.97 min |
| 14 | 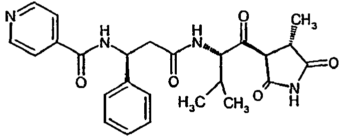 | 464.52 | MS (ES+), m/z (%): 465 (M+M)⁺ (100) | HPLC (Methode 5) Rₜ = 3.01 min |
| 15 | 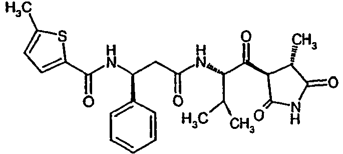 | 483.59 | MS (ES+), m/z (%): 506 (M+Na)⁺ (100) | HPLC (Methode 5) Rₜ = 3.74 min |
| 16 | 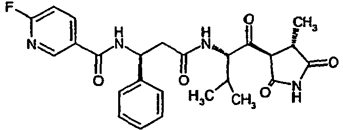 | 482.51 | MS (ES+), m/z (%): 505 (M+Na)⁺ (100) | HPLC (Methode 6) Rₜ = 3.48 min |
| 17 | 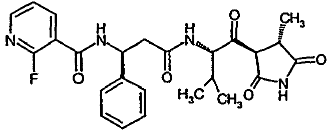 | 482.51 | MS (ES-), m/z (%): 481 (M-H⁺) (100) | HPLC (Methode 6) Rₜ = 3.46 min |
| 18 | 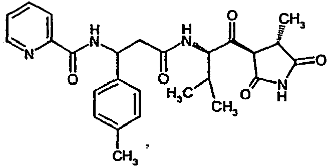 | 478.55 | MS (ES+), m/z (%): 479 (M+H)⁺ (45) | HPLC (Methode 9) Rₜ = 4.03 min |
| 19 | 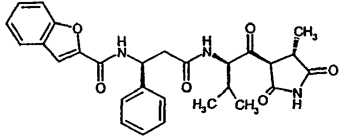 | 503.55 | MS (ES+), m/z (%): 523 (M+Na)⁺ (100) | HPLC (Methode 6) Rₜ = 3.94 min |
| 20 | 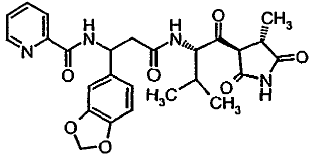 | 508.53 | MS (ES+), m/z (%): 509 (M+H⁺) (90) | HPLC (Methode 9) Rₜ = 3.82 min |
| 21 | 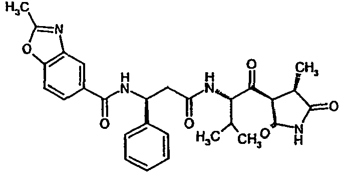 | 518.57 | MS (ES+), m/z (%): 519 (M+H)⁺ (100) | HPLC (Methode 3) Rₜ = 3.95 min |
| 22 | 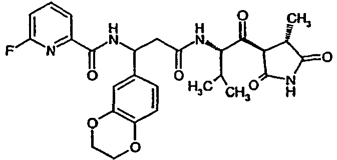 | 540.55 | MS (ES+), m/z (%): 541 (M+H)⁺ (35) | HPLC (Methode 9) Rₜ = 3.92 min |
| 23 | 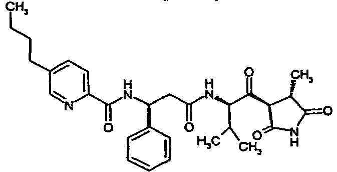 | 520.63 | MS (ES-), m/z (%): 519 (M-H)⁻ (100) | HPLC (Methode 5) Rₜ = 4.6min |
| 24 | 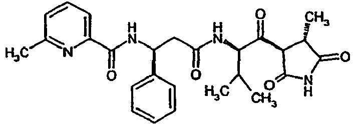 | 478.55 | MS (ES-), m/z (%): 477 (M-H)⁻ (100) | HPLC (Methode 5) Rₜ = 3.9 min |
| 25 | 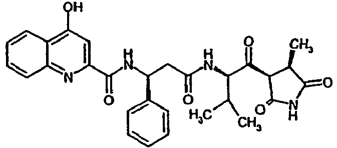 | 530.58 | MS (ES+), m/z (%): 531 (M+H)⁺ (100) | HPLC (Methode 6) Rₜ = 3.70 min |
| 26 | 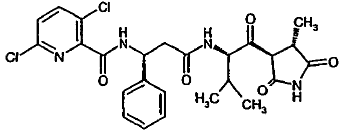 | 533.41 | MS (ES+), m/z (%): 533 (M+H⁺) (100) | HPLC (Methode 6) Rₜ = 4.20 min |
| 27 | 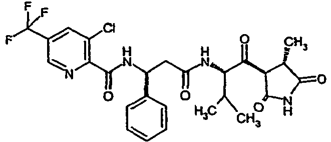 | 566.96 | MS (ES+), m/z (%): 567 (M+H⁺) (100) | HPLC (Methode 6) Rₜ = 4.42 min |
| 28 | 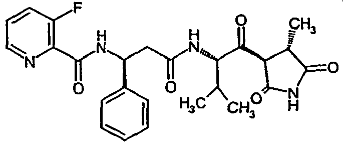 | 482.51 | MS(ES+), m/z (%): 483 (M+H)⁺ (100) | HPLC (Methode 11) Rₜ = 2.66 min |
| 29 | 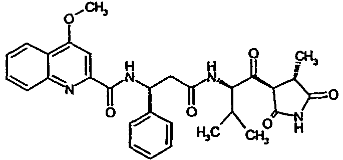 | 544.60 | MS (ES+), m/z (%): 545 (M+H)⁺ (100) | HPLC (Methode 14) Rₜ = 4.5 min |
| 30 | 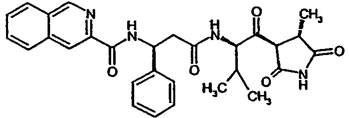 | 514.58 | MS (ES-), m/z (%): 513 (M-H)⁻ (100) | HPLC (Methode 14) Rₜ = 4.40 min |
| 31 | 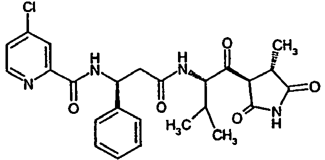 | 498.96 | MS (ES-), m/z (%): 497 (M-H)⁻ (100) | HPLC (Methode 14) Rₜ = 4.37 min |
| 32 | 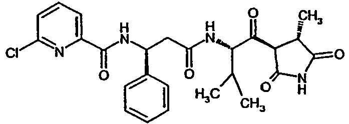 | 498.96 | MS (ES+), m/z (%): 521 (M+Na)⁺ (100) | HPLC (Methode 13) Rₜ = 4.05 min |
| 33 | 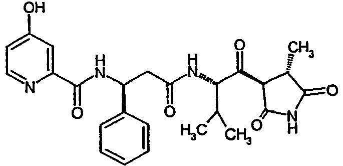 | 480.52 | MS (ES+), m/z (%): 481 (M+H)⁺ (100) | HPLC (Methode 14) Rₜ = 3.65 min |
| 34 | 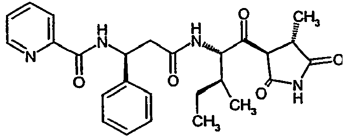 | 478.55 | MS (ES+), m/z (%): 479 (M+H)⁺ (100) | HPLC (Methode 13) Rₜ = 3.98 min |
| 35 | 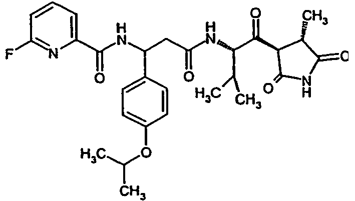 | 540.59 | MS (ES-), m/z (%): 539 (M-H)⁻ (100) | HPLC (Methode 11) Rₜ = 2.73 min |
| 36 | 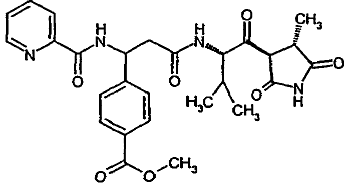 | 522.55 | MS (ES-), m/z (%): 521 (M-H)⁻ (100) | HPLC (Methode 5) Rₜ = 3.67 min |
| 37 | 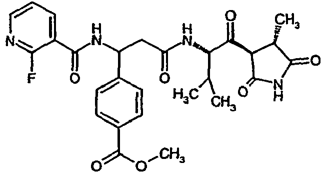 | 540.55 | MS (ES+), m/z (%): 541 (M+H)⁺ (100) | HPLC (Methode 9) Rₜ = 3.75 min |
| 38 | 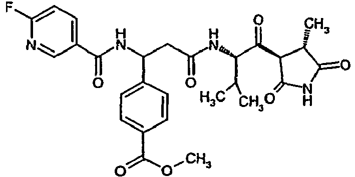 | 540.55 | MS (ES+), m/z (%): 541 (M+H)⁺ (100) | HPLC (Methode 9) Rₜ = 3.80 min |
| 39 | 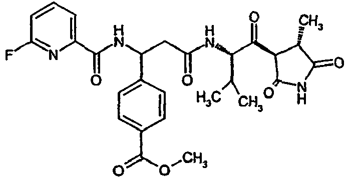 | 540.55 | MS (ES+), m/z (%): 541 (M+H)⁺ (100) | HPLC (Methode 14) Rₜ = 4.12 min |
| 40 | 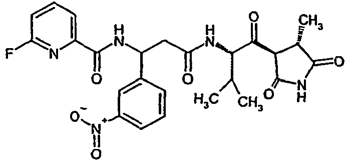 | 527.51 | MS (ES+), m/z (%): 528 (M+H)⁺ (100) | HPLC (Methode 12) Rₜ = 2.76 min |
| 41 | 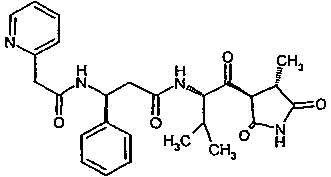 | 478.55 | MS (ES+), m/z (%): 479 (M+H)⁺ (100) | HPLC (Methode 5) Rₜ = 2.64 min |
| 42 | 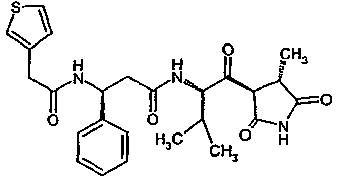 | 483.59 | MS (ES+), m/z (%): 484 (M+H)⁺ (100) | HPLC (Methode 15) Rₜ = 2.94 min |
| 43 | 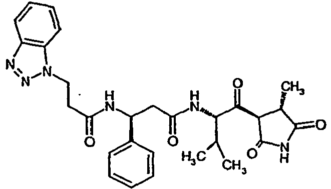 | 532.60 | MS (ES+), m/z (%): 555 (M+Na)⁺ (100) | HPLC (Methode 5) Rₜ = 3.52 min |
| 44 | 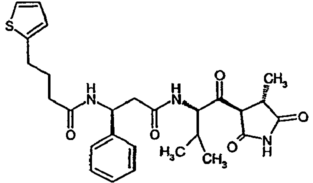 | 511.64 | MS (ES+), m/z (%): 534 (M+Na)⁺ (100) | HPLC (Methode 5) Rₜ = 4.00 min |

### Allgemeine Vorschrift I: Festphasengestütze Synthese

Das Aldehydharz (Nova Biochem) (0.78 mmol/g) wird in Toluol/Trimethylorthoformiat (1:1 bis 4:1) suspendiert, mit dem entsprechenden beta-Aminosäuremethylester (2.5 - 3 eq) bei Raumtemperatur versetzt und über Nacht geschüttelt. Das Harz wird zweimal mit N,N-Dimethylformamid gewaschen, in N,N-Dimethylformamid suspendiert und bei Raumtemperatur mit Tetrabutylammoniumborhydrid (2 - 5 eq) versetzt. Nach 30 Minuten Schütteln bei Raumtemperatur wird das Reaktionsgemisch bei -40°C bis Raumtemperatur langsam mit Eisessig (100 eq) versetzt, gegebenenfalls wieder auf Raumtemperatur erwärmt und für mindestens 1 h geschüttelt. Das Harz wird wiederholt mit Wasser, Methanol, Dichlormethan/10 % N,N-Diisopropylethylamin, Methanol, Dichlormethan und Diethylether gewaschen und getrocknet. Das Harz wird in Dichlormethan suspendiert, mit N,N-Diisopropylethylamin (10 - 20 eq) und dem entsprechenden Carbonsäurechlorid (5 eq) bei Raumtemperatur 1 - 2 h lang geschüttelt. Das Harz wird wiederholt mit Methanol, N,N-Dimethylformamid, Methanol, Dichlormethan und Diethylether gewaschen und getrocknet. Zur Verseifung wird das Harz mit einer Lösung von Kaliumhydroxid (30 eq) in Methanol/Dioxan (1:2, 30 mg Kaliumhydroxid/ml Lösung) versetzt und 3 h bei RT geschüttelt. Anschließend wird das Harz mit Wasser, Methanol, Dichlormethan/Eisessig, Dichlormethan, Dichlormethan/N,N-Diisopropylethylamin, Methanol, N,N-Dimethylformamid, Methanol, Dichlormethan und Diethylether gewaschen. Das Harz wird mit (Benzotriazol-1-yloxy)bisdimethylaminomethylium-fluoroborat (5eq) und N,N-Diisopropylethylamin (20 eq) in N,N-Dimethylacetamid bei RT 1h geschüttelt, zweimal mit N,N-Dimethylacetamid gewaschen und mit einer frisch zubereiteten Lösung von (3*R*,4*S*)-3-[(2*S*)-2-Amino-3-methylbutanoyl]-4-methyl-2,5-pyrrolidindion Hydrochlorid (1.5 - 2 eq) und N.N-Diisopropylethylamin (20 eq) versetzt und 3 h bei RT geschüttelt. Das Harz wird abschließend wiederholt mit Methanol, N,N-Dimethylformamid, Wasser, N,N-Dimethylformamid, Methanol, Dichlormethan und Diethylether gewaschen und getrocknet. Das Harz wird mit Trifluoressigsäure oder 50 %iger Trifluoressigsäure in Dichlormethan 30 min bis 3 h bei RT bis 50°C geschüttelt. Die Rohproduktlösung wird abfiltriert, zur Trockene eingedampft und durch Reversed Phase HPLC mit einem Wasser/Acetonitril-Gradienten gereinigt. Alternativ ist auch eine Chromatographie an Silicagel (Eluenten: Gemische aus Dichlormethan und Methanol) möglich.

### Beispiel 45

### N-{1-(3-Chlorphenyl)-3-[((1S)-2-methyl-1-{[(3R,4S)-4-methyl-2,5-dioxo-3-pyrrolidinyl]carbonyl}propyl)amino]-3-oxopropyl}-2-pyridincarboxamid

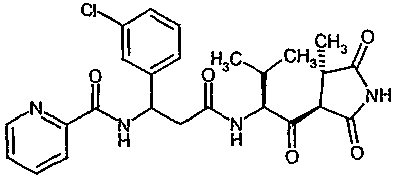

¹H-NMR (2 Diastereoisomere, Verhältnis 1:1, 300 MHz, DMSO-d₆): δ = 11.3 (d, 1H), 9.64 (d, 0.5 H), 9.45 (d, 0.5 H), 8.70-8.62 (m, 1 H), 8.28 (d, 0.5 H), 8.18 (d, 0.5 H), 8.02-7.94 (m, 2 H), 7.65-7.56 (m, 1 H), 7.50-7.41 (m, 1 H), 7.38-7.21 (m, 3 H), 5.48-5.32 (m, 1 H), 4.69 (dd, 0.5 H), 4.60 (dd, 0.5 H), 4.02 (d, 0.5 H), 3.93 (d, 0.5 H), 3.10-2.60 (m, 3 H), 2.35-2.15 (m, 1 H), 1.15-1.03 (m, 3 H), 0.75-0.54 (m, 6 H).
MS (ES+): m/z (%) = 499 (M+H)⁺ (100).
HPLC (Methode 6): Rₜ = 3.95 min.

### Beispiel 46

### N-[(1S-3-({(1S)-1-Cyclopentyl-2-[(3R,4S)-4-methyl-2,5-dioxo-3-pyrrolidinyl]-2-oxoethyl}amino)-3-oxo-1-phenylpropyl]-4-phenyl-2-pyridincarboxamid

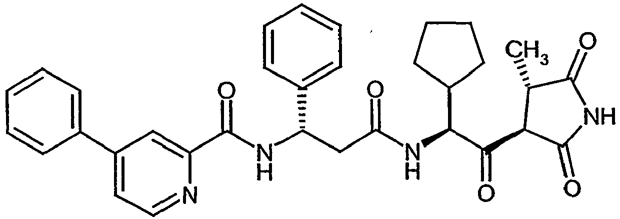

¹H-NMR (300 MHz, d₆-DMSO): δ = 11.33 (s, 1 H), 9.59 (d, 1 H), 8.74 (d, 1 H), 8.31 (d, 1 H), 8.23 (s, 1H), 7.98-7.89 (m, 1 H), 7.87-7.77 (m, 2H), 7.59-7.19 (m, 8 H), 5.50-5.38 (m, 1 H), 4.62 (t, 1 H), 3.99 (d, 1H), 3.48-3.00 (m, 2 H), 2.96-2.83 (m, 1 H), 2.75-2.18 (m, 4 H), 1.53-0.71 (m, 5 H), 1.08 (d, 3 H).
MS (ES+): m/z = 567 (M+H⁺).
HPLC (Methode 19): Rₜ = 4.28 min.

### Beispiel 47

### N-[(1S)-3-({(1S)-1-Cyclopentyl-2-[(3R,4S)-4-methyl-2,5-dioxo-3-pyrrolidinyl]-2-oxoethyl}amino)-3-oxo-1-phenylpropyl]-3-chinolincarboxamid

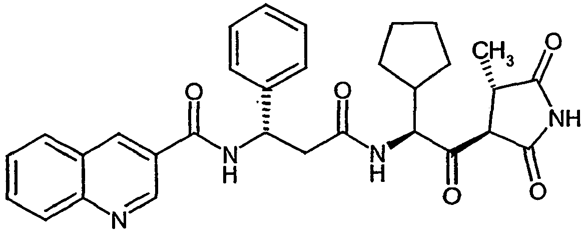

¹H-NMR (300 MHz, d₆-DMSO): δ = 11.34 (s, 1 H), 9.32 (d, 1 H), 9.22 (d, 1 H), 8.93 (d, 1 H), 8.32 (d, 1H), 8.11 (d, 2H), 7.89 (dt, 1H), 7.72 (t, 1H), 7.50-7.19 (m, 5 H), 5.58-5.44 (m, 1 H), 4.61 (t, 1 H), 3.93 (d, 1H), 3.77-3.55 (m, 1H), 3.10-2.21 (m, 5 H), 1.60-0.96 (m, 6 H), 1.09 (d, 3 H).
MS (ESI+): m/z = 541 (M+H⁺).
HPLC (Methode 19): Rₜ = 4.04 min.

### Beispiel 48

### N-[(1S)-3-({(1S)-1-Cyclopentyl-2-[(3R,4S)-4-methyl-2,5-dioxo-3-pyrrolidinyl]-2-oxoethyl}amino)-3-oxo-1-phenylpropyl]-5-fluor-1H-indol-2-carboxamid

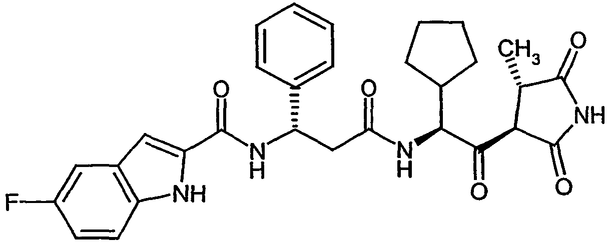

¹H-NMR (200 MHz, d₆-DMSO): δ = 11.66 (s, 1 H), 11.37 (s, 1H), 8.83 (d, 1H), 8.27 (d, 1H), 7.48-6.94 (m, 9 H) 5.54-5.36 (m, 1 H), 4.71-4.61 (m, 1H), 3.98 (d, 1 H), 3.28-3.19 (m, 1 H), 3.01-2.11 (m, 6 H), 1.54-0.97 (m, 5 H), 1.08 (d, 3 H).
MS (ESI+): m/z = 547 (M+H⁺).
HPLC (Methode 19): Rₜ = 3.44 min.

| **Beispiel** | **Struktur** | **MW** | **MS** | **HPLC** |
|---|---|---|---|---|
| 49 | 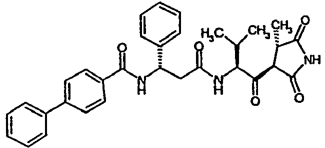 | 539.63 | MS (ES+), m/z: 540 (M+H)⁺ | HPLC (Methode 5): Rₜ = 4.22 min |
| 50 | 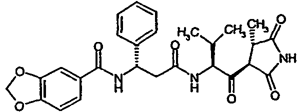 | 507.54 | MS (ES+), m/z: 508 (M+H)⁺ | HPLC (Methode 5): Rₜ = 3.61 min |
| 51 | 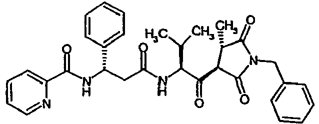 | 554.64 | MS (ES+), m/z: 555 (M+H)⁺ | HPLC (Methode 8): Rₜ = 2.72 min |
| 52 | 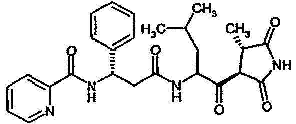 | 478.55 | MS (ES+), m/z: 479 (M+H)⁺ | HPLC (Methode 5): Rₜ = 3.67 min |
| 53 | 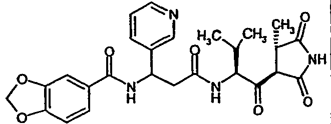 | 508.53 | MS (ES+), m/z: 509 (M+H)⁺ | HPLC (Methode 5): Rₜ = 2.45 und 2.52 min |
| 54 | 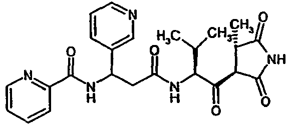 | 465.51 | MS (ES+), m/z: 466 (M+H)⁺ | |
| 55 | 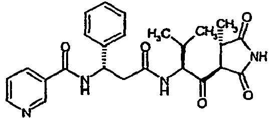 | 464.52 | MS (ES+), m/z: 465 (M+H)⁺ | HPLC (Methode 5): Rₜ = 3.02 min |
| 56 | 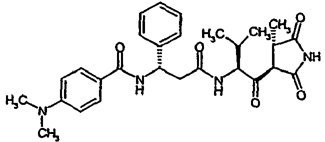 | 506.60 | MS(ES+), m/z: 507 (M+H)⁺ | HPLC (Methode 5): Rₜ = 3.72 min |
| 57 | 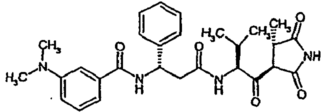 | 506.60 | MS (ES+), m/z: 507 (M+H)⁺ | HPLC (Methode 5): Rₜ = 3.62 min |
| 58 | 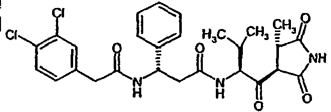 | 546.45 | MS (ES+), m/z: 546 (M+H)⁺ | HPLC (Methode 8): Rₜ = 2.60 min |
| 59 | 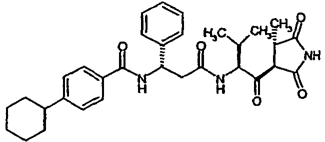 | 545.68 | MS (ES+), m/z: 546 (M+H)⁺ | HPLC (Methode 5): Rₜ = 4.65 min |
| 60 | 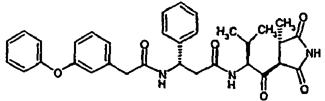 | 569.65 | MS (ES+), m/z: 570 (M+H)⁺ | HPLC (Methode 5): Rₜ = 4.19 min |
| 61 | 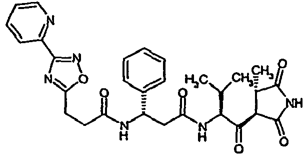 | 560.61 | MS (ES+), m/z: 561 (M+H)⁺ | HPLC (Methode 5): Rₜ = 3.28 min |
| 62 | 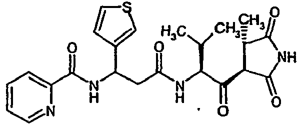 | 470.55 | MS (ES+), m/z: 471 (M+H)⁺ | HPLC (Methode 5): Rₜ = 3.5 min |
| 63 | 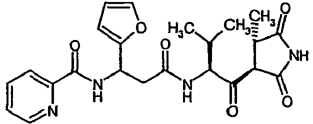 | 454.48 | MS (ES+), m/z: 455 (M+H)⁺ | HPLC (Methode 5): Rₜ = 3.4 min |
| 64 | 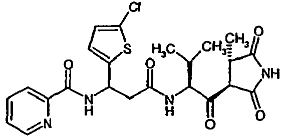 | 504.99 | MS (ES+), m/z: 505 (M+H)⁺ | HPLC (Methode 5): Rₜ = 3.8 min |
| 65 | 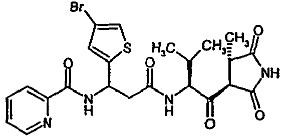 | 549.44 | MS (ES+), m/z: 551 (M+H)⁺ | HPLC (Methode 5): Rₜ = 3.8 min |
| 66 | 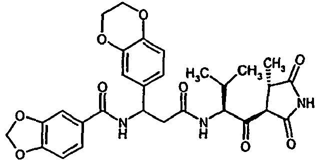 | 565.58 | MS (ES+), m/z: 566 (M+H)⁺ | HPLC (Methode 5): Rₜ = 3.5 min |
| 67 | 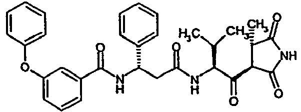 | 555.63 | MS (ES-), m/z: 554 (M-H)⁻ | HPLC (Methode 6): Rₜ = 4.28 min |
| 68 | 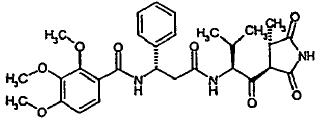 | 553.61 | MS (ES+), m/z: 554 (M+H)⁺ | HPLC (Methode 6): Rₜ = 3.85 min |
| 69 | 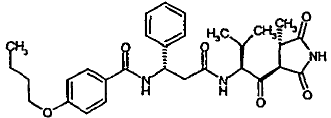 | 535.64 | MS (ES+), m/z: 536 (M+H)⁺ | HPLC (Methode 6): Rₜ = 4.34 min |
| 70 | 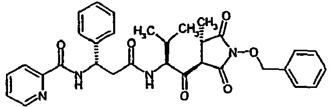 | 570.64 | MS (ES+), m/z: 571 (M+H)⁺ | HPLC (Methode 5): Rₜ = 4.48 min |
| 71 | 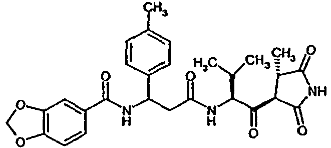 | 521.57 | MS (ES+), m/z: 522 (M+H)⁺ | HPLC (Methode 9): Rₜ = 4.05 min |
| 72 | 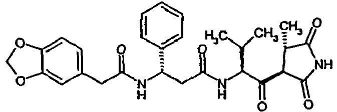 | 521.57 | Ms (ES+), m/z: 522 (M+H)⁺ | HPLC (Methode 5): Rₜ = 3.73 min |
| 73 | 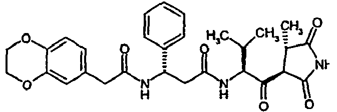 | 535.59 | MS (ES-), m/z: 534 (M-H)⁻ | HPLC (Methode 5): Rₜ = 3.71 min |
| 74 | 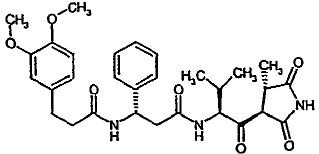 | 551.64 | MS (ES-), m/z: 550 (M-H)⁻ | HPLC (Methode 5): Rₜ = 3.65 min |
| 75 | 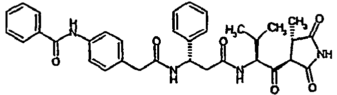 | 596.68 | MS (ES+), m/z: 597 (M+H)⁺ | HPLC (Methode 6): Rₜ = 3.78 min |
| 76 | 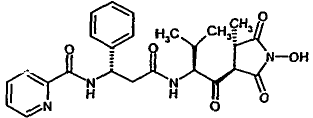 | 480.52 | MS (ES+), m/z: 481 (M+H)⁺ | HPLC (Methode 5): Rₜ = 3.63 min |
| 77 | 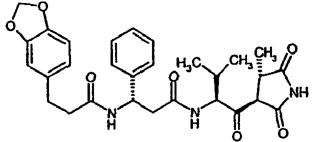 | 535.59 | MS (ES+), m/z: 536 (M+H)⁺ | HPLC (Methode 6): Rₜ = 3.76 min |
| 78 | 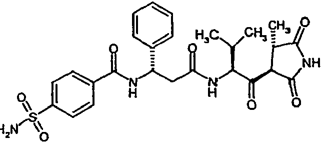 | 542.61 | MS (ES-), m/z: 541 (M-H)⁻ | HPLC (Methode 5): Rₜ = 3.39 min |
| 79 | 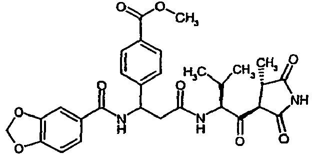 | 565.58 | MS (ES+), m/z: 566 (M+H)⁺ | HPLC (Methode 14): Rₜ = 4.19 min |
| 80 | 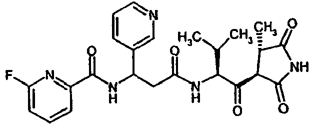 | 483.50 | MS (ES+), m/z: 484 (M+H)⁺ | HPLC (Methode 9): Rₜ = 2.93 min |
| 81 | 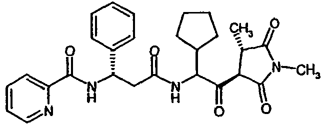 | 504.58 | MS (ES+), m/z: 505 (M+H)⁺ | HPLC (Methode 17): Rₜ = 3.57 min |
| 82 | 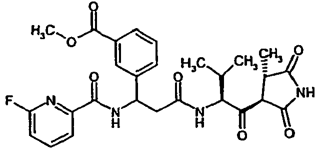 | 540.55 | MS (ES+), m/z: 563 (M+Na)⁺ | HPLC (Methode 14): Rₜ = 4.25 min |
| 83 | 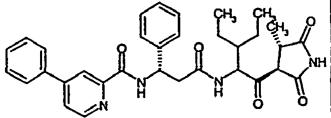 | 568.67 | MS (ES+), m/z: 570 (M+H)⁺ | HPLC (Methode 18): Rₜ = 3.96 min |
| 84 | 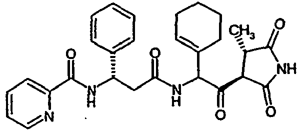 | 502.57 | MS (ES+), m/z: 503 (M+H)⁺ | HPLC (Methode 20): Rₜ = 3.45 min |
| 85 | 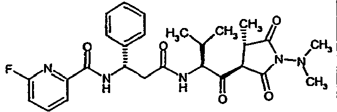 | 525.58 | MS (ES+), m/z: 526 (M+H)⁺ | HPLC (Methode 20): Rₜ = 3.55 min |
| 86 | 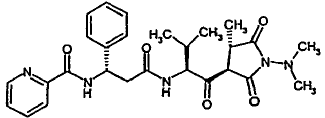 | 507.59 | MS (ES+), m/z: 508 (M+H)⁺ | HPLC (Methode 20): Rₜ = 3.43 min |
| 87 | 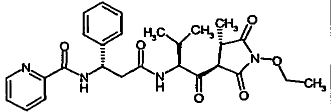 | 508.57 | MS (ES+), m/z: 509 (M+H)⁺ | HPLC (Methode 20): Rₜ = 3.60 min |
| 88 | 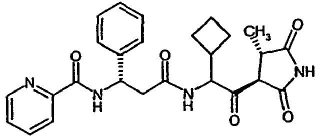 | 476.53 | MS (ES+), m/z: 477 (M+H)⁺ | HPLC (Methode 20): Rₜ = 3.12 min |
| 89 | 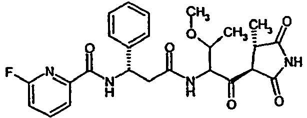 | 498.51 | MS (ES+), m/z: 499 (M+H)⁺ | HPLC (Methode 18): Rₜ = 3.35 min |
| 90 | 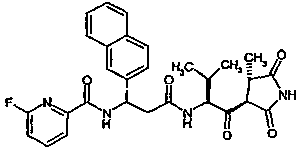 | 532.57 | MS (ES+), m/z: 533 (M+H)⁺ | HPLC (Methode 19): Rₜ = 4.24 min |
| 91 | 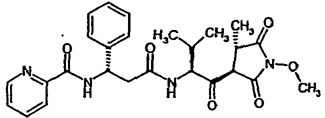 | 494.55 | MS (ES+), m/z: 495 (M+H)⁺ | HPLC (Methode 19): Rₜ = 3.47 min |
| 92 | 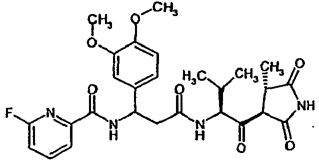 | 542.56 | MS (ES+), m/z: 543 (M+H)⁺ | HPLC (Methode 20): Rₜ = 3.02 min |
| 93 | 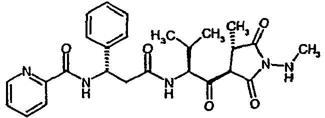 | 493.56 | MS (ES+), m/z: 494 (M+H)⁺ | HPLC (Methode 19): Rₜ = 4.06 min |
| 94 | 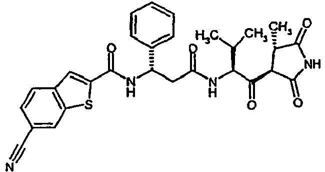 | 544.63 | MS (ES+), m/z: 545 (M+H)⁺ | HPLC (Methode 20): Rₜ = 3.42 min |
| 95 | 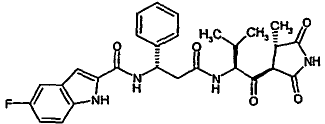 | 520.56 | MS (ES+), m/z: 521 (M+H)⁺ | HPLC (Methode 19): Rₜ = 4.17 min |
| 96 | 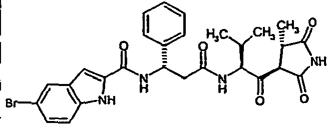 | 581.46 | MS (ES+), m/z: 583 (M+H)⁺ | HPLC (Methode 18): Rₜ = 3.6 min |
| 97 | 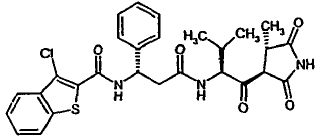 | 554.06 | MS (ES+), m/z: 554 (M+H)⁺ | HPLC (Methode 19): Rₜ = 4.17 min |
| 98 | 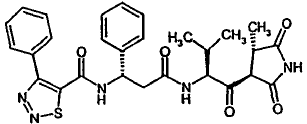 | 547.63 | MS (ES+), m/z: 548 (M+H)⁺ | HPLC (Methode 19): Rₜ = 4.01 min |
| 99 | 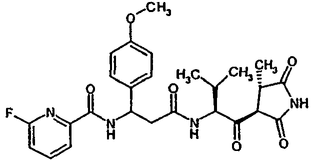 | 512.54 | MS (ES+), m/z: 513 (M+H)⁺ | HPLC (Methode 19): Rₜ = 4.04 min |
| 100 | 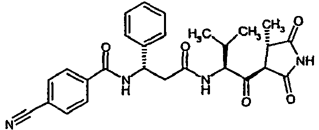 | 488.54 | MS (ES+), m/z: 489 (M+H)⁺ | HPLC (Methode 20): Rₜ = 3.15 min |
| 101 | 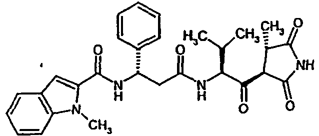 | 516.60 | MS (ES+), m/z: 517 (M+H)⁺ | HPLC (Methode 18): Rₜ = 3.5 min |
| 102 | 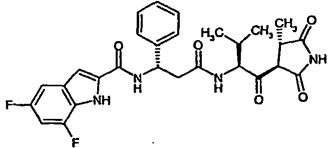 | 538.55 | MS (ES+), m/z: 539 (M+H)⁺ | HPLC (Methode 18): Rₜ = 3.5 min |
| 103 | 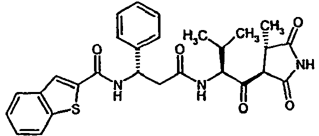 | 519.62 | MS (ES+), m/z: 520 (M+H)⁺ | HPLC (Methode 18): Rₜ = 3.5 min |
| 104 | 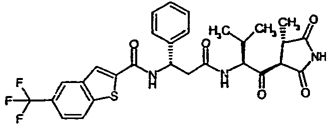 | 587.62 | MS (ES+), m/z: 588 (M+H)⁺ | HPLC (Methode 18): Rₜ = 3.7 min |
| 105 | 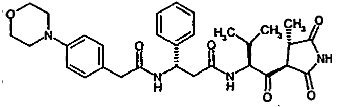 | 562.66 | MS (ES+), m/z: 563 (M+H)⁺ | HPLC (Methode 19): Rₜ = 2.42 min |
| 106 | 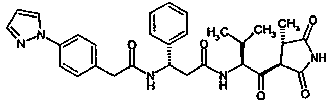 | 543.62 | MS (ES+), m/z: 544 (M+H)⁺ | HPLC (Methode 18): Rₜ = 3.2 min |
| 107 | 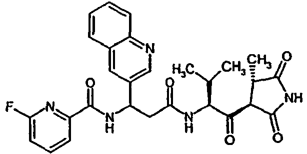 | 533.56 | MS (ES+), m/z: 534 (M+H)⁺ | HPLC (Methode 20): Rₜ = 2.45 min |
| 108 | 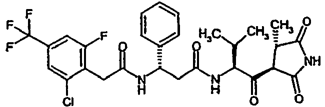 | 597.99 | MS (ES+), m/z: 598 (M+H)⁺ | HPLC (Methode 18): Rₜ = 3.6 min |
| 109 | 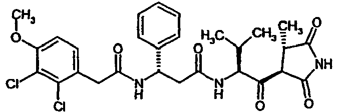 | 576.47 | MS (ES+), m/z: 576 (M+H)⁺ | HPLC (Methode 18): Rₜ = 3.5 min |
| 110 | 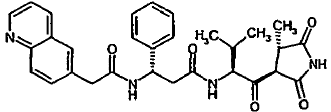 | 528.61 | MS (ES+), m/z: 529 (M+H)⁺ | HPLC (Methode 18): Rₜ = 2.6 min |
| 111 | 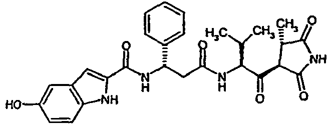 | 518.57 | MS (ES+), m/z: 519 (M+H)⁺ | HPLC (Methode 19): Rₜ = 2.26 min |
| 112 | 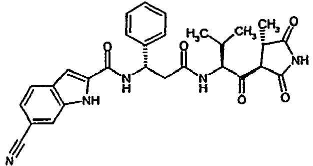 | 527.58 | MS (ES+), m/z: 528 (M+H)⁺ | HPLC (Methode 19): Rₜ = 2.61 min |
| 113 | 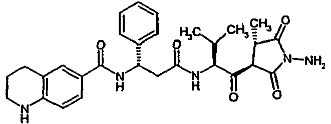 | 533.63 | MS (ES+), m/z: 534 (M+H)⁺ | HPLC (Methode 19): Rₜ = 2.55 min |
| 114 | 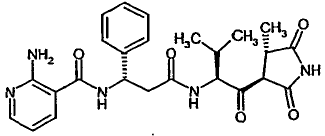 | 479.53 | MS (ES+), m/z: 480 (M+H)⁺ | HPLC (Methode 20): Rₜ = 1.97 min |
| 115 | 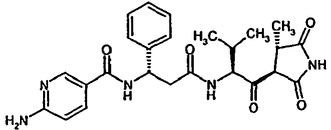 | 479.53 | MS (ES+), m/z: 480 (M+H)⁺ | HPLC (Methode 19): Rₜ = 1.82 min |

### B) Bewertung der physiologischen Wirksamkeit

Die Eignung der erfindungsgemäßen Verbindungen zur Behandlung bakterieller Erkrankungen kann in folgenden Tiermodellen gezeigt werden:

### Bestimmung der Minimalen Hemmkonzentration (MHK):

Die MHK wird im Flüssigdilutionstest bestimmt. Übernachtkulturen der Testkeime werden auf eine Zellzahl von 10⁵ Keimen pro ml in Isosensitest-Medium (Fa. Difco, Irvine, USA) verdünnt und mit Verdünnungen der Testsubstanzen (Verdünnungsstufen 1:2) inkubiert. Ausnahmen sind die Tests mit S. pneumoniae G9A, die in BHI-Bouillon (Fa. Difco) plus 20 % Rinderserum, und mit H. influenzae, die in BHI-Bouillon (Fa. Difco) plus 20 % Rinderserum, 10 µg/ml Haemin und 1 % Isovitale (Fa. Becton Dickinson, New Jersey, USA) durchgeführt werden.
Die Kulturen werden bei 37°C für 18-24 Stunden inkubiert; S. pneumoniae und H. influenzae in Gegenwart von 8 -10 % CO₂.

### Ergebnisse:

Die jeweils niedrigste Substanzkonzentration, bei der kein sichtbares Bakterienwachstum mehr auftritt, wird als MHK definiert. Die MHK-Werte in µmol/l einiger erfindungsgemäßer Verbindungen gegenüber einer Reihe von Testkeimen sind in der nachstehenden Tabelle beispielhaft aufgeführt.

| **Bsp. Nr.** | **Staphylokokkus aureus 133** | **Haemophilus influenzae Spain 7** |
|---|---|---|
| 2 | 15.63 | 7.81 |
| 4 | 15.63 | 31.25 |
| 6 | 7.81 | 15.63 |
| 9 | 7.81 | 62.5 |
| 48 | 0.98 | 15.63 |
| 109 | 0.98 | 62.5 |

### Systemische Infektion mit S. aureus 133

S. aureus 133 Zellen werden über Nacht in BH-Bouillon (Fa. Oxoid, New York, USA) angezüchtet. Die Übernachtkultur wird 1:100 in frische BH-Bouillon verdünnt und für 3 Stunden hochgedreht. Die in der logarithmischen Wachstumsphase befindlichen Bakterien werden abzentrifugiert und 2 x mit gepufferter, physiologischer Kochsalzlösung gewaschen. Danach wird am Photometer (Modell LP 2W, Fa. Dr. Lange, Berlin, Deutschland) eine Zellsuspension in Kochsalzlösung mit einer Extinktion von 50 Einheiten eingestellt. Nach einem Verdünnungsschritt (1:15) wird diese Suspension 1:1 mit einer 10 %igen Mucinsuspension gemischt. Von dieser Infektionslösung wird 0,25 ml/20 g Maus i.p. appliziert. Dies entspricht einer Zellzahl von etwa 1 x 10E⁶ Keimen/Maus. Die i.p.- oder i.v.Therapie erfolgt 30 Minuten nach der Infektion. Für den Infektionsversuch werden weibliche CFW1-Mäuse verwendet. Das Überleben der Tiere wird über 6 Tage protokolliert.

### C) Ausführungsbeispiele für pharmazeutische Zusammensetzungen

Die erfindungsgemäßen Substanzen können folgendermaßen in pharmazeutische Zubereitungen überführt werden:

### Tablette:

### Zusammensetzung:

100 mg der Verbindung des Beispiels 1, 50 mg Lactose (Monohydrat), 50 mg Maisstärke, 10 mg Polyvinylpyrolidon (PVP 25) (Fa. BASF, Deutschland) und 2 mg Magnesiumstearat.

Tablettengewicht 212 mg. Durchmesser 8 mm, Wölbungsradius 12 mm.

### Herstellung:

Die Mischung aus der Verbindung des Beispiels 1, Lactose und Stärke wird mit einer 5 %-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben).

### Orale Suspension:

### Zusammensetzung:

1000 mg der Verbindung des Beispiels 1, 1000 mg Ethanol (96 %), 400 mg Rhodigel (Xanthan gum) (Fa. FMC, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die Verbindung des Beispiels 1 wird der Suspension zugefügt. Unter Rühren erfolgt die Zugabe des Wassers. Bis zum Abschluss der Quellung des Rhodigels wird ca. 6h gerührt.

### Intravenös applizierbare Lösung:

### Zusammensetzung:

100-200 mg der Verbindung von Beispiel 1, 15 g Polyethylenglykol 400 und 250 g Wasser für Injektionszwecke.

### Herstellung:

Die Verbindung von Beispiel 1 wird zusammen mit Polyethylenglykol 400 in dem Wasser unter Rühren gelöst. Die Lösung wird sterilfiltriert (Porendurchmesser 0,22 µm) und unter aseptischen Bedingungen in hitzesterilisierte Infusionsflaschen abgefüllt. Diese werden mit Infusionsstopfen und Bördelkappen verschlossen.

## Patentansprüche

1. Verbindung der Formel 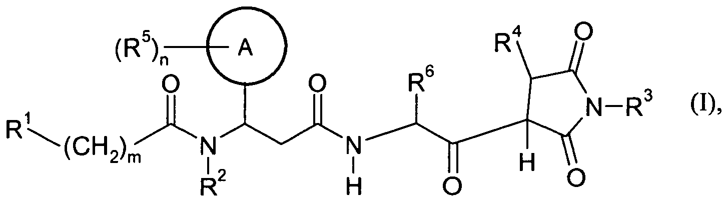 worin
R¹ 5- bis 10-gliedriges Heteroaryl mit bis zu 5 Heteroatomen aus der Reihe S, O und N bedeutet,
wobei Heteroaryl substituiert sein kann mit 0, 1, 2 oder 3 Substituenten R¹⁻¹, wobei die Substituenten R¹⁻¹ unabhängig voneinander ausgewählt werden aus der Gruppe bestehend aus Halogen, C₁-C₈-Alkyl, Nitro, Amino, C₁-C₆-Alkylamino, Cyano, Trifluormethyl, 3- bis 8-gliedriges Cycloalkyl, 4- bis 10-gliedriges Heterocyclyl mit bis zu 3 Heteroatomen und/oder Heterogruppen aus der Reihe N, O, S, SO, SO₂, C₆-C₁₄-Aryl, 5- bis 10-gliedriges Heteroaryl mit bis zu 5 Heteroatomen aus der Reihe S, O und N, Hydroxy, C₁-C₈-Alkoxy, C₆-C₁₄-Aryloxy, Benzyloxy, Carboxyl, C₁-C₈-Alkoxycarbonyl, Aminocarbonyl, C₁-C₈-Alkylcarbonylamino, C₁-C₆-Alkylaminocarbonyl und Aminosulfonyl,
oder
R¹ C₆-C₁₄-Aryl bedeutet,
wobei Aryl substituiert ist mit 1, 2 oder 3 Substituenten R¹⁻², wobei die Substituenten R¹⁻² unabhängig voneinander ausgewählt werden aus der Gruppe bestehend aus Halogen, C₁-C₈-Alkyl, Nitro, Amino, C₁-C₆-Alkylamino, Cyano, Trifluormethyl, 3- bis 8-gliedriges Cycloalkyl, 4-bis 10-gliedriges Heterocyclyl mit bis zu 3 Heteroatomen und/oder Heterogruppen aus der Reihe N, O, S, SO, SO₂, C₆-C₁₄-Aryl, 5- bis 10-gliedriges Heteroaryl mit bis zu 5 Heteroatomen aus der Reihe S, O und N, Hydroxy, C₁-C₈-Alkoxy, C₆-C₁₄-Aryloxy, Benzyloxy, Carboxyl, C₁-C₈-Alkoxycarbonyl, Aminocarbonyl, C₁-C₈-Alkylcarbonylamino, C₆-C₁₄-Arylcarbonylamino, C₁-C₆-Alkylaminocarbonyl und Aminosulfonyl,
oder
zwei Substituenten R¹⁻² zusammen mit den Kohlenstoffatomen, an die sie gebunden sind, ein 3- bis 8-gliedriges Cycloalkyl oder 4- bis 10-gliedriges Heterocyclyl mit bis zu 3 Heteroatomen und/oder Heterogruppen aus der Reihe N, O, S, SO, SO₂ bilden, wobei dieses Cycloalkyl oder Heterocyclyl substituiert sein kann mit 0, 1 oder 2 Substituenten R¹⁻²⁻¹, wobei die Substituenten R¹⁻²⁻¹ unabhängig voneinander ausgewählt werden aus der Gruppe bestehend aus Halogen, Nitro, Amino, Trifluormethyl, Hydroxy, C₁-C₈-Alkyl und C₁-C₈-Alkoxy,
R² Wasserstoff oder Methyl bedeutet,
R³ Wasserstoff, Hydroxy, Amino, C₁-C₃-Alkyl, Benzyl, C₁-C₃-Alkoxy, Benzyloxy, C₁-C₃-Alkylamino, C₁-C₃-Alkylcarbonylamino, Phenylcarbonylamino oder Benzylcarbonylamino bedeutet,
R⁴ Wasserstoff oder C₁-C₃-Alkyl bedeutet,
R⁵ Halogen, Trifluormethyl, Trifluormethoxy, Nitro, Amino, C₁-C₆-Alkylamino, Hydroxy, C₁-C₈-Alkyl, C₁-C₈-Alkoxy, Carboxyl, C₁-C₈-Alkoxycarbonyl, Aminocarbonyl, C₁-C₆-Alkylaminocarbonyl, C₆-C₁₄-Aryl oder 5- bis 10-gliedriges Heteroaryl mit bis zu 5 Heteroatomen aus der Reihe S, O und N bedeutet,
oder
zwei Substituenten R⁵ zusammen mit den Kohlenstoffatomen, an die sie gebunden sind, ein 3- bis 8-gliedriges Cycloalkyl oder 4- bis 10-gliedriges Heterocyclyl mit bis zu 3 Heteroatomen und/oder Heterogruppen aus der Reihe N, O, S, SO, SO₂ bilden, wobei dieses Cycloalkyl oder Heterocyclyl substituiert sein kann mit 0, 1 oder 2 Substituenten R⁵⁻¹, wobei die Substituenten R⁵⁻¹ unabhängig voneinander ausgewählt werden aus der Gruppe bestehend aus Halogen, Nitro, Amino, Trifluormethyl, Hydroxy, C₁-C₈-Alkyl und C₁-C₈-Alkoxy,
R⁶ C₁-C₈-Alkyl, 3- bis 8-gliedriges Cycloalkyl, 3- bis 8-gliedriges Cycloalkenyl oder 4- bis 10-gliedriges Heterocyclyl mit bis zu 3 Heteroatomen und/oder Heterogruppen aus der Reihe N, O, S, SO, SO₂ bedeutet,
wobei R⁶ substituiert sein kann mit 0, 1 oder 2 Substituenten R⁶⁻¹, wobei die Substituenten R⁶⁻¹ unabhängig voneinander ausgewählt werden aus der Gruppe bestehend aus Halogen, Nitro, Amino, Trifluormethyl, Hydroxy, C₁-C₈-Alkyl und C₁-C₈-Alkoxy,
n eine Zahl 0, 1, 2 oder 3 bedeutet,
wobei bei n gleich 2 oder 3 die Reste R⁵ gleich oder verschieden sein können,
m eine Zahl 0, 1, 2, 3 oder 4 bedeutet,
A C₆-C₁₄-Aryl oder 5- bis 10-gliedriges Heteroaryl mit bis zu 5 Heteroatomen aus der Reihe S, O und N bedeutet,
oder deren Salz, deren Solvat oder ein Solvat von deren Salz.

2. Verbindung nach Anspruch 1, **dadurch gekennzeichnet, dass** sie der Formel 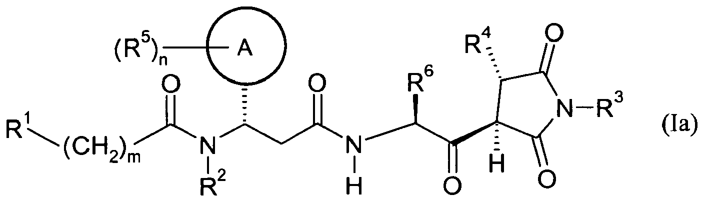 entspricht, worin R¹ bis R⁶, A, m und n die gleiche Bedeutung wie in Formel (I) haben.

3. Verbindung nach Anspruch 1 oder 2, **dadurch gekennzeichnet, dass**
R¹ Pyridyl, Imidazolyl, Thienyl, Furyl, Oxadiazolyl, Pyrazolyl, Pyrazinyl, Pyridazinyl, Pyrimidinyl, Chinolinyl oder Isochinolinyl bedeutet,
wobei R¹ substituiert sein kann mit 0, 1 oder 2 Substituenten R¹⁻¹, wobei die Substituenten R¹⁻¹ unabhängig voneinander ausgewählt werden aus der Gruppe bestehend aus Halogen, C₁-C₈-Alkyl, Amino, Trifluormethyl, Phenyl und C₁-C₈-Alkoxy,
oder
R¹ Phenyl oder Naphthyl bedeutet,
wobei Phenyl oder Naphthyl substituiert sind mit 1, 2 oder 3 Substituenten R¹⁻², wobei die Substituenten R¹⁻² unabhängig voneinander ausgewählt werden aus der Gruppe bestehend aus Halogen, C₁-C₄-Alkyl, Dimethylamino, Cyano, Trifluormethyl, 3- bis 7-gliedriges Cycloalkyl, 5- oder 6-gliedriges Heterocyclyl mit bis zu 2 Heteroatomen und/oder Heterogruppen aus der Reihe N, O, S, SO, SO₂, Phenyl, 5- oder 6-gliedriges Heteroaryl mit bis zu 4 Heteroatomen aus der Reihe S, O und N, C₁-C₃-Alkoxy, Phenyloxy, Benzyloxy, Phenylcarbonylamino und Aminosulfonyl,
oder
zwei Substituenten R¹⁻² zusammen mit den Kohlenstoffatomen, an die sie gebunden sind, ein 1,3-Benzodioxol oder ein 1,4-Benzodioxan bilden,
R² Wasserstoff bedeutet,
R³ Wasserstoff, Amino, Methyl, Methoxy, Ethoxy, Methylamino oder Dimethylamino bedeutet,
R⁴ Methyl bedeutet,
R⁵ Fluor, Chlor, Trifluormethyl, C₁-C₄-Alkoxy, Methoxycarbonyl, C₁-C₄-Alkyl, Phenyl oder Pyridyl bedeutet,
oder
zwei Substituenten R⁵ zusammen mit dem Phenylring, an den sie gebunden sind, ein 1,3-Benzodioxol oder ein 1,4-Benzodioxan bilden,
R⁶ C₃-C₆-Alkyl oder 3- bis 6-gliedriges Cycloalkyl bedeutet,
n eine Zahl 0, 1 oder 2 bedeutet,
wobei bei n gleich 2 die Reste R⁵ gleich oder verschieden sein können,
m eine Zahl 0, 1, 2 oder 3 bedeutet,
und
A Phenyl, Naphthyl, Pyridyl, Thienyl, Furanyl, Chinolinyl oder Isochinolinyl bedeutet.

4. Verbindung nach einem der Ansprüche 1 bis 3, **dadurch gekennzeichnet, dass**
R¹ Pyridyl, Thienyl, Furyl, Chinolinyl oder Isochinolinyl bedeutet,
wobei R¹ substituiert sein kann mit 0, 1 oder 2 Substituenten R¹⁻¹, wobei die Substituenten R¹⁻¹ unabhängig voneinander ausgewählt werden aus der Gruppe bestehend aus Halogen, C₁-C₄-Alkyl, Trifluormethyl, Phenyl und C₁-C₃-Alkoxy,
oder
R¹ Phenyl oder Naphthyl bedeutet,
wobei Phenyl oder Naphthyl substituiert sind mit 1, 2 oder 3 Substituenten R¹⁻², wobei die Substituenten R¹⁻² unabhängig voneinander ausgewählt werden aus der Gruppe bestehend aus Halogen, C₁-C₄-Alkyl, Dimethylamino, Cyano, Trifluormethyl, Tetrahydrofuran-2-yl, Tetrahydrothienyl, Pyrrolidin-2-yl, Pyrrolidin-3-yl, Pyrrolinyl, Pyranyl, Piperidin-1-yl, Piperidin-2-yl, Piperidin-3-yl, Piperidin-4-yl, Thiopyranyl, Morpholin-1-yl, Morpholin-2-yl, Morpholin-3-yl, Piperazin-1-yl, Piperazin-2-yl, Thienyl, Furyl, Pyrrolyl, Thiazolyl, Oxazolyl, Oxadiazolyl, Pyrazolyl, Imidazolyl, Pyridyl, Pyrimidyl, Pyridazinyl, Pyrazinyl,, C₁-C₃-Alkoxy, Phenyloxy oder Benzyloxy,
oder
zwei Substituenten R¹⁻² zusammen mit den Kohlenstoffatomen, an die sie gebunden sind, ein 1,3-Benzodioxol oder ein 1,4-Benzodioxan bilden,
R² Wasserstoffbedeutet,
R³ Wasserstoff, Amino, Methylamino oder Dimethylamino bedeutet,
R⁴ Methyl bedeutet,
R⁵ Fluor, Chlor, Trifluormethyl, C₁-C₃-Alkoxy, C₁-C₄-Alkyl, Phenyl oder Pyridyl bedeutet,
R⁶ Isopropyl, tert-Butyl, Isopentyl, Cyclopentyl oder Cyclohexyl bedeutet,
n eine Zahl 0, 1 oder 2 bedeutet,
wobei bei n gleich 2 die Reste R⁵ gleich oder verschieden sein können,
m eine Zahl 0, 1 oder 2 bedeutet,
und
A Phenyl, Naphthyl, Pyridyl, Thienyl, Chinolinyl oder Isochinolinyl bedeutet.

5. Verbindung nach einem der Ansprüche 1 bis 4, **dadurch gekennzeichnet, dass**
R¹ Pyridyl, Thienyl, Furyl, Chinolinyl oder Isochinolinyl bedeutet,
wobei R¹ substituiert sein kann mit 0, 1 oder 2 Substituenten R¹⁻¹, wobei die Substituenten R¹⁻¹ unabhängig voneinander ausgewählt werden aus der Gruppe bestehend aus Fluor, Chlor, Trifluormethyl, C₁-C₄-Alkyl, Phenyl und Methoxy.

6. Verbindung nach einem der Ansprüche 1 bis 4, **dadurch gekennzeichnet, dass**
R¹ Phenyl oder Naphthyl bedeutet,
wobei Phenyl oder Naphthyl substituiert sind mit 1, 2 oder 3 Substituenten R¹⁻², wobei die Substituenten R¹⁻² unabhängig voneinander ausgewählt werden aus der Gruppe bestehend aus Halogen, C₁-C₄-Alkyl, Dimethylamino, Cyano, Trifluormethyl, Tetrahydrofuran-2-yl, Tetrahydrothienyl, Pyrrolidin-2-yl, Pyrrolidin-3-yl, Pyrrolinyl, Pyranyl, Piperidin-1-yl, Piperidin-2-yl, Piperidin-3-yl, Piperidin-4-yl, Thiopyranyl, Morpholin-1-yl, Morpholin-2-yl, Morpholin-3-yl, Piperazin-1-yl, Piperazin-2-yl, Thienyl, Furyl, Pyrrolyl, Thiazolyl, Oxazolyl, Oxadiazolyl, Pyrazolyl, Imidazolyl, Pyridyl, Pyrimidyl, Pyridazinyl, Pyrazinyl,, C₁-C₃-Akoxy, Phenyloxy oder Benzyloxy,
oder
zwei Substituenten R¹⁻² zusammen mit den Kohlenstoffatomen, an die sie gebunden sind, ein 1,3-Benzodioxol oder ein 1,4-Benzodioxan bilden.

7. Verbindung nach einem der Ansprüche 1, 2, 5 oder 6, **dadurch gekennzeichnet, dass** R² Wasserstoff bedeutet.

8. Verbindung nach einem der Ansprüche 1 bis 7, **dadurch gekennzeichnet, dass** R³ Wasserstoffoder Amino bedeutet.

9. Verbindung nach einem der Ansprüche 1, 2 und 5 bis 8, **dadurch gekennzeichnet, dass** R⁴ Methyl bedeutet.

10. Verbindung nach einem der Ansprüche 1 bis 9, **dadurch gekennzeichnet, dass** n die Zahl Null bedeutet.

11. Verbindung nach einem der Ansprüche 1 bis 10, **dadurch gekennzeichnet, dass** n die Zahl 1 bedeutet, A Phenyl bedeutet und R⁵ Fluor, Chlor, Trifluormethyl, C₁-C₈-Alkoxy, C₁-C₄-Alkyl, Phenyl oder Pyridyl bedeutet, wobei R⁵ in der meta- oder para-Position zur Verknüpfungsstelle des Phenyl-Ringes vorliegt.

12. Verbindung nach einem der Ansprüche 1, 2 und 5 bis 11, **dadurch gekennzeichnet, dass** R⁶ C₃-C₆-Alkyl oder 3- bis 6-gliedriges Cycloalkyl bedeutet.

13. Verbindung nach einem der Ansprüche 1 bis 12, **dadurch gekennzeichnet, dass** m die Zahl Null bedeutet.

14. Verbindung nach einem der Ansprüche 1 bis 3 oder 5 bis 13, **dadurch gekennzeichnet, dass** A Phenyl, Naphthyl, Pyridyl, Thienyl, Chinolinyl oder Isochinolinyl bedeutet.

15. Verfahren zur Herstellung einer Verbindung der Formel (I) nach Anspruch 1, **dadurch gekennzeichnet, dass**
[A] eine Verbindung der Formel 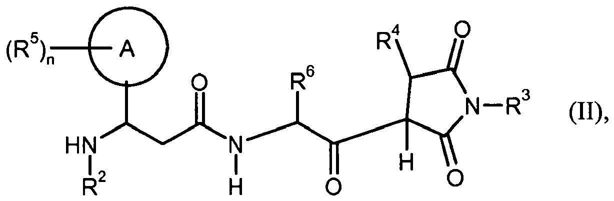 worin R² bis R⁶, A und n die in Anspruch 1 angegebene Bedeutung aufweisen, mit einer Verbindung der Formel 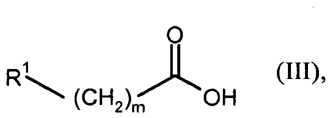 worin R¹ und m die in Anspruch 1 angegebene Bedeutung aufweisen, umgesetzt werden,
oder
[B] eine Verbindung der Formel 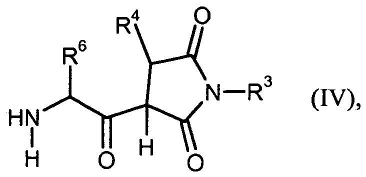 worin R³, R⁴ und R⁶ die in Anspruch 1 angegebene Bedeutung aufweisen, mit einer Verbindung der Formel 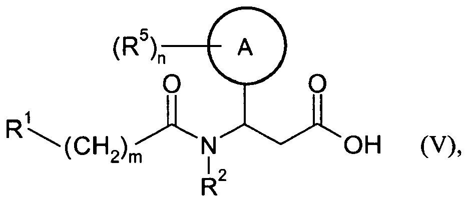 worin R¹, R², R⁵, A, m und n die in Anspruch 1 angegebene Bedeutung aufweisen, umgesetzt werden.

16. Verbindung nach einem der Ansprüche 1 bis 14 zur Behandlung und/oder Prophylaxe von Krankheiten.

17. Arzneimittel enthaltend mindestens eine Verbindung nach einem der Ansprüche 1 bis 14 in Kombination mit mindestens einem pharmazeutisch verträglichen, pharmazeutisch unbedenklichen Träger oder sonstigen Exzipienten.

18. Verwendung einer Verbindung nach einem der Ansprüche 1 bis 14 zur Herstellung eines Arzneimittels zur Behandlung und/oder Prophylaxe von bakteriellen Erkrankungen.

19. Arzneimittel nach Anspruch 17 zur Behandlung und/oder Prophylaxe von bakteriellen Infektionen.

## Claims

1. Compound of the formula 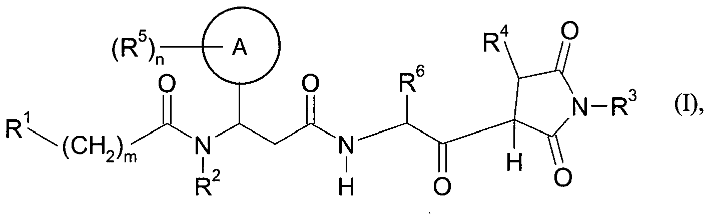 in which
R¹ is 5- to 10-membered heteroaryl having up to 5 heteroatoms from the series S, O and N,
where heteroaryl can be substituted by 0, 1, 2 or 3 substituents R¹⁻¹, the substituents R¹⁻¹ being selected independently of one another from the group consisting of halogen, C₁-C₈ alkyl, nitro, amino, C₁-C₆ alkylamino, cyano, trifluoromethyl, 3- to 8-membered cycloalkyl, 4- to 10-membered heterocyclyl having up to 3 heteroatoms and/or hetero-groups from the series N, O, S, SO₁ SO₂, C₆-C₁₄ aryl, 5- to 10-membered heteroaryl having up to 5 hereoatoms from the series S, O and N, hydroxyl, C₁-C₈ alkoxy, C₆-C₁₄ aryloxy, benzyloxy, carboxyl, C₁-C₈ alkoxycarbonyl, aminocarbonyl, C₁-C₈ alkylcarbonylamino, C₁-C₆ alkylaminocarbonyl and aminosulfonyl,
or
R¹ is C₆-C₁₄ aryl,
where aryl is substituted by 1, 2 or 3 substituents R¹⁻², the substituents R¹⁻² being selected independently of one another from the group consisting of halogen, C₁-C₈ alkyl, nitro, amino, C₁-C₆ alkylamino, cyano, trifluoromethyl, 3- to 8-membered cycloalkyl, 4- to 10-membered heterocyclyl having up to 3 heteroatoms and/or hetero-groups from the series N, O, S, SO₁ SO₂, C₆-C₁₄ aryl, 5- to 10-membered heteroaryl having up to 5 hereoatoms from the series S, O and N, hydroxyl, C₁-C₈ alkoxy, C₆-C₁₄ aryloxy, benzyloxy, carboxyl, C₁-C₈ alkoxycarbonyl, aminocarbonyl, C₁-C₈ alkylcarbonylamino, C₆-C₁₄ arylcarbonylamino, C₁-C₆ alkylaminocarbonyl and aminosulfonyl,
or
two substituents R¹⁻², together with the carbon atoms to which they are attached, form a 3- to 8-membered cycloalkyl or 4- to 10-membered heterocyclyl having up to 3 heteroatoms and/or hetero-groups from the series N, O, S, SO, SO₂, where this cycloalkyl or heterocyclyl can be substituted by 0, 1 or 2 substituents R¹⁻²⁻¹, the substituents R¹⁻²⁻¹ being selected independently of one another from the group consisting of halogen, nitro, amino, trifluoromethyl, hydroxyl, C₁-C₈ alkyl and C₁-C₈ alkoxy,
R² is hydrogen or methyl,
R³ is hydrogen, hydroxyl, amino, C₁-C₃ alkyl, benzyl, C₁-C₃ alkoxy, benzyloxy, C₁-C₃ alkylamino, C₁-C₃ alkylcarbonylamino, phenylcarbonylamino or benzylcarbonylamino,
R⁴ is hydrogen or C₁-C₃ alkyl,
R⁵ is halogen, trifluoromethyl, trifluoromethoxy, nitro, amino, C₁-C₆ alkylamino, hydroxyl, C₁-C₈ alkyl, C₁-C₈ alkoxy, carboxyl, C₁-C₈ alkoxycarbonyl, aminocarbonyl, C₁-C₆ alkylaminocarbonyl, C₆-C₁₄ aryl or 5- to 10-membered heteroaryl having up to 5 S, O and N,
or
two substituents R⁵ together with the carbon atoms to which they are attached form a 3- to 8-membered cycloalkyl or 4- to 10-membered heterocyclyl having up to 3 heteroatoms and/or hetero-groups from the series N, O, S, SO, SO₂, where this cycloalkyl or heterocyclyl can be substituted by 0, 1 or 2 substituents R⁵⁻¹, the substituents R⁵⁻¹ being selected independently of one another from the group consisting of halogen, nitro, amino, trifluoromethyl, hydroxyl, C₁-C₈ alkyl and C₁-C₈ alkoxy,
R⁶ is C₁-C₈ alkyl, 3- to 8-membered cycloalkyl, 3- to 8-membered cycloalkenyl or 4- to 10-membered heterocyclyl having up to 3 heteroatoms and/or hetero-groups from the series N, O, S, SO, SO₂,
it being possible for R⁶ to be substituted by 0, 1 or 2 substituents R⁶⁻¹, the substituents R⁶⁻¹ being selected independently of one another from the group consisting of halogen, nitro, amino, trifluoromethyl, hydroxyl, C₁-C₈ alkyl and C₁-C₈ alkoxy,
n is a number 0, 1, 2 or 3,
it being possible for the radicals R⁵ to be identical or different when n is 2 or 3,
m is a number 0, 1, 2, 3 or 4,
A is C₆-C₁₄ aryl or 3- to 8-membered cycloalkyl, 4- to 10-membered heterocyclyl having up to 3 heteroatoms and/or hetero-groups from the series N, O, S, SO₁ SO₂, C₆-C₁₄ aryl, 5- to 10-membered heteroaryl having up to 5 hereoatoms from the series S, O and N,
or a salt thereof, a solvate thereof or a solvate of a salt thereof.

2. Compound according to Claim 1, **characterized in that** it corresponds to the formula 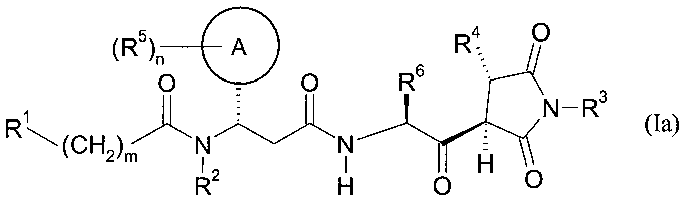 in which R¹ to R⁶, A, m and n have the same definition as in formula (I).

3. Compound according to Claim 1 or 2, **characterized in that**
R¹ is pyridyl, imidazolyl, thienyl, furyl, oxadiazolyl, pyrazolyl, pyrazinyl, pyridazinyl, pyrimidinyl, quinolinyl or isoquinolinyl,
where R¹ can be substituted by 0, 1 or 2 substituents R¹⁻¹, the substituents R¹⁻¹ being selected independently of one another from the group consisting of halogen, C₁-C₈ alkyl, amino, trifluoromethyl, phenyl and C₁-C₈ alkoxy,
or
R¹ is phenyl or naphthyl,
where phenyl or naphthyl are substituted by 1, 2 or 3 substituents R¹⁻², the substituents R¹⁻² being selected independently of one another from the group consisting of halogen, C₁-C₄ alkyl, dimethylamino, cyano, trifluoromethyl, 3- to 7-membered cycloalkyl, 5- or 6-membered heterocyclyl having up to 2 heteroatoms and/or hetero-groups from the series N, O, S, SO, SO₂, phenyl, 5- or 6-membered heteroaryl having up to 4 heteroatoms from the series S, O and N, C₁-C₃ alkoxy, phenyloxy, benzyloxy, phenylcarbonylamino and aminosulfonyl,
or
two substituents R¹⁻², together with the carbon atoms to which they are attached, form a 1,3-benzodioxole or a 1,4-benzodioxane,
R² is hydrogen,
R³ is hydrogen, amino, methyl, methoxy, ethoxy, methylamino or dimethylamino,
R⁴ is methyl,
R⁵ is fluoro, chloro, trifluoromethyl, C₁-C₄ alkoxy, methoxycarbonyl, C₁-C₄ alkyl, phenyl or pyridyl,
or
two substituents R⁵, together with the phenyl ring to which they are attached, form a 1,3-benzodioxole or a 1,4-benzodioxane,
R⁶ is C₃-C₆ alkyl or 3- to 6-membered cycloalkyl,
n is a number 0, 1 or 2,
and, if n is 2, the radicals R⁵ can be identical or different,
m is a number 0, 1, 2 or 3,
and
A is phenyl, naphthyl, pyridyl, thienyl, furanyl, quinolinyl or isoquinolinyl.

4. Compound according to any one of Claims 1 to 3, **characterized in that**
R¹ is pyridyl, thienyl, furyl, quinolinyl or isoquinolinyl,
where R¹ can be substituted by 0, 1 or 2 substituents R¹⁻¹, the substituents R¹⁻¹ being selected independently of one another from the group consisting of halogen, C₁-C₄ alkyl, trifluoromethyl, phenyl and C₁-C₃ alkoxy,
or
R¹ is phenyl or naphthyl,
where phenyl or naphthyl are substituted by 1, 2 or 3 substituents R¹⁻², the substituents R¹⁻² being selected independently of one another from the group consisting of halogen, C₁-C₄ alkyl, dimethylamino, cyano, trifluoromethyl, tetrahydrofuran-2-yl, tetrahydrothienyl, pyrrolidin-2-yl, pyrrolidin-3-yl, pyrrolinyl, pyranyl, piperidin-1-yl, piperidin-2-yl, piperidin-3-yl, piperidin-4-yl, thiopyranyl, morpholin-1-yl, morpholin-2-yl, morphoilin-3-yl, piperazin-1-yl, pipearazin-2-yl, thienyl, furyl, pyrrolyl, thiazolyl, oxazolyl, oxadiazoyl, pyrazolyl, imidazolyl, pyridyl, pyrimidyl, pyridazinyl, pyrazinyl, C₁-C₃ alkoxy, phenyloxy or benzyloxy,
or
two substituents R¹⁻², together with the carbon atoms to which they are attached, form a 1,3-benzodioxole or a 1,4-benzodioxane,
R² is hydrogen,
R³ is hydrogen, amino, methylamino or dimethylamino,
R⁴ is methyl,
R⁵ is fluoro, chloro, trifluoromethyl, C₁-C₃ alkoxy, C₁-C₄ alkyl, phenyl or pyridyl,
R⁶ is isopropyl, tert-butyl, isopentyl, cyclopentyl or cyclohexyl,
n is a number 0, 1 or 2, and, if n is 2, the radicals R⁵ can be identical or different,
m is a number 0, 1 or 2,
and
A is phenyl, naphthyl, pyridyl, thienyl, quinolinyl or isoquinolinyl.

5. Compound according to any one of Claims 1 to 4, **characterized in that**
R¹ is pyridyl, thienyl, furyl, quinolinyl or isoquinolinyl,
where R¹ can be substituted by 0, 1 or 2 substituents R¹⁻¹, the substituents R¹⁻¹ being selected independently of one another from the group consisting of fluoro, chloro, trifluoromethyl, C₁-C₄ alkyl, phenyl and methoxy.

6. Compound according to any of Claims 1 to 4, **characterized in that**
R¹ is phenyl or naphthyl,
where phenyl or naphthyl are substituted by 1, 2 or 3 substituents R¹⁻², the substituents R¹⁻² being selected independently of one another from the group consisting of halogen, C₁-C₄ alkyl, dimethylamino, cyano, trifluoromethyl, tetrahydrofuran-2-yl, tetrahydrothienyl, pyrrolidin-2-yl, pyrrolidin-3-yl, pyrrolinyl, pyranyl, piperidin-1-yl, piperidin-2-yl, piperidin-3-yl, piperidin-4-yl, thiopyranyl, morpholin-1-yl, morpholin-2-yl, morphoilin-3-yl, piperazin-1-yl, pipearazin-2-yl, thienyl, furyl, pyrrolyl, thiazolyl, oxazolyl, oxadiazoyl, pyrazolyl, imidazolyl, pyridyl, pyrimidyl, pyridazinyl, pyrazinyl, C₁-C₃ alkoxy, phenyloxy or benzyloxy,
or
two substituents R¹⁻², together with the carbon atoms to which they are attached, form a 1,3-benzodioxole or a 1,4-benzodioxane.

7. Compound according to any one of Claims 1, 2, 5 and 6, **characterized in that** R² is hydrogen.

8. Compound according to any one of Claims 1 to 7, **characterized in that** R³ is hydrogen or amino.

9. Compound according to any one of Claims 1, 2 and 5 to 8, **characterized in that** R⁴ is methyl.

10. Compound according to any one of Claims 1 to 9, **characterized in that** n is the number zero.

11. Compound according to any one of Claims 1 to 10, **characterized in that** n is the number 1, A is phenyl and R⁵ is fluoro, chloro, trifluoromethyl, C₁-C₈ alkoxy, C₁-C₄ alkyl, phenyl or pyridyl, R⁵ being positioned meta or para to the linkage site of the phenyl ring.

12. Compound according to any one of Claims 1, 2 and 5 to 11, **characterized in that** R⁶ is C₃-C₆ alkyl or 3- to 6-membered cycloalkyl.

13. Compound according to any one of Claims 1 to 12, **characterized in that** m is the number zero.

14. Compound according to any one of Claims 1 to 3 and 5 to 13, **characterized in that** A is phenyl, naphthyl, pyridyl, thienyl, quinolinyl or isoquinolinyl.

15. Process for preparing a compound of the formula (I) according to Claim 1, **characterized in that**
[A] a compound of the formula 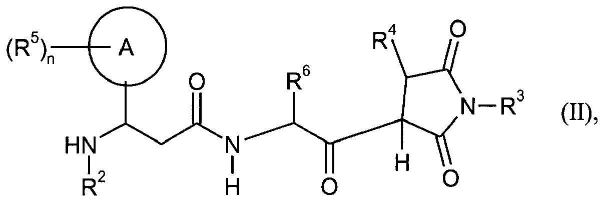 in which R² to R⁶, A and n are as defined in Claim 1, is reacted with a compound of the formula 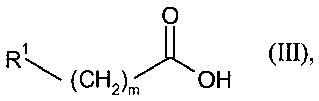 in which R¹ and m are as defined in Claim 1,
or
[B] a compound of the formula 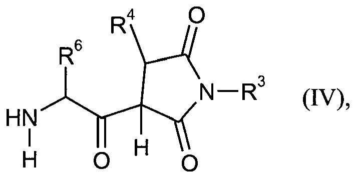 in which R³, R⁴ and R⁶ are as defined in Claim 1, is reacted with a compound of the formula 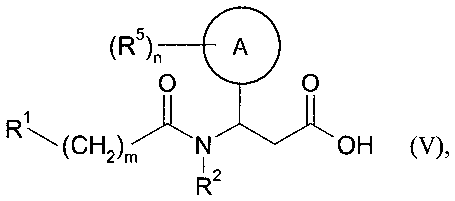 in which R¹, R², R⁵, A, m and n are as defined in Claim 1.

16. Compound according to any one of Claims 1 to 14 for the treatment and/or prophylaxis of diseases.

17. Medicinal product comprising at least one compound according to any one of Claims 1 to 14 in combination with at least one pharmaceutically compatible, pharmaceutically acceptable carrier or other excipients.

18. Use of a compound according to any one of Claims 1 to 14 for producing a medicinal product for the treatment and/or prophylaxis of bacterial diseases.

19. Medicinal product according to Claim 17 for the treatment and/or prophylaxis of bacterial infections.

## Revendications

1. Composé de formule 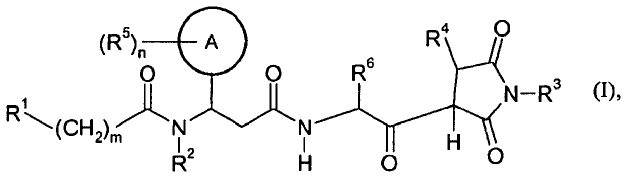 où
R¹ signifie hétéroaryle de 5 à 10 chaînons comprenant jusqu'à 5 hétéroatomes de la série S, O et N, hétéroaryle pouvant être substitué par 0, 1, 2 ou 3 substituants R¹⁻¹, les substituants R¹⁻¹ étant choisis indépendamment l'un de l'autre dans le groupe constitué par halogène, C₁-C₈-alkyle, nitro, amino, C₁-C₆-alkylamino, cyano, trifluorométhyle, cycloalkyle de 3 à 8 chaînons, hétérocyclyle de 4 à 10 chaînons comprenant jusqu'à 3 hétéroatomes et/ou hétérogroupes de la série N, O, S, SO, SO₂, C₆-C₁₄-aryle, hétéroaryle de 5 à 10 chaînons comprenant jusqu'à 5 hétéroatomes de la série S, O et N, hydroxy, C₁-C₈-alcoxy, C₆-C₁₄-aryloxy, benzyloxy, carboxyle, C₁-C₈-alcoxycarbonyle, aminocarbonyle, C₁-C₈-alkylcarbonylamino, C₁-C₆-alkylaminocarbonyle et aminosulfonyle, ou
R¹ signifie C₆-C₁₄-aryle,
aryle étant substitué par 1, 2 ou 3 substituants R¹⁻², les substituants R¹⁻² étant choisis indépendamment l'un de l'autre dans le groupe constitué par halogène, C₁-C₈-alkyle, nitro, amino, C₁-C₆-alkylamino, cyano, trifluorométhyle, cycloalkyle de 3 à 8 chaînons, hétérocyclyle de 4 à 10 chaînons comprenant jusqu'à 3 hétéroatomes et/ou hétérogroupes de la série N, 0, S, SO, SO₂, C₆-C₁₄-aryle, hétéroaryle de 5 à 10 chaînons comprenant jusqu'à 5 hétéroatomes de la série S, 0 et N, hydroxy, C₁-C₈-alcoxy, C₆-C₁₄-aryloxy, benzyloxy, carboxyle, C₁-C₈-alcoxycarbonyle, aminocarbonyle, C₁-C₈-alkylcarbonylamino, C₆-C₁₄-arylcarbonylamino, C₁-C₆-alkylaminocarbonyle et aminosulfonyle, ou
deux substituants R¹⁻², ensemble avec les atomes de carbone auxquels ils sont liés, formant ensemble cycloalkyle de 3 à 8 chaînons ou hétérocyclyle de 4 à 10 chaînons comprenant jusqu'à 3 hétéroatomes et/ou hétérogroupes de la série N, O, S, SO, SO₂, ce cycloalkyle ou ce hétérocyclyle pouvant être substitué par 0, 1 ou 2 substituants R¹⁻²⁻¹, les substituants R¹⁻²⁻¹ étant choisis indépendamment l'un de l'autre dans le groupe constitué par halogène, nitro, amino, trifluorométhyle, hydroxy, C₁-C₈-alkyle et C₁-C₈-alcoxy,
R² signifie hydrogène ou méthyle,
R³ signifie hydrogène, hydroxy, amino, C₁-C₃-alkyle, benzyle, C₁-C₃-alcoxy, benzyloxy, C₁-C₃-alkylamino, C₁-C₃-alkylcarbonylamino, phénylcarbonylamino ou benzylcarbonylamino,
R⁴ signifie hydrogène ou C₁-C₃-alkyle,
R⁵ signifie halogène, trifluorométhyle, trifluorométhoxy, nitro, amino, C₁-C₆-alkylamino, hydroxy, C₁-C₈-alkyle, C₁-C₈-alcoxy, carboxyle, C₁-C₈-alcoxycarbonyle, aminocarbonyle, C₁-C₆-alkylaminocarbonyle, C₆-C₁₄-aryle ou hétéroaryle de 5 à 10 chaînons comprenant jusqu'à 5 hétéroatomes de la série S, 0 et N, ou
deux substituants R⁵, ensemble avec les atomes de carbone auxquels ils sont liés, forment ensemble cycloalkyle de 3 à 8 chaînons ou hétérocyclyle de 4 à 10 chaînons comprenant jusqu'à 3 hétéroatomes et/ou hétérogroupes de la série N, 0, S, SO, SO₂, ce cycloalkyle ou ce hétérocyclyle pouvant être substitué par 0, 1 ou 2 substituants R⁵⁻¹, les substituants R⁵⁻¹ étant choisis indépendamment l'un de l'autre dans le groupe constitué par halogène, nitro, amino, trifluorométhyle, hydroxy, C₁-C₈-alkyle et C₁-C₈-alcoxy,
R⁶ signifie C₁-C₈-alkyle, cycloalkyle de 3 à 8 chaînons, cycloalcényle de 3 à 8 chaînons ou hétérocyclyle de 4 à 10 chaînons comprenant jusqu'à 3 hétéroatomes et/ou hétérogroupes de la série N, O, S, SO, SO₂,
R⁶ pouvant être substitué par 0, 1 ou 2 substituants R⁶⁻¹, les substituants R⁶⁻¹ étant choisis, indépendamment l'un de l'autre, dans le groupe constitué par halogène, nitro, amino, trifluorométhyle, hydroxy, C₁-C₈-alkyle et C₁-C₈-alcoxy,
n vaut un nombre 0, 1, 2 ou 3, où, pour n valant 2 ou 3, les radicaux R⁵ peuvent être identiques ou différents,
m vaut un nombre 0, 1, 2, 3 ou 4,
A signifie C₆-C₁₄-aryle ou hétéroaryle de 5 à 10 chaînons comprenant jusqu'à 5 hétéroatomes de la série S, O et N,
ou son sel, son solvate ou un solvate de son sel.

2. Composé selon la revendication 1, **caractérisé en ce qu'**il correspond à la formule 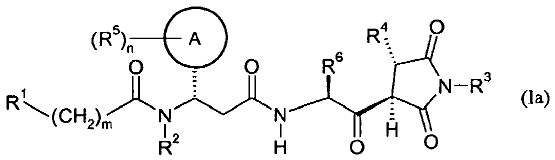 R¹ à R⁶, A, m et n ayant la même signification que dans la formule (I).

3. Composé selon la revendication 1 ou 2, **caractérisé en ce que**
R¹ signifie pyridyle, imidazolyle, thiényle, furyle, oxadiazolyle, pyrazolyle, pyrazinyle, pyridazinyle, pyrimidinyle, quinoléinyle ou isoquinoléinyle,
R¹ pouvant être substitué par 0, 1 ou 2 substituants R¹⁻¹, les substituants R¹⁻¹ étant choisis, indépendamment l'un de l'autre, dans le groupe constitué par halogène, C₁-C₈-alkyle, amino, trifluorométhyle, phényle et C₁-C₈-alcoxy, ou
R¹ signifie phényle ou naphtyle,
phényle ou naphtyle étant substitué par 1, 2 ou 3 substituants R¹⁻², les substituants R¹⁻² étant choisis indépendamment l'un de l'autre dans le groupe constitué par halogène, C₁-C₄-alkyle, diméthylamino, cyano, trifluorométhyle, cycloalkyle de 3 à 7 chaînons, hétérocyclyle de 5 ou 6 chaînons, comprenant jusqu'à 2 hétéroatomes et/ou hétérogroupes de la série N, 0, S, SO, SO₂, phényle, hétéroaryle de 5 ou 6 chaînons comprenant jusqu'à 4 hétéroatomes de la série S, O et N, C₁-C₃-alcoxy, phényloxy, benzyloxy, phénylcarbonylamino et aminosulfonyle, ou deux substituants R¹⁻² formant ensemble avec les atomes de carbone auxquels ils sont liés un 1,3-benzodioxole ou un 1,4-benzodioxane,
R² signifie hydrogène,
R³ signifie hydrogène, amino, méthyle, méthoxy, éthoxy, méthylamino ou diméthylamino,
R⁴ signifie méthyle,
R⁵ signifie fluor, chlore, trifluorométhyle, C₁-C₄-alcoxy, méthoxycarbonyle, C₁-C₄-alkyle, phényle ou pyridyle, ou
deux substituants R⁵ forment ensemble avec le cycle phényle auquel ils sont liés un 1,3-benzodioxole ou un 1,4-benzodioxane,
R⁶ signifie C₃-C₆-alkyle ou cycloalkyle de 3 à 6 chaînons,
n vaut un nombre 0, 1 ou 2,
où, pour n valant 2, les radicaux R⁵ peuvent être identiques ou différents,
m vaut un nombre 0, 1, 2 ou 3, et
A signifie phényle, naphtyle, pyridyle, thiényle, furannyle, quinoléinyle ou isoquinoléinyle.

4. Composé selon l'une quelconque des revendications 1 à 3, **caractérisé en ce que**
R¹ signifie pyridyle, thiényle, furyle, quinoléinyle ou isoquinoléinyle,
R¹ pouvant être substitué par 0, 1 ou 2 substituants R¹⁻¹, les substituants R¹⁻¹ étant choisis, indépendamment l'un de l'autre, dans le groupe constitué par halogène, C₁-C₄-alkyle, trifluorométhyle, phényle et C₁-C₃-alcoxy, ou
R¹ signifie phényle ou naphtyle,
phényle ou naphtyle étant substitué par 1, 2 ou 3 substituants R¹⁻², les substituants R¹⁻² étant choisis indépendamment l'un de l'autre dans le groupe constitué par halogène, C₁-C₄-alkyle, diméthylamino, cyano, trifluorométhyle, tétrahydrofurann-2-yle, tétrahydrothiényle, pyrrolidin-2-yle, pyrrolidin-3-yle, pyrrolinyle, pyrannyle, pipéridin-1-yle, pipéridin-2-yle, pipéridin-3-yle, pipéridin-4-yle, thiopyrannyle, morpholin-1-yle, morpholin-2-yle, morpholin-3-yle, pipérazin-1-yle, pipérazin-2-yle, thiényle, furyle, pyrrolyle, thiazolyle, oxazolyle, oxadiazolyle, pyrazolyle, imidazolyle, pyridyle, pyrimidyle, pyridazinyle, pyrazinyle, C₁-C₃-alcoxy, phényloxy ou benzyloxy, ou
deux substituants R¹⁻² formant ensemble avec les atomes de carbone auxquels ils sont liés un 1,3-benzodioxole ou un 1,4-benzodioxane,
R² signifie hydrogène,
R³ signifie hydrogène, amino, méthylamino ou diméthylamino,
R⁴ signifie méthyle,
R⁵ signifie fluor, chlore, trifluorométhyle, C₁-C₃-alcoxy, C₁-C₄-alkyle, phényle ou pyridyle, ou
R⁶ signifie isopropyle, tert-butyle, isopentyle, cyclopentyle ou cyclohexyle,
n vaut un nombre 0, 1 ou 2,
où, pour n valant 2, les radicaux R⁵ peuvent être identiques ou différents,
m vaut un nombre 0, 1 ou 2, et
A signifie phényle, naphtyle, pyridyle, thiényle, quinoléinyle ou isoquinoléinyle.

5. Procédé selon l'une quelconque des revendications 1 à 4, **caractérisé en ce que**
R¹ signifie pyridyle, thiényle, furyle, quinoléinyle ou isoquinoléinyle,
R¹ pouvant être substitué par 0, 1 ou 2 substituants R¹⁻¹, les substituants R¹⁻¹ étant choisis, indépendamment l'un de l'autre, dans le groupe constitué par fluor, chlore, trifluorométhyle, C₁-C₄-alkyle, phényle et méthoxy.

6. Procédé selon l'une quelconque des revendications 1 à 4, **caractérisé en ce que**
R¹ signifie phényle ou naphtyle,
phényle ou naphtyle étant substitué par 1, 2 ou 3 substituants R¹⁻², les substituants R¹⁻² étant choisis indépendamment l'un de l'autre dans le groupe constitué par halogène, C₁-C₄-alkyle, diméthylamino, cyano, trifluorométhyle, tétrahydrofurann-2-yle, tétrahydrothiényle, pyrrolidin-2-yle, pyrrolidin-3-yle, pyrrolinyle, pyrannyle, pipéridin-1-yle, pipéridin-2-yle, pipéridin-3-yle, pipéridin-4-yle, thiopyrannyle, morpholin-1-yle, morpholin-2-yle, morpholin-3-yle, pipérazin-1-yle, pipérazin-2-yle, thiényle, furyle, pyrrolyle, thiazolyle, oxazolyle, oxadiazolyle, pyrazolyle, imidazolyle, pyridyle, pyrimidyle, pyridazinyle, pyrazinyle, C₁-C₃-alcoxy, phényloxy ou benzyloxy, ou
deux substituants R¹⁻² formant ensemble avec les atomes de carbone auxquels ils sont liés un 1,3-benzodioxole ou un 1,4-benzodioxane.

7. Composé selon l'une quelconque des revendications 1, 2, 5 ou 6, **caractérisé en ce que** R² signifie hydrogène.

8. Composé selon l'une quelconque des revendications 1 à 7, **caractérisé en ce que** R³ signifie hydrogène ou amino.

9. Composé selon l'une quelconque des revendications 1, 2 et 5 à 8, **caractérisé en ce que** R⁴ signifie méthyle.

10. Composé selon l'une quelconque des revendications 1 à 9, **caractérisé en ce que** n vaut le nombre zéro.

11. Composé selon l'une quelconque des revendications 1 à 10, **caractérisé en ce que** n vaut le nombre 1, A signifie phényle et R⁵ signifie fluor, chlore, trifluorométhyle, C₁-C₈-alcoxy, C₁-C₄-alkyle, phényle ou pyridyle, R⁵ se trouvant dans la position méta ou para par rapport au site de liaison du cycle phényle.

12. Composé selon l'une quelconque des revendications 1, 2 et 5 à 11, **caractérisé en ce que** R⁶ signifie C₃-C₆-alkyle ou cycloalkyle de 3 à 6 chaînons.

13. Composé selon l'une quelconque des revendications 1 à 12, **caractérisé en ce que** m vaut le nombre zéro.

14. Composé selon l'une quelconque des revendications 1 à 3 ou 5 à 13, **caractérisé en ce que** A signifie phényle, naphtyle, pyridyle, thiényle, quinoléinyle ou isoquinoléinyle.

15. Procédé pour la préparation d'un composé de formule (I) selon la revendication 1, **caractérisé en ce qu'**on transforme
[A] un composé de formule 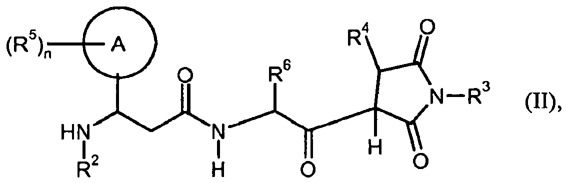 dans laquelle R² à R⁶, A et n présentent la signification indiquée dans la revendication 1,
avec un composé de formule 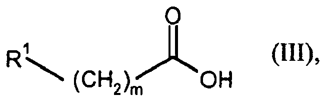 dans laquelle R¹ et m présentent la signification indiquée dans la revendication 1, ou
[B] un composé de formule 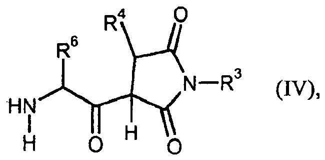 dans laquelle R³, R⁴ et R⁶ présentent la signification indiquée dans la revendication 1, avec un composé de formule 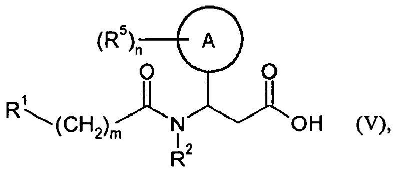 dans laquelle R¹, R², R⁵, A, m et n présentent la signification indiquée dans la revendication 1.

16. Composé selon l'une quelconque des revendications 1 à 14 destiné au traitement et/ou à la prophylaxie de maladies.

17. Médicament contenant au moins un composé selon l'une quelconque des revendications 1 à 14 en combinaison avec au moins un support pharmaceutiquement compatible, pharmaceutiquement inoffensif ou d'autres excipients.

18. Utilisation d'un composé selon l'une quelconque des revendications 1 à 14 pour la préparation d'un médicament destiné au traitement et/ou à la prophylaxie de maladies bactériennes.

19. Médicament selon la revendication 17 destiné au traitement et/ou à la prophylaxie d'infections bactériennes.
